Europäisches Patentamt

(19) European Patent Office

Office européen des brevets

(11) Publication number: **0 323 149 B1**

# EUROPEAN PATENT SPECIFICATION

(45) Date of publication of patent specification: **28.12.94**   (51) Int. Cl.⁵: **C12N  15/57**, C12N 9/64,
 A61K 37/54, C12N 5/10

(21) Application number: **88312201.2**

(22) Date of filing: **22.12.88**

(54) Vectors and compounds for expression of zymogen forms of human protein C.

(30) Priority: **28.12.87 US 138009**

(43) Date of publication of application:
 **05.07.89 Bulletin  89/27**

(45) Publication of the grant of the patent:
 **28.12.94 Bulletin  94/52**

(84) Designated Contracting States:
 **AT BE CH DE ES FR GB GR IT LI LU NL SE**

(56) References cited:
 **EP-A- 0 191 606**

(73) Proprietor: **ELI LILLY AND COMPANY**
 **Lilly Corporate Center**
 **Indianapolis**
 **Indiana 46285 (US)**

(72) Inventor: **Bang, Nils Ulrik**
 **706 East 71st Street**
 **Indianapolis**
 **Indiana 46220 (US)**
 Inventor: **Ehrlich, Hartmut Josef**
 **Badhoevelaan 65**
 **NL-1171 DB Badhoevedorp (NL)**
 Inventor: **Grinnell, Brian William**
 **5038 Haynes Court**
 **Indianapolis**
 **Indiana 46250 (US)**
 Inventor: **Yan, Sau-Chi Betty**
 **8131 Menlo Court East Drive**
 **Indianapolis**
 **Indiana 46240 (US)**

(74) Representative: **Hudson, Christopher Mark et al**
 **Lilly Industries Limited**
 **European Patent Operations**
 **Erl Wood Manor**
 **Windlesham**
 **Surrey GU20 6PH (GB)**

**Description**

The present invention relates to novel DNA compounds and recombinant DNA cloning vectors that encode novel zymogen forms of human protein C. These zymogens can be activated in vivo by thrombin alone at a rate of clinical significance and are much more susceptible to activation by thrombin/thrombomodulin than native protein C zymogen. The expression vectors provide a simple and efficient means for expressing these human protein C zymogens in recombinant host cells. Native human protein C zymogens require treatment with high levels of thrombin, or thrombin and thrombomodulin, or other expensive enzymes for activation. The present invention provides a method for producing zymogen forms of human protein C that serve as much better substrates for thrombin and consequently can be activated in the presence of lower levels of thrombin, or thrombin/thrombomodulin, or other enzymes. Most importantly, the zymogen forms of human protein C of the invention can be activated by thrombin even in the presence of physiological $Ca^{2+}$, which is inhibitory to the activation of native protein C zymogen by thrombin. The novel zymogen forms of human protein C differ from those known in the art in the amino acid residue sequence of the activation peptide, which is removed from the zymogen forms to produce activated human protein C. These novel zymogen forms of protein C offer special advantages in the treatment of blood disorders involving coagulation.

Protein C, a vitamin K dependent plasma protein, is of major physiological importance in the control of hemostasis and plays a significant role in the regulation of blood coagulation. Protein C is synthesized as an inactive molecule, herein called nascent protein C. Nascent protein C undergoes complex processing, giving rise to a number of different inactive molecules as is more fully described below. Inactive, secreted forms of protein C are referred to herein as zymogen protein C. Activation of protein C occurs in the blood by a reaction involving a thrombomodulin-thrombin complex. Activated protein C, together with its cofactor protein S, is an anticoagulant of important physiological significance. Activated protein C can prevent intravascular thrombosis and control the extension of existing clots. The mechanism of action of the activated form of protein C and the mechanism of activation of the inactive zymogen into the active protease have been clarified in recent years (for review, see J. E. Gardiner and J. H. Griffin, Progress in Hematology, Vol. XIII, pp. 265-278, ed. Elmer B. Brown, Grune and Stratton, Inc., 1983).

The activation of protein C involves thrombin, the final serine protease in the coagulation cascade, and an endothelial cell membrane-associated glycoprotein called thrombomodulin. Thrombomodulin forms a tight, stoichiometric complex with thrombin. Thrombomodulin, when complexed with thrombin, totally changes the functional properties of thrombin. Thrombin normally clots fibrinogen, activates platelets, and converts clotting cofactors V and VIII to their activated forms, Va and VIIIa. Finally, thrombin activates protein C, but only very slowly and inefficiently, and the activation is further inhibited by physiological $Ca^{2+}$. In contrast, thrombin complexed with thrombomodulin does not clot fibrinogen, activate platelets, or convert clotting factors V and VIII to their activated counterparts Va and VIIIa, but does become a very efficient activator of protein C zymogen in the presence of physiological $Ca^{2+}$. The rate constant of protein C zymogen activation by thrombomodulin-thrombin is over 1,000 fold higher than the rate constant for thrombin alone.

To understand how activated protein C down-regulates blood coagulation, the following brief description of the coagulation enzyme system is provided. The coagulation system is best looked at as a chain reaction involving the sequential activation of zymogens into active serine proteases. This chain reaction eventually produces the enzyme thrombin, which through limited proteolysis converts plasma fibrinogen into the insoluble gel fibrin. Two key events in the coagulation cascade are the conversion of clotting factor X to Xa by clotting factor IXa and the conversion of prothrombin into thrombin by clotting factor Xa. Both of these reactions occur on cell surfaces, most notably the platelet surface, and both reactions require cofactors. The major cofactors, factors V and VIII, in the system circulate as relatively inactive precursors, but when the first few molecules of thrombin are formed, thrombin loops back and activates the cofactors through limited proteolysis. The activated cofactors, Va and VIIIa, accelerate both the conversion of prothrombin into thrombin and also the conversion of factor X to factor Xa by approximately five orders of magnitude. Activated protein C preferentially acts on, to proteolytically degrade, hydrolyze, and irreversibly destroy clotting cofactors Va and VIIIa, the activated forms of the inactive clotting factors V and VIII. Clotting factors V and VIII, in contrast, are very poor substrates for activated protein C in vivo.

An important cofactor for activated protein C is protein S, another vitamin K-dependent plasma protein. Protein S substantially increases activated protein C-mediated hydrolysis of factors Va and VIIIa 25 fold.

Protein C is recognized as a valuable therapeutic agent (see, for example, European Patent Publications Nos. 0215548 and 0191606). Activated protein C is a novel antithrombotic agent with a wider therapeutic index than available anticoagulants, such as heparin and the oral hydroxycoumarin type anticoagulants.

Neither zymogen protein C nor activated protein C is effective until thrombin is generated, because thrombin is needed to convert clotting factors V to Va and VIII to VIIIa; the activated forms of these two cofactors are the preferred substrate for activated protein C. Thrombin is also required to activate zymogen protein C, for without the thrombomodulin-thrombin complex, the protein C zymogen is not converted into its active counterpart.

Activated protein C is an on-demand anticoagulant, because activated protein C works by inactivating cofactors Va and VIIIa. Because thrombin is required to convert factors V and VIII to their activated counterparts Va and VIIIa, protein C only acts as an anticoagulant after thrombin is generated. Conventional anticoagulants, in contrast to activated protein C, maintain a constant anticoagulant state throughout the circulation for as long as they are given to the patient, thereby substantially increasing the risk of bleeding complications over that for protein C or activated protein C. Activated protein C is therefore an on-demand anticoagulant of wide clinical utility for use as an alternative to heparin and the hydroxycoumarins.

In some disease states, such as hereditary protein C deficiency, protein C zymogen is of great therapeutic importance. In congenital homozygous protein C deficiency, affected individuals die in early childhood from purpura fulminans, an often lethal form of disseminated intravascular coagulation. In heterozygous protein C deficiency, affected individuals suffer severe, recurrent thromboembolic episodes. It is well established clinically that plasma protein concentrates designed to treat hemophilia B or factor IX deficiency, which contain protein C as an impurity, are effective in the prevention and treatment of intravascular clotting in heterozygous protein C deficiency. Protein C levels have also been noted to be abnormally low in thrombotic states such as disseminated intravascular coagulation and in disease states predisposing to thrombosis, such as major trauma, major surgery, and cancer.

To facilitate an understanding of the activation of protein C and the invention, the coding sequence, and corresponding amino acid residue sequence, for nascent human protein C is depicted below. This amino acid residue sequence, and relevant portions thereof, also characterizes "native human protein C" for purposes of the present invention.

```
                    10                  20                  30                  40
     5'-ATG TGG CAG CTC ACA AGC CTC CTG CTG TTC GTG GCC ACC TGG GGA ATT
     H2N-MET TRP GLN LEU THR SER LEU LEU LEU PHE VAL ALA THR TRP GLY ILE
                         5                               10                  15

         50                  60                  70                  80                  90
     TCC GGC ACA CCA GCT CCT CTT GAC TCA GTG TTC TCC AGC AGC GAG CGT
     SER GLY THR PRO ALA PRO LEU ASP SER VAL PHE SER SER SER GLU ARG
                        20                      25                      30

             100                 110                 120                 130                 140
     GCC CAC CAG GTG CTG CGG ATC CGC AAA CGT GCC AAC TCC TTC CTG GAG
     ALA HIS GLN VAL LEU ARG ILE ARG LYS ARG ALA ASN SER PHE LEU GLU
                        35                      40                      45

             150                 160                 170                 180                 190
     GAG CTC CGT CAC AGC AGC CTG GAG CGG GAG TGC ATA GAG GAG ATC TGT
     GLU LEU ARG HIS SER SER LEU GLU ARG GLU CYS ILE GLU GLU ILE CYS
                        50                      55                      60

                 200                 210                 220                 230                 240
     GAC TTC GAG GAG GCC AAG GAA ATT TTC CAA AAT GTG GAT GAC ACA CTG
     ASP PHE GLU GLU ALA LYS GLU ILE PHE GLN ASN VAL ASP ASP THR LEU
     65                          70                      75                          80

                 250                 260                 270                 280
     GCC TTC TGG TCC AAG CAC GTC GAC GGT GAC CAG TGC TTG GTC TTG CCC
     ALA PHE TRP SER LYS HIS VAL ASP GLY ASP GLN CYS LEU VAL LEU PRO
                        85                      90                          95

         290                 300                 310                 320                 330
     TTG GAG CAC CCG TGC GCC AGC CTG TGC TGC GGG CAC GGC ACG TGC ATC
     LEU GLU HIS PRO CYS ALA SER LEU CYS CYS GLY HIS GLY THR CYS ILE
                        100                     105                     110
```

```
        340           350           360           370           380
GAC GGC ATC GGC AGC TTC AGC TGC GAC TGC CGC AGC GGC TGG GAG GGC
ASP GLY ILE GLY SER PHE SER CYS ASP CYS ARG SER GLY TRP GLU GLY
            115                   120                   125

        390           400           410           420           430
CGC TTC TGC CAG CGC GAG GTG AGC TTC CTC AAT TGC TCG CTG GAC AAC
ARG PHE CYS GLN ARG GLU VAL SER PHE LEU ASN CYS SER LEU ASP ASN
            130                   135                   140

            440           450           460           470           480
GGC GGC TGC ACG CAT TAC TGC CTA GAG GAG GTG GGC TGG CGG CGC TGT
GLY GLY CYS THR HIS TYR CYS LEU GLU GLU VAL GLY TRP ARG ARG CYS
145                   150                   155                   160

            490           500           510           520
AGC TGT GCG CCT GGC TAC AAG CTG GGG GAC GAC CTC CTG CAG TGT CAC
SER CYS ALA PRO GLY TYR LYS LEU GLY ASP ASP LEU LEU GLN CYS HIS
                    165                   170                   175

530           540           550           560           570
CCC GCA GTG AAG TTC CCT TGT GGG AGG CCC TGG AAG CGG ATG GAG AAG
PRO ALA VAL LYS PHE PRO CYS GLY ARG PRO TRP LYS ARG MET GLU LYS
                    180                   185                   190

        580           590           600           610           620
AAG CGC AGT CAC CTG AAA CGA GAC ACA GAA GAC CAA GAA GAC CAA GTA
LYS ARG SER HIS LEU LYS ARG ASP THR GLU ASP GLN GLU ASP GLN VAL
            195                   200                   205

        630           640           650           660           670
GAT CCG CGG CTC ATT GAT GGG AAG ATG ACC AGG CGG GGA GAC AGC CCC
ASP PRO ARG LEU ILE ASP GLY LYS MET THR ARG ARG GLY ASP SER PRO
            210                   215                   220

            680           690           700           710           720
TGG CAG GTG GTC CTG CTG GAC TCA AAG AAG AAG CTG GCC TGC GGG GCA
TRP GLN VAL VAL LEU LEU ASP SER LYS LYS LYS LEU ALA CYS GLY ALA
225                   230                   235                   240

            730           740           750           760
GTG CTC ATC CAC CCC TCC TGG GTG CTG ACA GCG GCC CAC TGC ATG GAT
VAL LEU ILE HIS PRO SER TRP VAL LEU THR ALA ALA HIS CYS MET ASP
                    245                   250                   255
```

```
      770            780            790            800            810
GAG TCC AAG AAG CTC CTT GTC AGG CTT GGA GAG TAT GAC CTG CGG CGC
GLU SER LYS LYS LEU LEU VAL ARG LEU GLY GLU TYR ASP LEU ARG ARG
            260                        265                    270

      820            830            840            850            860
TGG GAG AAG TGG GAG CTG GAC CTG GAC ATC AAG GAG GTC TTC GTC CAC
TRP GLU LYS TRP GLU LEU ASP LEU ASP ILE LYS GLU VAL PHE VAL HIS
            275                        280                    285

      870            880            890            900            910
CCC AAC TAC AGC AAG AGC ACC ACC GAC AAT GAC ATC GCA CTG CTG CAC
PRO ASN TYR SER LYS SER THR THR ASP ASN ASP ILE ALA LEU LEU HIS
            290                        295                    300

            920            930            940            950            960
CTG GCC CAG CCC GCC ACC CTC TCG CAG ACC ATA GTG CCC ATC TGC CTC
LEU ALA GLN PRO ALA THR LEU SER GLN THR ILE VAL PRO ILE CYS LEU
305                        310                        315                    320

            970            980            990            1000
CCG GAC AGC GGC CTT GCA GAG CGC GAG CTC AAT CAG GCC GGC CAG GAG
PRO ASP SER GLY LEU ALA GLU ARG GLU LEU ASN GLN ALA GLY GLN GLU
                  325                        330                        335

      1010            1020            1030            1040            1050
ACC CTC GTG ACG GGC TGG GGC TAC CAC AGC AGC CGA GAG AAG GAG GCC
THR LEU VAL THR GLY TRP GLY TYR HIS SER SER ARG GLU LYS GLU ALA
                  340                        345                        350

            1060            1070            1080            1090            1100
AAG AGA AAC CGC ACC TTC GTC CTC AAC TTC ATC AAG ATT CCC GTG GTC
LYS ARG ASN ARG THR PHE VAL LEU ASN PHE ILE LYS ILE PRO VAL VAL
            355                        360                        365

            1110            1120            1130            1140            1150
CCG CAC AAT GAG TGC AGC GAG GTC ATG AGC AAC ATG GTG TCT GAG AAC
PRO HIS ASN GLU CYS SER GLU VAL MET SER ASN MET VAL SER GLU ASN
            370                        375                        380

            1160            1170            1180            1190            1200
ATG CTG TGT GCG GGC ATC CTC GGG GAC CGG CAG GAT GCC TGC GAG GGC
MET LEU CYS ALA GLY ILE LEU GLY ASP ARG GLN ASP ALA CYS GLU GLY
385                        390                        395                    400
```

```
            1210            1220          1230           1240
GAC AGT GGG GGG CCC ATG GTC GCC TCC TTC CAC GGC ACC TGG TTC CTG
ASP SER GLY GLY PRO MET VAL ALA SER PHE HIS GLY THR TRP PHE LEU
            405                           410                      415

        1250        1260          1270          1280          1290
GTG GGC CTG GTG AGC TGG GGT GAG GGC TGT GGG CTC CTT CAC AAC TAC
VAL GLY LEU VAL SER TRP GLY GLU GLY CYS GLY LEU LEU HIS ASN TYR
            420                           425                      430

        1300          1310          1320          1330          1340
GGC GTT TAC ACC AAA GTC AGC CGC TAC CTC GAC TGG ATC CAT GGG CAC
GLY VAL TYR THR LYS VAL SER ARG TYR LEU ASP TRP ILE HIS GLY HIS
            435                           440                      445

        1350          1360          1370          1380
ATC AGA GAC AAG GAA GCC CCC CAG AAG AGC TGG GCA CCT TAG-3'
ILE ARG ASP LYS GLU ALA PRO GLN LYS SER TRP ALA PRO-COOH
            450                           455                      460
```

wherein A is deoxyadenyl, G is deoxyguanyl, C is deoxycytidyl, T is thymidyl, ALA is Alanine, ARG is Arginine, ASN is Asparagine, ASP is Aspartic acid, -COOH is the carboxy terminus, CYS is Cysteine, GLN is Glutamine, GLU is Glutamic Acid, GLY is Glycine, $H_2N$ is the amino terminus, HIS is Histidine, $H_2N$-is the amino terminus, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine.

The DNA sequence depicted above was derived from cDNA clones prepared from human liver mRNA that encodes human protein C. Those skilled in the art recognize that the degenerate nature of the genetic code enables one to construct many different DNA sequences that encode the same amino acid residue sequence. The cDNA sequence for nascent human protein C depicted above is thus only one of many possible nascent human protein C-encoding sequences. In constructing the cDNA clones, a 5' poly G sequence, a 3' poly C sequence, and both 5' and 3' PstI restriction enzyme recognition sequences were constructed at the ends of the protein C-encoding cDNA. Two of these cDNA clones were manipulated to construct a DNA molecule comprising both the coding sequence of nascent human protein C and also portions of the DNA encoding the untranslated mRNA at the 5' and 3' ends of the coding region. This DNA molecule was inserted into the PstI site of plasmid pBR322 to construct plasmid pHC7. Plasmid pHC7 thus comprises the coding sequence above and, again depicting only one strand of the molecule, also contains these additional sequences:

```
5'-C TGC AGG GGG GGG GGG GGG GGG GGG CTG TCA TGG CGG CAG GAC

    GGC GAA CTT GCA GTA TCT CCA CGA CCC GCC CCT ACA GGT GCC

    AGT GCC TCC AGA-3'
```

and

```
5'-CGA CCC TCC CTG CAG GGC TGG GCT TTT GCA TGG CAA TGG ATG GGA

    CAT TAA AGG GAC ATG TAA CAA GCA CAC CCC CCC CCC CCC CCC CCC

    CCC CCC CCT GCA G-3'
```

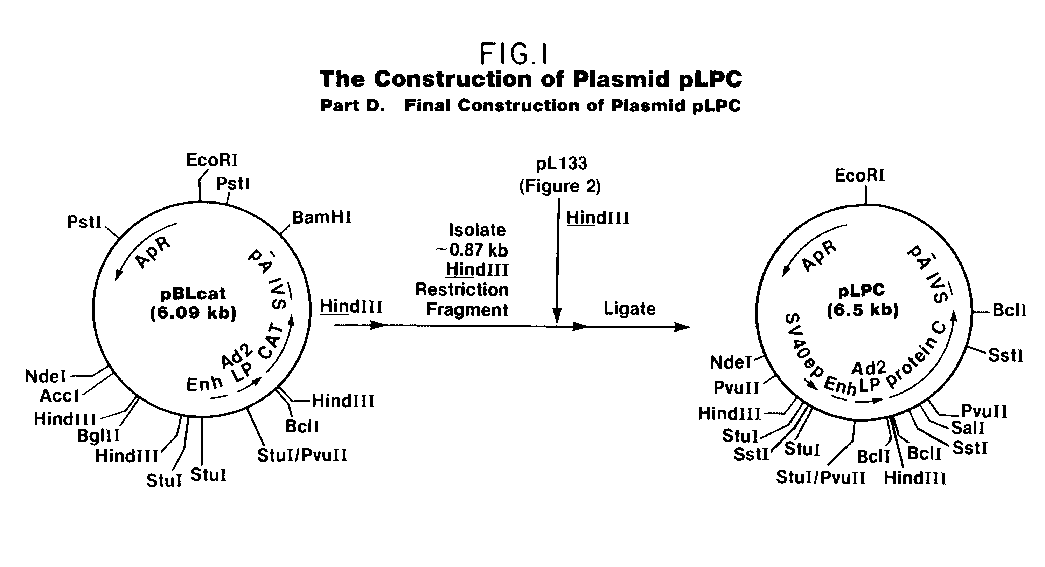

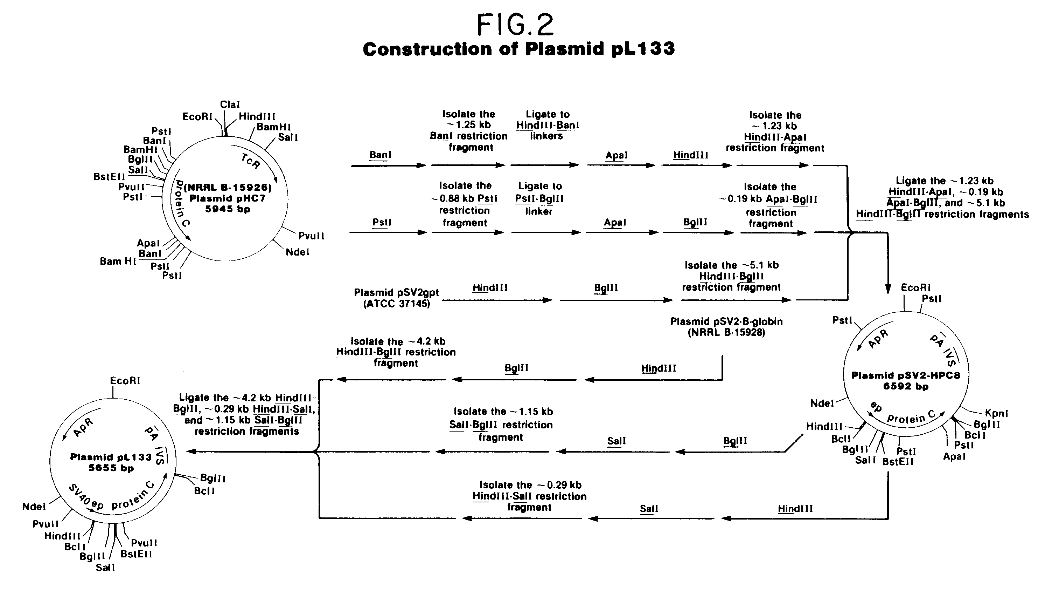

at the 5' and 3' ends, respectively, of the coding strand of the nascent human protein C coding sequence. Due to the complementary nature of DNA base-pairing, the sequence of one strand of a double-stranded DNA molecule is sufficient to determine the sequence of the opposing strand. Plasmid pHC7 can be conventionally isolated from E. coli K12 RR1/pHC7, a strain deposited with and made part of the permanent stock culture collection of the Northern Regional Research Laboratory (NRRL), Peoria, Illinois. A culture of E. coli K12 RR1/pHC7 can be obtained from the NRRL under the accession number NRRL B-15926. A restriction site and function map of plasmid pHC7 is presented in Figure 2 of the accompanying drawings.

Nascent protein C can also be depicted schematically, as shown below.

```
|1        42|43        197|198   199|200  211|212        461|
|pre-pro    |    LC       |   KR    |   AP   |    AHC       |

                                    <———————HC———————>
```

pre-pro -     amino acid residues 1-42 of nascent human protein C encode the signal peptide and propeptide of human protein C, important for directing secretion and $\gamma$-carboxylation of protein C.

LC -     amino acid residues 43-197 of nascent protein C, once post-translationally modified, constitute the light chain (LC) of both the two-chain zymogen (formed from one-chain zymogen by removal of the KR dipeptide, as discussed below) and activated forms of protein C.

KR -     amino acid residues 198-199 of nascent human protein C; these residues are believed to be removed (on the basis of homology with bovine protein C), probably by a two-step process comprising a first cleavage (either between residues 197-198 or 199-200) followed by carboxypeptidase or aminopeptidase action, to form two-chain protein C.

AP -     amino acid residues 200-211 of nascent protein C constitute the activation peptide, which is removed from the zymogen forms of protein C to obtain activated protein C.

AHC -     amino acid residues 212-461 of nascent protein C, once post-translationally modified, constitute the activated heavy chain (AHC) of active protein C.

HC -     the heavy chain of the two chain form of protein C zymogen, once post-translationally modified, is composed of amino acid residues 200-461, the AP and AHC.

Human protein C zymogen is a serine protease precursor synthesized in the liver and present in the blood. For expression of complete biological activity, protein C requires post-translational modifications for which vitamin K is needed. The two-chain, disulfidelinked, protein C zymogen arises from the single-chain zymogen by limited proteolysis. This limited proteolysis is believed to include cleavage and removal of amino acid residues 198 and 199. The activation of the two-chain zymogen into the active serine protease involves the proteolytic cleavage of an ARG-LEU peptide bond (residues 211 and 212). This latter cleavage releases a dodecapeptide (residues 200-211), the activation peptide, that constitutes the amino-terminus of the larger (heavy) chain of the two-chain zymogen molecule. Protein C is significantly glycosylated; the mature enzyme contains ~23% carbohydrate. Protein C also contains a number of unusual amino acids, including $\gamma$-carboxyglutamic acid and $\beta$-hydroxyaspartic acid (erythro-L-$\beta$-hydroxy aspartate). $\gamma$-carboxyglutamic acid (gla) is produced by $\gamma$-glutamyl carboxylation from glutamic acid residues with the aid of a hepatic microsomal carboxylase which requires vitamin K as a cofactor.

The activation of human protein C can also be represented schematically and is shown below. Those skilled in the art recognize that the order of the steps shown in the schematic do not necessarily reflect the order of the steps in the in vivo pathway.

```
                          pre-pro-LC-KR-AP-AHC        nascent protein C

                                        |
  post-translational modification,      |
  i.e., γ-carboxylation of specific|
  glutamic acid residues, β-             |
  hydroxylation of an aspartic           |
  acid residue, and glycosylation        ↓


  secretion, the removal of              |
  residues 1-42, which may               |
  involve more than one                  |
  proteolytic cleavage                   ↓


                          LC-KR-AP-AHC          one-chain zymogen

                                        |
  removal of residues 198-199,          |
  about 90% of the zymogen protein |
  C found in human blood is the         |
  two chain form (S-S= disulfide        |
  bond)                                 |
                                        ↓

                          LC
                          |
                          S-S                   two-chain zymogen
                          |
                          AHC-AP

  activation by                         |
  thrombin-thrombomodulin               |
                                        ↓

                          LC
                          |
                          S-S                   activated protein C
                          |
                          AHC
```

The present invention provides novel compounds, vectors, transformants, and methods for the recombinant expression of novel protein C zymogens.

For purposes of the present invention, as disclosed and claimed herein, the following terms are as defined below.

Ad2LP - the major late promoter of adenovirus type 2.

Amino acid residues in proteins or peptides described herein as abbreviated as follows:

| Three-Letter Abbreviation | Amino Acid Residue | One-Letter Abbreviation |
|---|---|---|
| PHE | Phenylalanine | F |
| LEU | Leucine | L |
| ILE | Isoleucine | I |
| MET | Methionine | M |
| VAL | Valine | V |
| SER | Serine | S |
| PRO | Proline | P |
| THR | Threonine | T |
| ALA | Alanine | A |
| TYR | Tyrosine | Y |
| HIS | Histidine | H |
| GLN | Glutamine | Q |
| ASN | Asparagine | N |
| LYS | Lysine | K |
| ASP | Aspartic Acid | D |
| GLU | Glutamic Acid | E |
| CYS | Cysteine | C |
| TRP | Tryptophan | W |
| ARG | Arginine | R |
| GLY | Glycine | G |

ApR - the ampicillin-resistant phenotype or gene conferring same.

BK - DNA from BK virus.

CAT - the chloramphenicol acetyltransferase gene.

Enh or enhancer - the enhancer of BK virus.

ep or SV40ep - a DNA segment comprising the SV40 early promoter of the T-antigen gene, the T-antigen binding sites, the SV40 enhancer, and the SV40 origin of replication.

$\gamma$-carboxylation - a reaction which adds a carboxyl group to glutamic acids at the $\gamma$-carbon.

$\gamma$-carboxylated protein - a protein in which some glutamic acids residues have undergone $\gamma$-carboxylation.

IVS - DNA encoding an intron, also called an intervening sequence.

MMTpro - the promoter of the mouse metallothionein-I gene.

Nascent protein - the polypeptide produced upon translation of a mRNA transcript, prior to any post-translational modifications. However, post-translational modifications such as $\gamma$-carboxylation of glutamic acid residues and hydroxylation of aspartic acid residues may occur before a protein is fully translated from an mRNA transcript.

NeoR - a neomycin resistance-conferring gene, which can also be used to confer resistance to the antibiotic G418.

pA - a DNA sequence encoding a polyadenylation signal.

Promoter - a DNA sequence that directs transcription of DNA into RNA.

Protein C activity - any property of human protein C responsible for proteolytic, amidolytic, esterolytic, and biological (anticoagulant or profibrinolytic) activities. Methods for testing for protein anticoagulant activity are well known in the art, i.e., see Grinnell et al., 1987, Biotechnology 5:1189.

Recombinant DNA Cloning Vector - any agent, including, but not limited to; chromosomally integrating agents, autonomously replicating plasmids, and phages, comprising a DNA molecule to which one or more additional DNA segments can be or have been added.

Recombinant DNA Expression Vector - any recombinant DNA cloning vector into which a promoter has been incorporated and positioned to drive expression of a gene product.

Recombinant DNA Vector - any recombinant DNA cloning or expression vector.

Replicon - A DNA sequence that controls and allows for autonomous replication of a plasmid or other vector.

Restriction Fragment - any linear DNA sequence generated by the action of one or more restriction endonuclease enzymes.

Sensitive Host Cell - a host cell that cannot grow in the presence of a given antibiotic or other toxic compound without a DNA segment that confers resistance thereto.

10

TcR - the tetracycline-resistant phenotype or gene conferring same.

Transformation - the introduction of DNA into a recipient host cell that changes the genotype of the recipient cell.

Transformant - a recipient host cell that has undergone transformation.

Translational Activating Sequence - any DNA sequence, inclusive of that encoding a ribosome binding site and translational start codon, such as 5′-ATG-3′, that provides for the translation of a mRNA transcript into a peptide or polypeptide.

Zymogen - an enzymatically inactive precursor of a proteolytic enzyme. Protein C zymogen, as used herein, refers to secreted, inactive forms, whether one chain or two chain, of protein C.

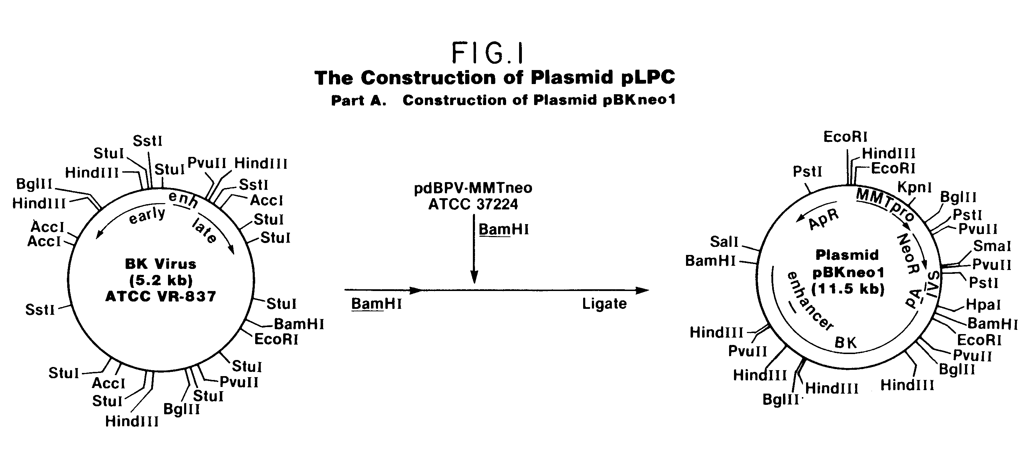

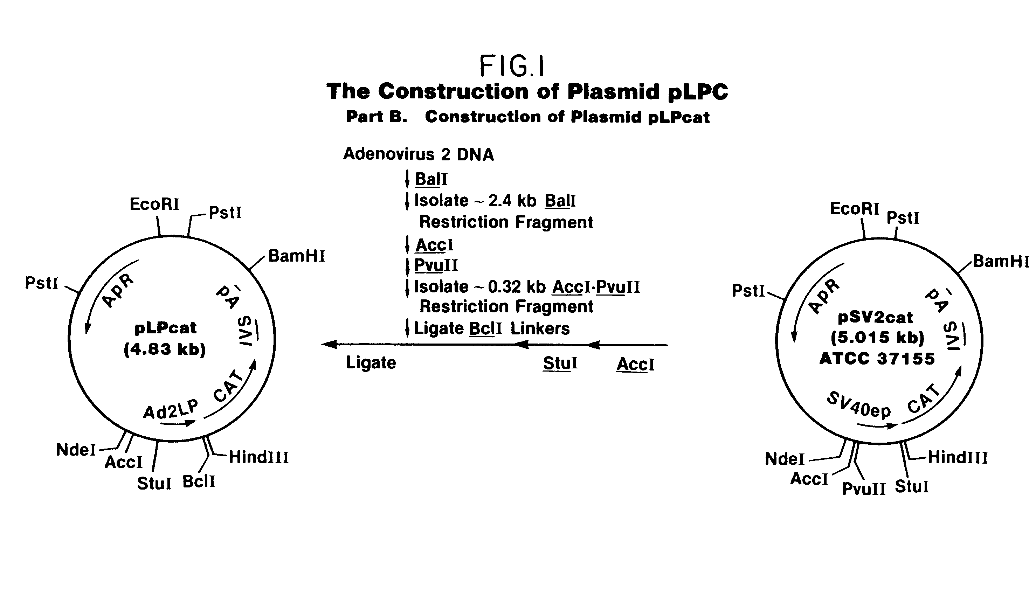

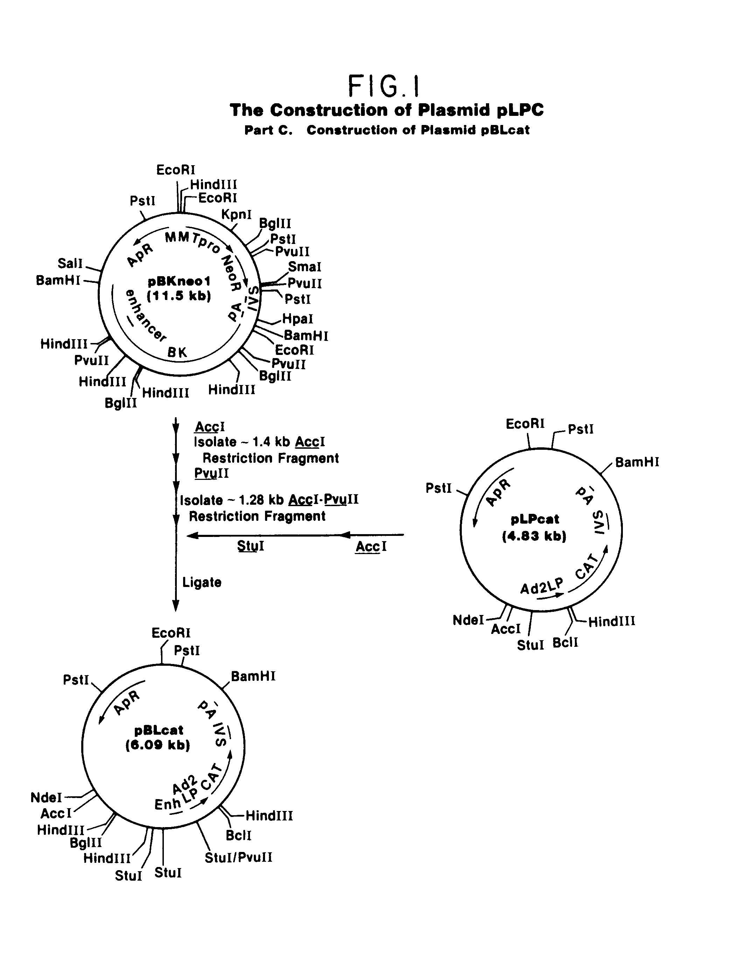

Figure 1 consists of four parts and schematically illustrates the construction protocol for plasmid pLPC, a starting material used in the construction of starting plasmid pLAPC.

Figure 1, Part A depicts the construction of plasmid pBKneo1 from BK virus and plasmid pdBPV-MMtneo.

Figure 1, Part B depicts the construction of plasmid pLPcat from adenovirus 2 and plasmid pSV2cat.

Figure 1, Part C depicts the construction of plasmid pBLcat from plasmid pBKneo1 and plasmid pLPcat.

Figure 1, Part D depicts the construction of plasmid pLPC from plasmid pBLcat and plasmid pL133.

Figure 2 schematically illustrates the construction of plasmid pL133, a starting material used in the construction of plasmid pLPC.

The present invention relates to DNA compounds that code for the expression of novel zymogen forms of human protein C. Several methods of producing native human protein C zymogen and nascent human protein C have been described (see European Patent Publications 215548 and 191606). These prior art methods provide for the expression of zymogen forms of human protein C that do not differ from the zymogen forms present in human blood. The protein C zymogen produced by these methods must be treated with substances such as $\alpha$-thrombin, trypsin, or a mixture of thrombin and thrombomodulin (whether in vivo or in vitro) to obtain activated protein C. In addition, a zymogen form of human protein C produced by recombinant DNA technology that is identical to zymogen forms of human protein C found naturally in human blood will only be activated in the body by the natural activation pathway involving the thrombin-thrombomodulin complex. Native human protein C zymogen can be activated by thrombin alone; however, the activation requires the absence of $Ca^{2+}$ and such high levels of thrombin and/or protein C zymogen that it is not a significant in vivo pathway to activated protein C.

The present invention provides zymogen forms of human protein C that can be activated in vivo by thrombin alone at a rate of clinical significance. In addition, these zymogen forms are much more susceptible to activation by thrombin/thrombomodulin than native human protein C zymogen. The present invention also provides DNA compounds, recombinant DNA expression vectors, transformed cell lines, and methods for the recombinant expression of these novel zymogen forms of human protein C. The method for producing these zymogen forms of human protein C comprises:

(A) transforming a eukaryotic host cell with a recombinant DNA vector, said vector comprising:

(i) a DNA sequence that encodes an amino acid residue sequence, said amino residue sequence comprising, from the amino terminus to the carboxy terminus:

a) a signal peptide and pro-peptide of a $\gamma$-carboxylated, secreted protein;

b) the light chain of human protein C;

c) a dipeptide selected from the group consisting of LYS-ARG, ARG-LYS, LYS-LYS, and ARG-ARG; and

11

d) the amino acid residue sequence:

```
                                        ASP THR GLU ASP GLN GLU ASP GLN VAL

     R₁  R₂  ARG LEU ILE R₃  GLY LYS MET THR ARG ARG GLY ASP SER PRO

     TRP GLN VAL VAL LEU LEU ASP SER LYS LYS LYS LEU ALA CYS GLY ALA

     VAL LEU ILE HIS PRO SER TRP VAL LEU THR ALA ALA HIS CYS MET ASP

     GLU SER LYS LYS LEU LEU VAL ARG LEU GLY GLU TYR ASP LEU ARG ARG

     TRP GLU LYS TRP GLU LEU ASP LEU ASP ILE LYS GLU VAL PHE VAL HIS

     PRO ASN TYR SER LYS SER THR THR ASP ASN ASP ILE ALA LEU LEU HIS

     LEU ALA GLN PRO ALA THR LEU SER GLN THR ILE VAL PRO ILE CYS LEU

     PRO ASP SER GLY LEU ALA GLU ARG GLU LEU ASN GLN ALA GLY GLN GLU

     THR LEU VAL THR GLY TRP GLY TYR HIS SER SER ARG GLU LYS GLU ALA

     LYS ARG ASN ARG THR PHE VAL LEU ASN PHE ILE LYS ILE PRO VAL VAL

     PRO HIS ASN GLU CYS SER GLU VAL MET SER ASN MET VAL SER GLU ASN

     MET LEU CYS ALA GLY ILE LEU GLY ASP ARG GLN ASP ALA CYS GLU GLY

     ASP SER GLY GLY PRO MET VAL ALA SER PHE HIS GLY THR TRP PHE LEU

     VAL GLY LEU VAL SER TRP GLY GLU GLY CYS GLY LEU LEU HIS ASN TYR

     GLY VAL TYR THR LYS VAL SER ARG TYR LEU ASP TRP ILE HIS GLY HIS

     ILE ARG ASP LYS GLU ALA PRO GLN LYS SER TRP ALA PRO-COOH
```

wherein R₁ is selected from the group consisting of PHE, GLY, TYR, and TRP, R₂ is selected from the group consisting of VAL and PRO, R₃ is selected from the group consisting of ASP and ASN, ARG is Arginine, ASN is Asparagine, ASP is Aspartic acid, -COOH is the carboxy terminus, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine; and

(ii) a promoter positioned to drive expression of said DNA sequence; and

(B) culturing said host cell transformed in step (A) under conditions that allow for expression of said DNA sequence. This method and compounds useful in the method are more fully described below.

The invention also provides DNA compounds for use in the method of producing these novel zymogen forms of human protein C. These novel compounds all encode a pre-propeptide comprising a signal peptide for directing secretion and a propeptide from a γ-carboxylated (through the action of a vitamin K-dependent carboxylase) protein. Such propeptide sequences are well-known in the art. See, for example, Suttie et al., 1987, Proc. Natl. Acad. Sci. 84:634-637. Preferably, and for ease of construction, both the signal peptide coding sequence and the propeptide coding sequence will be derived from the amino acid residue sequence of the pre-propeptide of a γ-carboxylated protein. Examples of such γ-carboxylatedproteins include, but are not limited to, factor VII, factor IX, factor X, prothrombin, protein S, protein Z, and, protein C. A DNA sequence encoding the pre-propeptide of human protein C is most preferred for use in the vectors of the invention.

The DNA compounds of the invention further comprise the coding sequence for the light chain of human protein C positioned immediately adjacent to, downstream of, and in translational reading frame with the pre-propeptide coding sequence. The light chain of human protein C contains amino acid residues 43 to 197, inclusive, of nascent protein C, as depicted in the background section above. The amino-terminal portions of the vitamin K-dependent plasma proteins, such as the amino-terminal portion of the light chain of protein C, are responsible for calcium-binding activity of these proteins. The calcium-binding domains of these plasma proteins, such as factor VII, factor IX, factor X, prothrombin, and protein S, are interchangeable (see European Patent Publication No. 0215548A1, at pages 12 and 13) and equivalent to the calcium-binding domain of the light chain of human protein C.

The DNA compounds of the invention further comprise the coding sequence for the dipeptide LYS-ARG (KR) positioned immediately adjacent to, downstream of, and in translational reading frame with the light chain coding sequence. A dibasic dipeptide such as LYS-ARG is positioned in the nascent protein at the carboxyl-terminal side of the light chain. The orientation of the LYS-ARG dipeptide in the expressed protein is irrelevant for purposes of the present invention. Dibasic dipeptides such as LYS-LYS or ARG-ARG are equivalent to the LYS-ARG dipeptide for purposes of the present invention. For purposes of the present invention, however, the dipeptide LYS-ARG, which is the dipeptide in native human protein C, is preferred.

Immediately downstream of the codons for the LYS-ARG dipeptide is the coding sequence of the activation peptide. In the compounds of the invention, changes in the activation peptide coding sequence (and corresponding amino acid sequence) are primarily responsible for the property of increased thrombin-sensitivity of these novel zymogens.

Those skilled in the art will recognize that the zymogen forms of the present invention primarily differ from native zymogen forms of human protein C as described below. In native human protein C the activation peptide is:

```
200 201 202 203 204 205 206 207 208 209 210 211
ASP-THR-GLU-ASP-GLN-GLU-ASP-GLN-VAL-ASP-PRO-ARG,
```

in which the numbers refer to the position of the amino acid residues in nascent human protein C. The present invention discloses that changing the ASP residue at position 209 to either a PHE, GLY, TYR, or TRP residue will result in the corresponding zymogen form having a greater sensitivity to cleavage by thrombin alone, in addition to a greater sensitivity to cleavage by the thrombin-thrombomodulin complex.

Other amino acid substitutions, in conjunction with the substitutions at position 209, can also enhance the thrombin-sensitivity of the resulting zymogen. The phrase "resulting zymogen" is used to indicate that although substitutions are described with reference to amino acid positions in nascent human protein C, nascent human protein C must first be secreted (resulting in removal of amino acid residues 1 through 42) to obtain a zymogen form. Substitution of the proline residue (in the activation peptide) at position 210 in nascent human protein C, in addition to one of the four substitutions at position 209 described above, for a valine residue thus results in a novel zymogen of the present invention. Substitution of the aspartic acid residue (in the activated heavy chain) at position 214 in nascent human protein C, in addition to one of the four substitutions at position 209 described above, and whether or not in addition to the substitution at position 210 described above, for an asparagine residue also results in a novel zymogen of the present invention.

Thus, the preferred novel zymogen forms of human protein C of the present invention result from secretion and processing of nascent human protein C molecules with the amino acid residue sequence depicted below:

H₂N-MET TRP GLN LEU THR SER LEU LEU LEU PHE VAL ALA THR TRP GLY ILE

SER GLY THR PRO ALA PRO LEU ASP SER VAL PHE SER SER SER GLU ARG

ALA HIS GLN VAL LEU ARG ILE ARG LYS ARG ALA ASN SER PHE LEU GLU

GLU LEU ARG HIS SER SER LEU GLU ARG GLU CYS ILE GLU GLU ILE CYS

ASP PHE GLU GLU ALA LYS GLU ILE PHE GLN ASN VAL ASP ASP THR LEU

ALA PHE TRP SER LYS HIS VAL ASP GLY ASP GLN CYS LEU VAL LEU PRO

LEU GLU HIS PRO CYS ALA SER LEU CYS CYS GLY HIS GLY THR CYS ILE

ASP GLY ILE GLY SER PHE SER CYS ASP CYS ARG SER GLY TRP GLU GLY

```
ARG PHE CYS GLN ARG GLU VAL SER PHE LEU ASN CYS SER LEU ASP ASN

GLY GLY CYS THR HIS TYR CYS LEU GLU GLU VAL GLY TRP ARG ARG CYS

SER CYS ALA PRO GLY TYR LYS LEU GLY ASP ASP LEU LEU GLN CYS HIS

PRO ALA VAL LYS PHE PRO CYS GLY ARG PRO TRP LYS ARG MET GLU LYS

LYS ARG SER HIS LEU LYS ARG ASP THR GLU ASP GLN GLU ASP GLN VAL

R₁  R₂  ARG LEU ILE R₃  GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

wherein R₁ is PHE, GLY, TYR, or TRP; R₂ is PRO or VAL; and R₃ is ASP or ASN.

Those skilled in the art will recognize that, due to the degeneracy of the genetic code, a variety of DNA compounds can encode the polypeptide depicted above. Consequently, the constructions described below and in the accompanying Examples for the preferred DNA compounds, vectors, and transformants of the invention are merely illustrative and do not limit the scope of the invention.

The novel coding sequences of the invention can be readily constructed starting from a coding sequence for nascent human protein C from which the AP-encoding region has been deleted by site-specific mutagenesis. Shown schematically, this coding sequence has the structure:

| pre-pro |   LC   |  KR  |   AHC   |

As described in the accompanying examples, this coding sequence was inserted into a recombinant DNA expression vector and the resulting vector was designated plasmid pLAPC. Plasmid pLAPC serves as useful

15

starting material for the construction of illustrative vectors of the invention that drive high-level recombinant expression of the novel zymogen forms of human protein C of the invention. The construction protocol for plasmid pLAPC from starting plasmid pHC7 is described in Example 1. Plasmid pHC7 is available from the Northern Regional Research Center (NRRL), Peoria, IL 61604 in E. coli K12 RR1/pHC7 under the accession number NRRL B-15926.

Plasmid pLPC-167G is an illustrative expression vector of the invention in which the codon for aspartic acid at position 209 in nascent human protein C has been changed to a codon for glycine. The construction protocol for plasmid pLPC-167G is described in detail in Example 3. Essentially, the construction involved site-specific mutagenesis of the protein C coding sequence. A portion of the protein C coding sequence comprising the activation peptide-encoding DNA was isolated from plasmid pHC7, inserted into phage M13mp18, and then altered by site-specific mutagenesis. The mutagenized coding sequence was then cloned into a eukaryotic cloning vector to achieve a plasmid, designated pLPC-167G, identical to plasmid pLAPC, except for the insertion of a coding sequence for the activation peptide in which the codon for glycine has been substituted for the codon for aspartic acid at position 209.

Plasmid pLPC-167F is an illustrative expression vector of the invention in which the codon for aspartic acid at position 209 in nascent human protein C has been changed to a codon for phenylalanine. The construction protocol for plasmid pLPC-167F is described in detail in Example 4. Other than the different mutagenizing oligonucleotide used in the construction, the construction protocol for plasmid pLPC-167F was substantially the same as the construction protocol for plasmid pLPC-167G.

The methods of site-specific mutagenesis described in the accompanying Examples are illustrative and can be used to generate other compounds and vectors of the invention. As stated above, these other compounds of the invention include the nascent proteins produced upon translation of the mRNA transcripts generated from the DNA coding sequences of the invention. The compounds of the invention also include the zymogen forms generated upon secretion of the nascent proteins of the invention. In addition, in the case of the compounds of the invention in which the aspartic acid residue at position 214 has been changed to an asparagine residue, the activated protein C derivative produced upon activation of the zymogen form is also a compound of the invention. Thus, the compounds of the invention include DNA coding sequences, expression vectors that drive expression of those sequences, nascent proteins produced upon translation of mRNA transcripts generated from those coding sequences, zymogens produced upon secretion of those nascent proteins, and activated derivatives of certain of the zymogens.

In the preferred coding sequences of the invention (and thus the preferred nascent proteins, zymogens, and activated molecules), the coding sequence encodes an amino acid residue sequence identical to that of nascent human protein C except for the substitutions at positions 209, 210, and 214. These substitutions are depicted below in Table I.

Table I

| Amino Acid Residues Encoded at Positions 209, 210, and 214 in the Preferred Coding Sequences of the Invention | | | |
|---|---|---|---|
| Compound | 209 | 210 | 214 |
| 1 | PHE | PRO | ASP |
| 2 | PHE | PRO | ASN |
| 3 | PHE | VAL | ASP |
| 4 | PHE | VAL | ASN |
| 5 | GLY | PRO | ASP |
| 6 | GLY | PRO | ASN |
| 7 | GLY | VAL | ASP |
| 8 | GLY | VAL | ASN |
| 9 | TYR | PRO | ASP |
| 10 | TYR | PRO | ASN |
| 11 | TYR | VAL | ASP |
| 12 | TYR | VAL | ASN |
| 13 | TRP | PRO | ASP |
| 14 | TRP | PRO | ASN |
| 15 | TRP | VAL | ASP |
| 16 | TRP | VAL | ASN |

The DNA compounds of the invention can also be synthesized chemically, or by combining restriction fragments, or by a combination of techniques known in the art. DNA synthesizing machines are also available and can be used to construct the compounds of the invention.

The illustrative vectors of the invention, plasmids pLPC-167G and pLPC-167F, comprise the BK enhancer positioned to stimulate transcription by the adenovirus major late promoter of the coding sequence of the invention. Those skilled in the art recognize that a great number of eukaryotic promoters, enhancers, and expression vectors are known in the art and can be used in the method of the present invention. Those skilled in the art also recognize that a eukaryotic expression vector can function without an enhancer element. The key aspect of the present invention does not reside in the particular enhancer, if any, or promoter, used to drive expression of the protein C zymogen but rather resides in the novel coding sequence and corresponding proteins produced from that sequence.

However, choice of vector elements, such as promoters, enhancers, and selectable markers, can have great impact on the ultimate levels of protein produced by a eukaryotic host cell. European Publication No. 0245949 discloses a number of expression vectors for native zymogen protein C that utilize the BK enhancer to stimulate a eukaryotic promoter positioned to drive expression of nascent human protein C. These vectors drive especially high expression levels when transformed into eukaryotic cells that also express an immediate-early gene product of a large DNA virus, such as the E1A gene product of adenovirus. As is evident from the illustrative vectors pLPC-167G and pLPC-167F disclosed herein, the BK enhancer-E1A gene product expression method is especially preferred for use with the vectors of the present invention.

The present invention is not limited to use in a particular eukaryotic host cell. A variety of eukaryotic host cells are available from depositories such as the American Type Culture Collection (ATCC) Rockville, MD 20852, and are suitable for use with the vectors of the invention. The choice of a particular host cell depends to some extent on the particular expression vector used to drive expression of the protein C-encoding DNA compounds of the invention. Because nascent human protein C and the nascent human protein C derivatives of the invention undergo substantial post-translational modification, however, some host cells are more preferred for use with the vectors of the invention. Grinnell et al., 1987, Bio/Technology 5:1189 disclose that adenovirus-transformed, human embryonic kidney cells are especially preferred for use in the recombinant production of $\gamma$-carboxylated proteins such as human protein C. One such adenovirus-transformed, human embryonic kidney cell line is the 293 cell line, available from the ATCC under the accession number ATCC CRL 1573. The 293 cell line is also preferred for use with the vectors of the present invention.

However, the advantages of producing a $\gamma$-carboxylated protein, such as human protein C zymogen, in an adenovirus-transformed cell line are not limited to adenovirus-transformed human embryonic kidney

17

cells. In fact, adenovirus-transformed cells in general are exceptional hosts for the production of $\gamma$-carboxylated human protein C. One especially preferred cell line of this type is the AV12-664 (hereinafter "AV12") cell line, available from the ATCC under the accession number ATCC CRL 9595. The AV12 cell line was constructed by injecting a Syrian hamster in the scruff of the neck with human adenovirus 12 and isolating cells from the resulting tumor. Example 5, below, describes the transformation of both the 293 and AV12 cell lines with illustrative vectors pLPC-167G and pLPC-167F.

The vectors of the invention can be transformed into and expressed in a variety of eukaryotic, especially mammalian, host cells. Vectors of the invention that possess no selectable marker with which to isolate and identify stable eukaryotic transformants are useful not only for purposes of transient assay but also for purposes of cotransformation, a procedure disclosed in U.S. Patent No. 4,399,216. The vectors of the invention can also comprise sequences that allow for replication in E. coli, as it is usually more efficient to prepare plasmid DNA in E. coli than in other host organisms.

Expression of the coding sequences for human protein C contained on the vectors of the invention occurs in those host cells in which the particular promoter associated with the structural gene functions. Exemplary host cells suitable for use in the invention are listed in Table II, along with appropriate comments.

Table II

| Host Cell | Origin | Source | Comments |
|---|---|---|---|
| HepG-2 | Human Liver Hepatoblastoma | *ATCC # HB 8065 | U.S. Patent No. 4,393,133 describes the use of this cell line. |
| CV-1 | African Green Monkey Kidney | ATCC # CCL 70 | |
| LLC-MK$_2$ original | Rhesus Monkey Kidney | ATCC # CCL 7 | |
| LLC-MK$_2$ derivative | Rhesus Monkey Kidney | ATCC # CCL 7.1 | Grows faster than ATCC # CCL 7 |
| 3T3 | Mouse Embryo Fibroblasts | ATCC # CCL 92 | |
| CHO-K1 | Chinese Hamster Ovary | ATCC # CCL 61 | Proline-requiring. Derivatives of CHO-K1, such as the dhfr- derivative DXB11, can be generated from this host. |
| HeLa | Human Cervix Epitheloid | ATCC # CCL 2 | |
| RPMI8226 | Human Myeloma | ATCC # CCL 155 | IgG lambda-type light chain secreting |
| H4IIEC3 | Rat Hepatoma | ATCC # CRL 1600 | Derivatives, such as 8-azaguanine-resistant FAZA host cells, can be generated from this host. |
| C127I | Mouse Fibroblast | ATCC # CRL 1616 | |
| HS-Sultan | Human Plasma Cell Plasmocytoma | ATCC # CRL 1484 | |
| BHK-21 | Baby Hamster Kidney | ATCC #CCL 10 | |

*American Type Culture Collection, 12301 Parklawn Drive, Rockville, Maryland 20852-1776

As indicated by Table II, many mammalian host cells possess the necessary cellular machinery for the recognition and proper processing of the signal peptide on the nascent proteins of the invention and provide the post-translational modifications, such as glycosylation, $\gamma$-carboxylation, and $\beta$-hydroxylation, as are observed in human protein C present in blood plasma. A wide variety of vectors, discussed below, exists for the transformation of such eukaryotic host cells, but the specific vectors exemplified below are in no way

19

EP 0 323 149 B1

intended to limit the scope of the present invention.

The pSV2-type vectors comprise segments of the SV40 genome that constitute a defined eukaryotic transcription unit--promoter (ep), intervening sequence (IVS), and polyadenylation (pA) site. In the absence of SV40 T-antigen, the plasmid pSV2-type vectors transform mammalian and other eukaryotic host cells by integrating into the host cell chromosomal DNA. A variety of plasmid pSV2-type vectors have been constructed (see Eukaryotic Viral Vectors, edited by Gluzman, published by Cold Spring Harbor Laboratories, Cold Spring Harbor, New York, 1982), such as plasmids pSV2-gpt, pSV2-neo, pSV2-dhfr, pSV2-hyg, and pSV2-$\beta$-globin, in which the SV40 promoter drives transcription of an inserted gene. These vectors are suitable for use with the coding sequences of the invention and are available either from the American Type Culture Collection (ATCC) in Rockville, Maryland or from the Northern Regional Research Laboratory (NRRL) in Peoria, Illinois.

Plasmid pSV2-dhfr (ATCC 37146) comprises a murine dihydrofolate reductase (dhfr) gene under the control of the SV40 early promoter. Under the appropriate conditions, the dhfr gene is known to be amplified, or copied, in the host chromosome. This amplification, described in a review article by Schimke, 1984, Cell 37:705-713, can involve DNA sequences closely contiguous with the dhfr gene, such as a nascent human protein C-encoding sequence of the invention, and thus can be used to increase production of the protein C zymogens of the invention.

Plasmids which were constructed for expression of the nascent protein C and protein C zymogens of the invention in mammalian and other eukaryotic host cells can utilize a wide variety of promoters. The present invention is in no way limited to the use of the particular eukaryotic promoters exemplified herein. Promoters such as the SV40 late promoter or the eukaryotic promoters disclosed in Bucher et al., 1986, Nuc. Acids Res. 14(24):1009, or promoters from eukaryotic genes, such as, for example, the estrogen-inducible chicken ovalbumin gene, the interferon genes, the glucocorticoid-inducible tyrosine aminotransferase gene, the thymidine kinase gene, and the major early and late adenovirus genes, can be readily isolated and modified for use on recombinant DNA expression vectors designed to produce human protein C zymogen in eukaryotic host cells. Eukaryotic promoters can also be used in tandem to drive expression of a coding sequence of the invention. Furthermore, a large number of retroviruses are known that infect a wide range of eukaryotic host cells. The long terminal repeats in the retrovirus DNA often encode promoter activity and thus can be used to drive expression of the coding sequences of the invention.

Plasmid pRSVcat (ATCC 37152) comprises portions of the long terminal repeat of the Rous Sarcoma virus (RSV), a virus known to infect chicken and other host cells. The RSV long terminal repeat sequences can be isolated on an ~0.76 kb NdeI-HindIII restriction fragment of plasmid pRSVcat. The promoter in the RSV long terminal repeat (Gorman et al., 1982, P.N.A.S. 79:6777) is suitable for use in vectors of the invention. Plasmid pMSVi (NRRL B-15929) comprises the long terminal repeats of the Murine Sarcoma virus (MSV), a virus known to infect mouse and other host cells. These repeat sequences are suitable for use as a promoter in the vectors of the invention. The mouse metallothionein (MMT) promoter has also been well characterized for use in eukaryotic host cells and is suitable for use in the vectors of the invention. The MMT promoter is present in the 15 kb plasmid pdBPV-MMTneo (ATCC 37224), which can serve as the starting material for the construction of other plasmids of the present invention.

Many modifications and variations of the present illustrative DNA sequences and plasmids are possible. For example, the degeneracy of the genetic code allows for the substitution of nucleotides throughout polypeptide coding regions, as well as in the translational stop signal, without alteration of the encoded polypeptide coding sequence. Such substitutable sequences can be deduced from the known amino acid or DNA sequence of human protein C and can be constructed by following conventional synthetic or site specific mutagenesis procedures. Synthetic methods can be carried out in substantial accordance with the procedures of Itakura et al., 1977 Science 198:1056 and Crea et al., 1978, Proc. Nat. Acad. Sci. USA 75:5765. Therefore, the present invention is in no way limited to the DNA sequences and plasmids specifically exemplified.

After transformation of a vector of the invention into a eukaryotic host cell, one can select transformants on the basis of a selectable phenotype. This selectable phenotype can be conferred either by a selectable marker present on the expression vector or present on another vector cotransformed with the expression vector into the host cell. Once transformants are selected, it is desirable to identify which transformants are expressing the highest levels of the desired protein encoded on the expression vector. Such identification is especially important after a cotransformation procedure, which generates a number of transformants that contain only the plasmid containing the selectable marker and so do not contain the expression vector. In Example 6, below, a protocol not only for identifying cells that express and secrete a desired protein but also for quantifying, relative to the other cells examined using the method, the amount of protein secreted is described. The protocol also allows for the isolation of viable cells secreting the highest levels of a desired

20

protein.

Activated protein C has substantial antithrombotic properties in the prevention of extension of intravenous thrombi, in the prevention of formation of arterial thrombi, and in the prevention of death and organ failure from Gram negative sepsis, endotoxemia, and disseminated intravascular coagulation. In animal experiments, infusion of native zymogen protein C was without effect in the treatment of Gram negative septicemia with shock and disseminated intravascular coagulation (DIC). These negative results indicated that in this form of widespread microvascular thrombosis involving massive thrombin generation, insufficient thrombomodulin was present to complex with thrombin and activate the infused zymogen.

The major disadvantage of activated protein C, as with any activated serine protease, is its short half-life ($T\frac{1}{2}$) as compared to the zymogen precursor. The $T\frac{1}{2}$ in dogs was established to be 11 minutes and the $T\frac{1}{2}$ in monkeys to be 22 to 26 minutes. In comparison, the $T\frac{1}{2}$ of native protein C zymogen in man is estimated at 6 hours. The reason for the shorter biological half lives of activated serine proteases, including activated protein C, as compared to their zymogens, are complex and involve both cellular and humoral mechanisms. Activated serine proteases also form complexes with serine protease inhibitors normally present in plasma. Activated protein C (APC) complexes with a newly described APC inhibitor as well as with alpha-2 macroglobulin. The inactive zymogens, including the protein C zymogens of the invention, do not react with serine protease inhibitors.

The advantage of the protein C zymogens of this invention is that they are better activated by thrombin than native protein C zymogen, because thrombin no longer has an absolute requirement for complexing with thrombomodulin to activate these zymogens in the presence of $Ca^{2+}$. It follows that these protein C zymogens, when administered, can be activated at sites of intravascular thrombin generation, i.e., at any site where an intravascular thrombus is under developement. Thus, these recombinant protein C zymogens can be used as pro drugs and will become activated only at the sites of thrombin generation. Because these thrombin-sensitive zymogens can be administered in the zymogen form, they will not complex with protein C inhibitors and will exhibit a biological half-life equal to that of native protein C zymogen.

The recombinant protein C zymogens of the invention are useful in the prevention and treatment of a wide variety of acquired disease states involving intravascular coagulation, including deep vein thrombosis, pulmonary embolism, peripheral arterial thrombosis, emboli originating from the heart or peripheral arteries, acute myocardial infarction, thrombotic strokes, and dis-seminated intravascular coagulation. These protein C derivatives can also be used efficiently in the treatment of the significant numbers of patients with heterozygous protein C deficiencies presenting recurrent deep vein thrombosis and in the case of the homozygous protein C deficient patients with purpura fulminans.

Experimental and clinical data suggest that conventional anticoagulants, particularly warfarin, are useful in the treatment of invasive cancers and act to prevent or reduce the distant metastatic lesions of these malignancies. In addition, it is well established that inflammatory stimuli, such as endotoxins, tumor necrosis factor, and interleukin 1, deplete thrombomodulin from the surface of endothelial cells, which is thought to trigger microvascular and macrovascular thrombosis. The recombinant protein C zymogens of the invention represent an attractive alternative to conventional anticoagulants in these clinical situations.

The doses of the protein C zymogens of the invention, because of their prolonged $T\frac{1}{2}$, can be substantially reduced in clinical situations, as compared to activated protein C. In homozygous protein C deficiency, the dose of a protein C zymogen of the invention will range from about 5 mg to 100 mg per treatment, and in heterozygous protein C deficiency, the dose will range from about 2.5 mg to 50 mg per treatment.

An attractive therapeutic indication for activated protein C is in the prevention of deep vein thrombosis and pulmonary embolism, currently treated with low doses of heparin. In high risk patients, particularly patients undergoing surgery, the dose of recombinant activated protein C for prevention of deep vein thrombosis is in the range from 1-10 mg/day. The dose of a protein C zymogen of the invention will range from about 0.25 to 5 mg per day. The added advantage of these zymogens is that they may be given as bolus injections rather than constant IV infusions. Activated protein C must be given by continuous IV infusion because of the short $T\frac{1}{2}$ of that protein. In established, objectively-documented, deep vein thrombosis and/or pulmonary embolism, the dose of activated protein C ranges from 1 - 10 mg as a loading dose followed by a continuous infusion in amounts ranging from 3-30 mg/day. The protein C zymogens of the invention, on the other hand, may be given by repeated bolus injection in doses not to exceed about 12 mg per 24 hours.

Similar dosage schedules are applicable for the treatment of peripheral arterial thrombi. There is a lower likelihood of bleeding complications from infusions of the protein C zymogens of the invention. Thus, these zymogens can replace heparin intra- and post-surgically in conjunction with thrombectomies or embolectomies, surgical procedures which are often necessary to save ischemic limbs from amputation in the setting of an acute arterial obstruction. Because of their long T½, as compared to activated protein C, and their relative ease of administration, these zymogens are better suited than activated protein C for the treatment of arterial emboli originating from the heart. The long term administration of these zymogens in doses comparable to those used for the treatment of established deep vein thrombois-pulmonary embolism has substantial utility in the prevention of cardiogenic emboli.

Similarly, the protein C zymogens of the invention can be used for the treatment of emboli originating from thrombi in peripheral arteries, most notably the carotid arteries, which are not treated or prevented satisfactorily with currently used regimens, which include drugs capable of suppressing platelet function, oral anticoagulants, or combinations thereof. As in the case of cardiogenic emboli, these zymogens can be administrated long term in the same manner as outlined for cardiogenic emboli and have major potential in the prevention of emboli originating from carotid artery thrombi and resulting in embolic strokes.

The protein C zymogens of the invention are also useful in thrombotic strokes. Today, strokes are not usually treated with conventional anticoagulants. Treatment of strokes with either heparin or oral anticoagulants, although occasionally beneficial, carries a high risk for bleeding into the infarcted brain area, thereby aggravating the neurological deficit accompanying the stroke. Because of their low potential for causing bleeding complications and their selectivity, the zymogens of the invention can be given to stroke victims and can be beneficial in preventing the local extension of the occluding arterial thrombus, thereby reducing the neurological deficit resulting from the stroke. The amount of the zymogen effective in the treatment of stroke will be lower, as compared with activated protein C, but the dose will vary with each patient depending on the nature and severity of the stroke.

The zymogens of the invention will also be useful in treating acute myocardial infarction, because of their pro-fibrinolytic properties, once activated. These zymogens can be given with tissue plasminogen activator during the acute phases of the myocardial infarction. After the occluding coronary thrombus is dissolved, the zymogens can be given for additional days to prevent acute myocardial reinfarction. If activated protein C is administered in this situation, the patient is given a loading dose of 1-10 mg at the time plasminogen activator treatment is initiated followed by a continuous infusion of activated protein C ranging from 3-30 mg/day. In contrast, the zymogens of the invention can be given through bolus injection 3 to 4 times a day in doses not to exceed about 12 mg/day.

Activated protein C is useful in the treatment of disseminated intravascular coagulation. Heparin and the oral anticoagulants have been given to patients with disseminated intravascular coagulation (DIC) in extensive clinical trials, but the results have been disappointing. In disseminated intravascular coagulation, activated protein C, as well as the zymogens of the present invention, has a distinct advantage over conventional anticoagulants. As mentioned above, it has been established in animal experiments that the protein C zymogen is ineffective in the prevention of death and organ damage from Gram negative septicemia and disseminated intravascular coagulation. In contrast, the protein C zymogens of the invention, being highly susceptible to activation by thrombin, will be effective treatment for disseminated intravascular coagulation. The estimated requirements for activated protein C to treat DIC is approximately 100 mg/day; the doses of the zymogen forms of the invention for treatment of DIC are not to exceed about 30 mg/day, administered as repeated bolus injections.

Conventional anticoagulant drugs, particularly warfarin, are useful in the treatment of invasive malignant tumors. Many tumor cells produce substances which trigger the activation of the coagulation system resulting in local fibrin deposits. These fibrin deposits function as "nests" in which cancer cells can divide to form metastatic lesions. However, it is not possible to administer warfarin or other conventional anticoagulants in combination with the more intensive and effective forms of chemotherapy, because such therapy always produces a sharp drop in the platelet count, and thrombocytopenia combined with warfarin therapy puts the patient at an unacceptably high risk for serious bleeding complications. The protein C derivatives of the invention, like activated protein C, being more selective than conventional anticoagulants and having a far higher therapeutic index than either heparin or the oral anticoagulants, can be given relatively safely to the thrombocytopenic patient, thus making possible the treatment of patients with invasive cancers with effective and intensive chemotherapy in combination with a protein C zymogen of the invention. Treatment will follow a dosage regimen comparable to that used in deep vein thrombosis-pulmonary embolism.

The zymogens, and activated counterparts, of the present invention can be formulated according to known methods to prepare pharmaceutically useful compositions, whereby a human protein C zymogen or activated protein C of the invention is combined in admixture with a pharmaceutically acceptable carrier vehicle. Suitable carrier vehicles and their formulation, inclusive of other human proteins, e.g., human serum albumin, are described, for example, in Remington's Pharmaceutical Sciences 16th ed., 1980, Mack Publishing Co., edited by Osol et al., which is hereby incorporated by reference. Such compositions will contain an effective amount of a protein C zymogen, or activated counterpart, together with a suitable amount of carrier vehicle to prepare pharmaceutically acceptable compositions suitable for effective administration to the host. The protein C composition can be administered parenterally, or by other methods that ensure its delivery to the bloodstream in an effective form.

It should also be noted that the zymogens of the present invention can be used to prepare activated protein C in vitro. Although recombinant methods for producing activated protein C directly in eukaryotic cells are known, these methods require that the activated protein C remain in the culture media for long periods of time. In addition, the activated protein C must be purified from the culture medium, an expensive, multi-step process. Because activated protein C is relatively unstable, these direct expression methods can yield low amounts of activated protein C. In contrast, the zymogens of the invention can be activated by thrombin alone, even in the presence of $Ca^{2+}$, and thus offer significant advantages over known methods for producing activated protein C.

The following Examples illustrate the methods and describe the construction protocols for representative compounds, vectors and transformants of the invention without limiting the same thereto.

Example 1

Construction of Plasmid pLAPC

This Example provides a detailed protocol for the construction of plasmid pLAPC. In brief, Example 1A describes the isolation of a DNA fragment encoding a portion of the protein C molecule, including the activation peptide, from plasmid pHC7. Example 1B describes the cloning of this DNA fragment into phage M13mp18 and the removal of the DNA encoding the activation peptide from the resulting recombinant phage by site specific mutagenesis. Example 1C describes the final steps in the construction of plasmid pLAPC, more specifically, the isolation of the mutagenized fragment and its ligation with two fragments derived from plasmid pLPC to yield plasmid pLAPC. The construction protocol for plasmid pLPC is described in Example 2.

A. Isolation of a DNA Fragment Containing the Coding Sequence for the Activation Peptide of Human Protein C

Plasmid pHC7 contains the complete coding sequence for nascent human protein C. One liter of L broth (10 g peptone, 10 g NaCl, and 5 g yeast extract) containing 15 $\mu$g/ml tetracycline was inoculated with a culture of E. coli K12 RR1/pHC7 (NRRL B-15926) and incubated in an air-shaker incubator at 37°C until the optical density (O.D.) at 590 nm was ~1 absorbance unit, at which time 150 mg of chloramphenicol were added to the culture. The incubation was continued for about 16 hours; the chloramphenicol addition inhibits protein synthesis, and thus inhibits further cell division, but allows plasmid replication to continue.

The culture was centrifuged in a Sorvall GSA rotor (DuPont Co., Instrument Products, Biomedical Division, Newtown, CN 06470) at 6000 rpm for 5 minutes at 4°C. The resulting supernatant was discarded, and the cell pellet was washed in 40 ml of TES buffer (10 mM Tris-HCl, pH = 7.5; 10 mM NaCl; and 1 mM EDTA) and then repelleted. The supernatant was again discarded, and the cell pellet was frozen in a dry ice-ethanol bath and then thawed. The thawed cell pellet was resuspended in 10 ml of a 25% sucrose/50 mM EDTA solution. About one ml of a 5 mg/ml lysozyme solution; 3 ml of 0.25 M EDTA, pH = 8.0; and 100 $\mu$l of 10 mg/ml RNAse A were added to the solution, which was then incubated on ice for 15 minutes. Three ml of lysing solution (prepared by mixing 3 ml 10% Triton-X 100; 75 ml 0.25 M EDTA, pH = 8.0; 15 ml of 1 M Tris-HCl, pH = 8.0; and 7 ml of water) were added to the lysozyme-treated cells, mixed, and the resulting solution incubated on ice for another 15 minutes. The lysed cells were frozen in a dry ice-ethanol bath and then thawed.

The cellular debris was removed from the solution by centrifugation at 25,000 rpm for 40 minutes in an SW27 rotor (Beckman, 7360 N. Lincoln Ave., Lincolnwood, IL 60646). About 30.44 g of CsCl and ~1 ml of a 5 mg/ml ethidium bromide solution were added to the solution, the volume of which was then adjusted to 40 ml. The solution was decanted into a Vti50 ultra-centrifuge tube (Beckman). The tube was sealed and then centrifuged in a Vti50 rotor at 42,000 rpm for ~16 hours. The resulting plasmid band, visualized with ultraviolet light, was isolated and then placed in a ti75 tube and rotor (Beckman) and centrifuged at 55,000 rpm for 16 hours. Any necessary volume adjustments were made using TES containing 0.761 g/ml CsCl. The plasmid band was again isolated, the ethidium bromide extracted with salt-saturated isopropanol, and finally diluted 1:3 with TES buffer. Two volumes of ethanol were then added to the solution, and the resulting mixture was incubated overnight at -20°C. The plasmid DNA was pelleted by centrifuging the solution in an SS34 rotor (DuPont Co.) for 15 minutes at 10,000 rpm.

The ~1 mg of plasmid pHC7 DNA obtained by this procedure was suspended in 1 ml of TE buffer (10 mM Tris-HCl, pH = 7.6, and 0.1 mM EDTA) and stored at -20°C. A restriction site and function map of plasmid pHC7 is presented in Figure 2 of the accompanying drawings.

About 7 μg (7 μl) of plasmid pHC7 DNA were added to 25 μl of 10X Core buffer™ (Core buffer™, BRL, is 500 mM Tris-HCl, pH = 8.0; 500 mM NaCl; and 100 mM MgCl₂), 198 μl of H₂O, and 12 μl of restriction enzyme SstI (~60 units, Bethesda Research Laboratories (BRL), Gaithersburg, MD 20877; all enzymes referred to in these Examples are available, unless otherwise indicated, from BRL or New England Biolabs (NEB), Beverly, MA 01915-9990, and are used in substantial accordance with the manufacturer's recommendations), and 8 μl (80 units) of restriction enzyme SalI. The reaction mixture was incubated at 37°C for four hours; then, the SstI-SalI digested plasmid pHC7 DNA was extracted first with phenol and then with chloroform, collected by precipitation with ethanol and centrifugation, and finally suspended in 15 μl of TE/10 buffer (10 mM Tris-base, pH = 7.6, and 0.1 mM EDTA) buffer.

The reaction mixture was then electrophoresed on an ~0.6% low-gelling-temperature agarose (FMC Corporation, Marine Colloids Division, Rockland, Maine 04841) gel for 2-3 hours at ~130 V and ~65 mA in Tris-Acetate buffer. The gel was stained in a dilute solution of ethidium bromide, and the band of DNA constituting the ~0.7 kb SstI-SalI restriction fragment, which was visualized with long-wave UV light, was cut from the gel in a small segment. The volume of the segment was determined by weight and density of the segment, and four volumes of TE containing 0.25 M NaCl were added to the tube containing the segment. The segment was then melted by incubation at 72°C. About 0.5 μg of the ~0.7 kb SstI-SalI restriction fragment of plasmid pHC7 was obtained in a volume of about 400 μl. Further purification of the DNA was obtained by passing the solution of DNA through a NACS-prepac® column (BRL) in accordance with the manufacturer's recommendations; the purified fragment was resuspended in 15 μl of deionized water.

B. Construction of Recombinant Phage and Removal of the Activation Peptide-encoding DNA by Site-Specific Mutagenesis

About 1 μg of phage M13mp18 (obtained from New England Biolabs) RF (replicative form) DNA was digested with restriction enzymes SstI and SalI in substantial accordance with the procedure described in Example 1A. The reaction was stopped by extracting the reaction mixture with phenol and then chloroform; then, the DNA was precipitated, collected by centrifugation, and resuspended in about 15 μl of TE buffer. The two fragments resulting from the digestion were separated on an ~0.6% low-gelling-temperature agarose gel, and the larger fragment was cut out from the gel and purified as described in Example 1A.

About 0.1 μg (in 7 μl of H₂O) of the ~0.7 kb SstI-SalI restriction fragment of plasmid pHC7 was added to 5 μl of the SstI-SalI-digested M13mp18 RF DNA together with 2 μl of 10X ligase buffer (0.5 M Tris-HCl, pH = 7.8; 60 mM MgCl₂; and 0.2 M dithiothreitol (DTT)), 2 μl of 1 mg/ml BSA, 1 μl of 25 mM ATP, 1 μl (~400 units) of T4 DNA ligase (NEB), and 2 μl of H₂O. The ligation reaction was incubated at 25°C overnight; the ligated DNA constituted the desired phage M13mp18-HE1 DNA in double-stranded form.

About 300 μl of an overnight culture of E. coli K12 JM101 (New England Biolabs) were used to inoculate 30 ml of 2X TY broth (TY broth is 10 g/L tryptone, 10 g/L NaCl, and 5 g/L yeast extract), and the resulting culture was incubated at 37°C with aeration until the O.D.₆₀₀ was ~0.5. The culture was chilled for 10 minutes in an ice-water bath, collected by centrifugation, and resuspended in 15 ml of cold, 10 mM NaCl. The cells were again collected by centrifugation and then resuspended in 15 ml of cold, 30 mM CaCl₂. The cells were placed on ice for 20 minutes and collected by centrifugation. The cells were resuspended in 1.5 ml of cold, 30 mM CaCl₂; a 200 μl aliquot of the cells was removed, added to 9 μl of the ligated DNA prepared above, and incubated on ice for about 30 minutes. The cell-DNA mixture was then incubated at 42°C for 2 minutes and then added to 3 ml of top agar (TY broth with 0.5% agar kept molten at 45°C) that also contained 50 μl of 2% X-Gal ("X-Gal" is 5-Bromo-4-chloro-3-indolyl-β-D-galactopyranoside), 50 μl of 100 mM IPTG ("IPTG" is isopropyl β-D-thiogalactopyranoside), and 100 μl of E. coli K12 JM101 in logarithmic growth phase. The cell-top agar mixture was then plated on TY-agar plates, and the plates were incubated at 37°C overnight.

The following morning, four clear plaques were individually used to inoculate 2 ml of 2X TY broth, and the resulting cultures were incubated at 37°C with aeration for 6 hours. Then, the cultures were centrifuged, and 500 μl of the resulting supernatant (the cell pellets were used to prepare phage DNA for restriction enzyme analysis) were added to 500 μl cultures (O.D.₅₅₀ = 0.5) of E. coli K12 JM101 and 50 ml of 2X TY broth. These cultures were incubated overnight at 37°C. The phage RF DNA was isolated from the cell pellets using a scaled-down version of the procedure described in Example 1A, except that no antibiotic was used in the culture media, and the ultracentrifugation steps were replaced with phenol and chloroform extractions. Transformants containing phage M13mp18-HE1 DNA were identified by restriction enzyme analysis of their phage DNA.

The overnight cultures were centrifuged, and about 1 ml of a solution composed of 20% polyethylene glycol (PEG) 6000 and 2.5 mM NaCl was added per 5 ml of supernatant, which was then incubated at room temperature for 10 minutes. The mixture was centrifuged for 10 minutes at 10,000 r.p.m., and the resulting pellet, which contained single-stranded phage M13mp18-HE1 DNA, was resuspended in 500 $\mu$l of TES buffer (20 mM Tris-HCl, pH = 7.5; 0.1 M EDTA; and 10 mM NaCl). The DNA solution was extracted first with chloroform, then twice with TE-saturated phenol, and then again with chloroform. The single-stranded DNA was then precipitated using NaOAc and ethanol, centrifuged, and, after the pellet was washed with 70% ethanol and dried, the resulting pellet was dissolved in 80 $\mu$l of $H_2O$. This phage preparation was used in the next step, the site-specific mutagenesis, to remove the activation peptide-encoding DNA.

The single-stranded DNA fragment used in the mutagenesis to remove the activation peptide-encoding DNA was synthesized on an automated DNA synthesizer and is depicted below:

5′-GCGCAGTCACCTGAAACGACTCATTGATGGGAAGATGA-3′

About 30 picomoles (1 $\mu$l) of the single-stranded DNA fragment depicted above (the "mutagenic oligonucleotide") and 1.5 $\mu$l (7.5 picomoles) of the M13 universal primer (marketed by Boehringer-Mannheim Biochemicals (BMB), 7941 Castleway Drive, P.O. Box 50816, Indianapolis, IN 46250) were individually treated with 5 units (Pharmacia, P-L Biochemicals, Inc., 800 Centennial Avenue, Piscataway, NJ 08854) of T4 polynucleotide kinase in 10 $\mu$l of 1X kinase buffer (100 mM Tris-HCl, pH = 8.3; 100 mM DDT; and 100 mM $MgCl_2$) containing 1 $\mu$l of 1 mM ATP for 30 minutes at 37°C, followed by a 10 minute, 65°C incubation and subsequent freezing. The kinase-treated DNAs were used in the mutagenesis procedure described below.

In the first step of the mutagenesis procedure, the mutagenic oligonucleotide and the M13 universal primer were annealed to the single-stranded phage DNA. The annealing reaction was carried out by adding 300 nanograms (0.5 $\mu$l) of single-stranded phage M13mp18-HE1 to 1 picomole (1.2 $\mu$l) of the universal primer, 1 picomole (0.3 $\mu$l) of the mutagenic oligonucleotide, 2 $\mu$l of 10X annealing buffer (100 mM Tris-HCl, pH = 7.5; 1 mM EDTA; and 500 mM NaCl), and 16 $\mu$l of $H_2O$, incubating the mixture at 80°C for 2 minutes and then at 50°C for 5 minutes, and, finally, allowing the mixture to cool to room temperature.

Once the oligonucleotides were annealed, the phage DNA was made double-stranded by extending the primers with DNA polymerase. The extension reaction was carried out by adding 3 $\mu$l of 10X extension buffer (500 mM Tris-HCl, pH = 8; 1 mM EDTA; and 120 mM $MgCl_2$); 3 $\mu$l of 10X ligase buffer; 1.5 $\mu$l of 0.2 mM DTT; 3 $\mu$l of dNTP mix (0.5 mM in each dNTP); 1.2 $\mu$l of 25 mM ATP; 0.5 $\mu$l of Klenow enzyme (5 U/$\mu$l, BMB); 1 $\mu$l of T4 DNA ligase (400 U, NEB); and 19.8 $\mu$l of $H_2O$ to the mixture of annealed DNA. The extension reaction was incubated at room temperature for 30 minutes, then at 37°C for 4 hours, and then overnight at 4°C.

The reaction was stopped by a phenol-chloroform extraction and precipitation of the DNA with ethanol and sodium acetate (NaOAc). The DNA was collected by centrifugation and resuspended in 40 $\mu$l of S1 buffer (0.3 M NaCl; 0.03 M NaOAc, pH = 4.5; and 0.3 mM $ZnCl_2$) were then added to the solution of DNA. The S1 treatment described below has been reported to be beneficial in site-specific mutagenesis procedures. However, the present inventors found no significant advantage in the S1 treatment and, in the construction protocols described in subsequent Examples herein, omitted the S1 treatment entirely.

The solution of DNA was split equally into two tubes, and to one of the tubes, 100 units (BMB) of S1 nuclease were added. The S1 reaction was incubated at room temperature for 5 minutes and stopped by extracting the reaction mixture once with TE-saturated phenol-chloroform (50:50). The DNA was precipitated from the reaction mixture and from the non-S1-treated sample with NaOAc and ethanol.

The DNA pellets were resuspended in 60 $\mu$l of $H_2O$ and used to transform E. coli K12 JM101 in accordance with the procedure used during the construction of phage M13mp18-HE1, except that no IPTG or X-Gal was added to the plates. The mutants were screened for by using a small portion of the mutagenic oligonucleotide, 5′-TGAAACGACTCATTGA-3′ (radioactively labelled), as a probe in plaque and dot-blot hybridizations. Several plaques that appeared positive from the hybridizations were picked and individually inoculated into 2 ml of a culture of E. coli K12 JM101 in logarithmic growth phase. These cultures were incubated at 37°C with aeration for about 6 hours, when they were then used to prepare single-stranded DNA as described above for phage M13mp18-HE1.

The single-stranded DNA was sequenced using the dideoxy-sequencing method (J.H. Smith, 1980, Methods in Enzymology 65:560-580). Several phage were identified with the desired mutation. Phage in which the coding sequence for the activation peptide was deleted were designated phage M13mp18-HE2. The mutation in phage M13mp18-HE2 causes a decrease in size of 36 bp with respect to the natural coding sequence, a difference that can be used to facilitate identification of DNA that contains the mutated region.

The RF form of phage M13mp18-HE2 was prepared for use in subsequent constructions.

C. Final Construction of Plasmid pLAPC From Phage M13mp18-HE2 and Plasmid pLPC

The mutagenized SstI-SalI (~0.7 kb) restriction fragment of the RF form of phage M13mp18-HE2 was cut from the phage and isolated in substantial accordance with the procedure of Example 1A. However, the ~100 $\mu$l of solution containing ~0.1 $\mu$g of the desired 0.7 kb fragment in a 1:2 dilution of low-gelling agarose were not passed through any purification column but were used directly in the ligation to produce plasmid pLAPC, described below.

Three DNA fragments were ligated together to form plasmid pLAPC: the ~0.7 kb SstI-SalI restriction fragment of phage M13mp18-HE2, described above, and two DNA fragments from plasmid pLPC. The construction protocol for plasmid pLPC is described in Example 2. A restriction site and function map of plasmid pLPC is presented in Figure 1 of the accompanying drawings. Because of the positioning of SalI, SstI, and EcoRI restriction enzyme recognition sites on plasmid pLPC, the desired EcoRI-SalI and EcoRI-SstI restriction fragments had to be prepared in two separate digestions.

To prepare the EcoRI-SstI fragment, about 40 $\mu$g of plasmid pLPC in 25 $\mu$l of $H_2O$ were added to 10 $\mu$l of 1 mg/ml BSA, 10 $\mu$l of 10X Core buffer™ (BRL), 5 $\mu$l of restriction enzyme EcoRI (50 U, BRL), 5 $\mu$l of restriction enzyme SstI (25 U, BRL), and 45 $\mu$l of $H_2O$, and the resulting reaction was incubated at 37°C for 1.5 hours. The SstI-EcoRI-digested plasmid pLPC DNA was collected by precipitation with ethanol and centrifugation. The SstI-EcoRI-digested DNA was resuspended in water and then loaded onto an ~0.6% low-gelling-temperature agarose gel to separate the DNA fragments by electrophoresis.

To prepare the EcoRI-SalI fragment, about 15 $\mu$g of plasmid pLPC in 9 $\mu$l of $H_2O$ were first treated with restriction enzyme ApaI to eliminate contamination by similarly-sized restriction fragments. About 10 $\mu$l of 10X ApaI buffer (60 mM NaCl; 60 mM Tris-HCl, pH = 7.4; 60 mM $MgC_2$; and 60 mM DTT), 10 $\mu$l of 1 mg/ml BSA, 69 $\mu$l of $H_2O$, and 2 $\mu$l of restriction enzyme ApaI (50 U, NEB) were added to the solution of plasmid pLPC DNA, and the resulting reaction was incubated at 37°C for one hour. Then, 15 $\mu$l of 2 M NaCl, 69 $\mu$l of $H_2O$, 8 $\mu$l of restriction enzyme SalI (NEB), and 8 $\mu$l of restriction enzyme EcoRI (NEB), were added to the solution of ApaI-digested plasmid pLPC DNA, and the resulting reaction was incubated at 37°C for one hour. The ApaI-SalI-EcoRI-digested plasmid pLPC DNA was extracted first with phenol and then with chloroform, then collected by precipitation with ethanol and centrifugation, and finally resuspended in 25 $\mu$l of $H_2O$. The DNA was then loaded onto an ~0.6% low-gelling-temperature agarose gel and the DNA fragments separated by electrophoresis.

The ~3.76 kb EcoRI-SalI and the ~2.0 kb EcoRI-SstI restriction fragments were cut from the gels and the gel fragments melted after adding equal volumes of 10 mM Tris-HCl, pH = 7.6, as described in Example 1A. About 2 $\mu$g of the ~3.76 kb EcoRI-SalI restriction fragment of plasmid pLPC were thus obtained in ~200 $\mu$l of 10 mM Tris-HCl, pH = 7.6, which also contained the melted agarose. About 2 $\mu$g of the ~2.0 kb EcoRI-SalI restriction fragment of plasmid pLPC were obtained in a separate ~200 $\mu$l of 10 mM Tris-HCl, pH = 7.6, containing agarose.

About 12.5 $\mu$l of each solution of the two purified restriction fragments (the ~3.76 kb EcoRI-SalI restriction fragment of plasmid pLPC and the ~2.0 kb EcoRI-SstI restriction fragment of plasmid pLPC) were added to 20 $\mu$l of the ~0.7 kb SstI-SalI restriction fragment of phage M13mp18-HE2, 10 $\mu$l of 1 mg/ml BSA, 10 $\mu$l of 10 mM ATP, 10 $\mu$l of 10X ligase buffer, 2 $\mu$l (~800 U, NEB) of T4 DNA ligase, and 23 $\mu$l of $H_2O$, and the resulting ligation reaction was incubated at 15°C overnight. The ligated DNA constituted the desired plasmid pLAPC. Plasmid pLAPC only differs from plasmid pLPC (Figure 1) in the deletion of the activation peptide-encoding DNA.

To check plasmid structure and obtain large amounts of plasmid pLAPC for eukaryotic cell transformation and further constructions, the ligated DNA containing plasmid pLAPC was used to transform E. coli K12 RV308, available from the NRRL under the accession number NRRL B-15624.

A 50 ml culture of E. coli K12 RV308 in L broth was grown to an optical density (O.D.) at 590 nm of ~0.6. The culture was chilled on ice for ten minutes, and the cells were collected by centrifugation. The cell pellet was resuspended in 25 ml of cold, 10 mM NaCl. The cells were again pelleted by centrifugation, and the pellet was resuspended in 25 ml of cold, 30 mM $CaCl_2$ and incubated on ice for 30 minutes. The cells were again collected by centrifugation and resuspended in 2.5 ml of cold, 30 mM $CaCl_2$.

Two hundred $\mu$l of this cell suspension were mixed with the ligated DNA containing plasmid pLAPC and incubated on ice for 60 minutes. The mixture was then incubated at 42°C for 2 minutes, followed by a 10 minute incubation at room temperature. About 10 ml of 2X TY broth were added to the cell-DNA mixture, and then the cells were incubated in an air-shaker incubator in a 125 ml flask at 37°C for two hours.

Aliquots of the cell mixture were plated on TY-agar (TY broth with 15 g/l agar) plates containing 100 μg/ml ampicillin, and the plates were then incubated at 37°C overnight. E. coli K12 RV308/pLAPC transformants were verified by restriction enzyme analysis of their plasmid DNA. Plasmid DNA was obtained from the E. coli K12 RV308/pLAPC transformants in substantial accordance with the teaching of Example 1A, except that 50 μg/ml of ampicillin, and not tetracycline, was used as the selective agent.

Example 2

The Construction of Plasmid pLPC

Plasmid pLPC was used as an intermediate vector in the construction of plasmid pLAPC (see Example 1C). Plasmid pLPC comprises a segment of DNA that encodes the BK virus enhancer and the adenovirus 2 late promoter positioned to drive expression of human protein C. The construction protocol for plasmid pLAPC essentially results in the replacement of the human protein C coding sequence on plasmid pLPC with another protein C coding sequence from which the activation peptide-encoding DNA has been removed.

The BK enhancer/adenovirus late promoter expression control sequences on plasmids pLPC and pLAPC are greatly stimulated in activity by the presence of an immediate early gene product of a large DNA virus, i.e., the E1A gene product of adenovirus.

The construction protocol for plasmid pLPC is set forth below. The entire construction protocol for plasmid pLPC is schematically illustrated in Figure 1 of the accompanying drawings. In brief, Example 2A describes the isolation of BK virus DNA, from which the BK enhancer can be obtained. Example 2B sets forth the construction protocol for plasmid pBKneol, a plasmid resulting from the insertion of the BK enhancer into plasmid pdBPV-MMTneo. Example 2C teaches the construction protocol for plasmid pLPcat, a plasmid resulting from the insertion of the adenovirus 2 late promoter into plasmid pSV2cat. Example 2D teaches the construction protocol for plasmid pBLcat, a plasmid that contains the BK enhancer positioned to stimulate the activity of the adenovirus late promoter. Example 2E describes the construction protocol for plasmid pL133, a protein C expression vector, beginning with starting material plasmid pHC7 and proceeding through the construction of intermediate plasmid pSV2-HPC8 and then the final construction of plasmid pL133. Finally, Example 2F teaches the construction protocol for plasmid pLPC, which comprises the BK enhancer/adenovirus late promoter expression control sequence of plasmid pBLcat inserted into plasmid pL133 to drive expression of human protein C.

A. Preparation of BK Virus DNA

BK virus is obtained from the American Type Culture Collection under the accession number ATCC VR-837. The virus is delivered in freeze-dried form and resuspended in Hank's balanced salts (Gibco, 3175 Staley Road, Grand Island, NY 14072) to a titer of about $10^5$ plaque-forming units (pfu)/ml. The host of choice for the preparation of BK virus DNA is primary human embryonic kidney (PHEK) cells, which can be obtained from Flow Laboratories, Inc., 7655 Old Springhouse Road, McLean, VA 22101, under catalogue number 0-100 or from M.A. Bioproducts under catalogue number 70-151.

About five 75 mm² polystyrene flasks comprising confluent monolayers of about $10^6$ PHEK cells are used to prepare the virus. About 1 ml of BK virus at a titer of $10^5$ pfu/ml is added to each flask, which is then incubated at 37°C for one hour, and then, fresh culture medium (Dulbecco's Modified Eagle Medium, Gibco, Grand Island, NY 14072, supplemented with 10% fetal bovine serum) is added, and the infected cells are incubated at 37°C for 10-14 days or until the full cytopathogenic effect of the virus is noted. This cytopathogenic effect varies from cell line to cell line and from virus to virus but usually consists of cells rounding up, clumping, and sloughing off the culture disk.

The virus is released from the cells by three freeze-thaw cycles, and the cellular debris is removed by centrifugation at 5000Xg. The virus in 1 liter of supernatant fluid is precipitated and collected by the addition of 100 g of PEG-6000, incubation of the solution for 24 hours at 4°C, and centrifugation at 5000Xg for 20 minutes. The pellet is dissolved in 0.1X SSC buffer (1XSSC = 0.15 M NaCl and 0.015 M NaCitrate, pH = 7) at 1/100th of the original volume. The virus suspension is layered onto a 15 ml solution of saturated KBr in a tube, which is centrifuged at 75,000Xg for 3 hours. Two bands are evident in the KBr solution after centrifugation. The lower band, which contains the complete virion, is collected and desalted on a Sephadex® G-50 column (Sigma Chemical Co., St. Louis, MO 63178) using TE (10 mM Tris-HCl, pH = 7.8, and 1 mM EDTA) as an elution buffer.

Sodium dodecyl sulfate (SDS) is added to the solution of purified virions obtained from the column to a concentration of 1%; pronase® (Sigma) protease is added to a concentration of 100 $\mu$g/ml, and the solution is incubated at 37°C for 2 hours. Cesium chloride is then added to the solution to a density of 1.56 g/ml, and ethidium bromide is added to the solution to a final concentration of 100 $\mu$g/ml. The solution is centrifuged in a Sorvall 865 rotor (DuPont Co., Newton, CT 06470) or similar vertical rotor at 260,000Xg for 24 hours. After centrifugation, the band of virus DNA is isolated and extracted five times with isoamyl alcohol saturated with 100 mM Tris-HCl, pH = 7.8. The solution of BK virus DNA is then dialyzed against TE buffer until the 260 nm/280 nm absorbance ratio of the DNA is between 1.75 and 1.90. The DNA is precipitated by adjusting the NaCl concentration to 0.15 M, adding two volumes of ethanol, incubating the solution at -70°C for at least 2 hours, and centrifuging the solution at 12,000Xg for 10 minutes. The resulting pellet of BK virus DNA is suspended in TE buffer at a concentration of 1 mg/ml. A restriction site and function map of BK virus is presented in Figure 1 of the accompanying drawings.

B. Construction of Plasmid pBKneo1

E. coli K12 HB101/pdBPV-MMTneo cells are obtained in lyophilized form from the American Type Culture Collection under the accession number ATCC 37224. The lyophilized cells are plated on L-agar plates containing 100 $\mu$g/ml ampicillin and incubated at 37°C to obtain single colony isolates.

One liter of L broth (10 g tryptone, 10 g NaCl, and 5 g yeast extract per liter) containing 50 $\mu$g/ml ampicillin was inoculated with a colony of E. coli K12 HB101/pdBPV-MMTneo and incubated in an airshaker at 37°C until the O.D.$_{590}$ was ~1 absorbance unit, at which time 150 mg of chloramphenicol were added to the culture. The incubation was continued for about 16 hours; the chloramphenicol addition inhibits protein synthesis, and thus inhibits further cell division, but allows plasmid replication to continue. Plasmid pdBPV-MMTneo DNA was then prepared from the culture in substantial accordance with the procedure described in Example 1A.

The ~1 mg of plasmid pdBPV-MMTneo DNA obtained by this procedure was suspended in 1 ml of TE buffer and stored at -20°C. The plasmid isolation procedure described in Example 1A is generally used when large amounts of very pure plasmid DNA are desired. The procedure can be modified to obtain rapidly a smaller, less pure amount of DNA, such as is needed when screening transformants for the presence of a given plasmid, by using only about 5 ml of cultured cells, lysing the cells in an appropriately scaled-down amount of lysis buffer, and replacing the centrifugation steps with phenol and chloroform extractions.

About 5 $\mu$g (5 $\mu$l) of the plasmid pdBPV-MMTneo DNA prepared as described above and five $\mu$g (5 $\mu$l) of the BK virus DNA prepared as described above were each digested at 37°C for 2 hours in a solution containing 2 $\mu$l of 10X BamHI buffer (1.5 M NaCl; 60 mM Tris-HCl, pH=7.9; 60 mM MgCl$_2$; and 1 mg/ml BSA), 1 $\mu$l (~10 units) of restriction enzyme BamHI, and 7 $\mu$l of H$_2$O. The reaction was stopped by an extraction with an equal volume of phenol, followed by two extractions with chloroform. Each BamHI-digested DNA was then precipitated, collected by centrifugation, and resuspended in 5 $\mu$l of H$_2$O.

About 1 $\mu$l of 10X ligase buffer was added to a mixture of BamHI-digested plasmid pdBPV-MMTneo (1 $\mu$l) and BamHI-digested BK virus DNA (1 $\mu$l). After 1 $\mu$l (~5 units) of T4 DNA ligase and 6 $\mu$l of H$_2$O were added to the mixture of DNA, the resulting reaction was incubated at 16°C overnight. The ligated DNA constituted the desired plasmids pBKneoI and pBKneo2, which differ only with respect to the orientation of the BK virus DNA. A restriction site and function map of plasmid pBKneo1 is presented in Figure 1 of the accompanying drawings.

E. coli K12 HB101 cells are available in lyophilized form from the Northern Regional Research Laboratory under the accession number NRRL B-15626. A 50 ml culture of E. coli K12 HB101 in L broth was grown to an optical density at 650 nanometers (O.D.$_{650}$) of approximately 0.4 absorbance units. The culture was chilled on ice for ten minutes, and the cells were collected by centrifugation. The cell pellet was resuspended in 25 ml of cold 100 mM MgCl$_2$ and incubated on ice for 25 minutes. The cells were once again pelleted by centrifugation, and the pellet was resuspended in 2.5 ml of cold 100 mM CaCl$_2$ and incubated for 30 minutes on ice. After the incubation, the cells are competent for the uptake of transforming DNA.

Two hundred $\mu$l of this cell suspension were mixed with the ligated DNA prepared above and incubated on ice for 30 minutes. At the end of this period, the cells were placed in a water bath at 42°C for 2 minutes and then returned to the ice for an additional 10 minutes. The cells were collected by centrifugation and resuspended in one ml of L broth and incubated at 37°C for 1 hour. The transformed cells were plated on L-agar plates containing 100 $\mu$g/ml ampicillin. E. coli K12 HB101/pBKneo1 and E. coli K12/pBKneo2 transformants were identified by their ampicillin-resistant phenotype and by restriction enzyme analysis of

28

their plasmid DNA. A restriction site and function map of plasmid pBKneo1 is presented in Figure 1, Part A, of the accompanying drawings.

C. Construction of Plasmid pLPcat, an Intermediate Plasmid Used in the Construction of Plasmid pBLcat

The virion DNA of adenovirus 2 (Ad2) is a double-stranded linear molecule about 35.94 kb in size. The Ad2 late promoter can be isolated on an ~0.32 kb AccI-PvuII restriction fragment of the Ad2 genome; this ~0.32 kb restriction fragment corresponds to the sequence between nucleotide positions 5755 and 6071 of the Ad2 genome. To isolate the desired ~0.32 kb AccI-PvuII restriction fragment, Ad2 DNA is first digested with restriction enzyme BalI, and the ~2.4 kb BalI restriction fragment that comprises the entire sequence of the ~0.32 kb AccI-PvuII restriction fragment is isolated. Then, the ~2.4 kb BalI restriction fragment is digested with AccI and PvuII to obtain the desired fragment.

About 50 $\mu$g of Ad2 DNA (available from BRL) are dissolved in 80 $\mu$l of $H_2O$ and 10 $\mu$l of 10X BalI buffer (100 mM Tris-HCl, pH = 7.6; 120 mM $MgCl_2$; 100 mM DTT; and 1 mg/ml BSA). About 10 $\mu$l (~20 units) of restriction enzyme BalI are added to the solution of Ad2 DNA, and the resulting reaction is incubated at 37°C for 4 hours.

The BalI-digested DNA is loaded onto an agarose gel and electrophoresed until the restriction fragments are well separated. Visualization of the electrophoresed DNA is accomplished by staining the gel in a dilute solution (0.5 $\mu$g/ml) of ethidium bromide and exposing the stained gel to long-wave ultraviolet (UV) light. One method to isolate DNA from agarose is as follows. A small slit is made in the gel in front of the desired fragment, and a small piece of NA-45 DEAE membrane (Schleicher and Schuell, Keene, NH 03431) is placed in each slit. Upon further electrophoresis, the DNA non-covalently binds to the DEAE membrane. After the desired fragment is bound to the DEAE membrane, the membrane is removed and rinsed with low-salt buffer (100 mM KCl; 0.l mM EDTA; and 20 mM Tris-HCl, pH = 8). Next, the membrane is placed in a small tube and immersed in high-salt buffer (1 M NaCl; 0.1 mM EDTA; and 20 mM Tris-HC1, pH = 8) and then incubated at 65°C for one hour to remove the DNA from the DEAE paper. After the 65°C incubation, the incubation buffer is collected and the membrane rinsed with high-salt buffer. The high-salt rinse solution is pooled with the high-salt incubation buffer.

The volume of the high salt-DNA solution is adjusted so that the NaCl concentration is 0.25 M, and then three volumes of cold, absolute ethanol are added to the solution. The resulting solution is mixed and placed at -70°C for 10-20 minutes. The solution is then centrifuged at 15,000 rpm for 15 minutes. After another precipitation to remove residual salt, the DNA pellet is rinsed with ethanol, dried, resuspended in 20 $\mu$l of TE buffer, and constitutes about 3 $\mu$g of the desired restriction fragment of Ad2. The purified fragment obtained is dissolved in 10 $\mu$l of TE buffer.

About 6 $\mu$l of $H_2O$ and 2 $\mu$l of 10X AccI buffer (60 mM NaCl; 60 mM Tris-HCl, pH = 7.5; 60 mM $MgCl_2$; 60 mM DTT; and 1 mg/ml BSA) are added to the solution of the ~2.4 kb BalI restriction fragment of Ad2. After the addition of about 2 $\mu$l ( 10 units) of restriction enzyme AccI to the solution of DNA, the reaction is incubated at 37°C for 2 hours. After the AccI digestion, the DNA is collected by ethanol precipitation and resuspended in 16 $\mu$l of $H_2O$ and 2 $\mu$l of 10X PvuII buffer (600 mM NaCl; 60 mM Tris-HCl, pH = 7.5; 60 mM $MgCl_2$; 60 mM DTT; and 1 mg/ml BSA). After the addition of about 2 $\mu$l (about 10 units) of restriction enzyme PvuII to the solution of DNA, the reaction is incubated at 37°C for 2 hours.

The AccI-PvuII-digested, ~2.4 kb BalI restriction fragment of Ad2 is loaded onto an ~6% polyacrylamide gel and electrophoresed until the 0.32 kb AccI-PvuII restriction fragment that comprises the Ad2 late promoter is separated from the other digestion products. The gel is stained with ethidium bromide and viewed using UV light, and the segment of gel containing the ~0.32 kb AccI-PvuII restriction fragment is cut from the gel, crushed, and soaked overnight at room temperature in ~250 $\mu$l of extraction buffer (500 mM $NH_4OAc$; 10 mM MgOAc; 1 mM EDTA; and 0.1% SDS). The following morning, the mixture is centrifuged, and the pellet is discarded. The DNA in the supernatant is precipitated with ethanol; about 2 $\mu$g of tRNA are added to ensure complete precipitation of the desired fragment. About 0.2 $\mu$g of the ~0.32 kb AccI-PvuII restriction fragment are obtained and suspended in 7 $\mu$l of $H_2O$.

To convert the AccI-PvuII restriction fragment to an AccI-BclI restriction fragment, BclI linkers were ligated to the ~0.32 AccI-PvuII restriction fragment. Because the BclI linkers were blunt-ended, the linkers only attached to the PvuII end of the restriction fragment. The BclI linkers (New England Biolabs), which had the following sequence:

$$5'-CTGATCAG-3'$$
$$|\ |\ |\ |\ |\ |\ |\ |$$
$$3'-GACTAGTC-5',$$

were kinased and prepared for ligation by the following procedure. Four $\mu$l of linkers (~2 $\mu$g) were dissolved in 20.15 $\mu$l of $H_2O$ and 5 $\mu$l of 10X kinase buffer (500 mM Tris-HCl, pH = 7.6 and 100 mM $MgCl_2$), incubated at 90°C for two minutes, and then cooled to room temperature. Five $\mu$l of $\gamma$-$^{32}$P-ATP (~20 $\mu$Ci), 2.5 $\mu$l of 1 M DTT, and 5 $\mu$l of polynucleotide kinase (~10 units) were added to the mixture, which was then incubated at 37°C for 30 minutes. Then, 3.35 $\mu$l of 0.01 M ATP and 5 $\mu$l of kinase were added, and the reaction was continued for another 30 minutes at 37°C. The radioactive ATP aids in determining whether the linkers have ligated to the target DNA.

About 0.25 $\mu$g (in 0.5 $\mu$l) of the kinased BclI linkers was added to the solution of the ~0.32 kb AccI-PvuII restriction fragment, and then, 1 $\mu$l (~1000 units) of T4 DNA ligase and 1 $\mu$l of 10X ligase buffer were added to the solution of DNA, and the resulting reaction was incubated at 16°C overnight. The BclI linkers could only ligate to the PvuII end of the AccI-PvuII restriction fragment. DNA sequencing later revealed that four BclI linkers attached to the PvuII end of the AccI-PvuII restriction fragment. These extra BclI linkers can be removed by BclI digestion and religation; however, the extra BclI linkers were not removed as the linkers do not interfere with the proper functioning of the vectors that comprise the extra linkers.

E. coli K12 HB101/pSV2cat cells are obtained in lyophilized form from the ATCC under the accession number ATCC 37155, and plasmid pSV2cat DNA was isolated from the cells in substantial accordance with the procedure of Example 1A, except that ampicillin, at 50 $\mu$g/ml, was used in place of tetracycline. A restriction site and function map of plasmid pSV2cat is presented in Figure 1, Part B, of the accompanying drawings. About 1 mg of plasmid pSV2cat DNA is obtained and dissolved in 1 ml of TE buffer. About 3 $\mu$g (3 $\mu$l) of the plasmid pSV2cat DNA were added to 2 $\mu$l of 10X AccI buffer and 16 $\mu$l of $H_2O$, and then, 3 $\mu$l (about 9 units) of restriction enzyme AccI were added to the solution of pSV2cat DNA, and the resulting reaction was incubated at 37°C for 2 hours. The AccI-digested plasmid pSV2cat DNA was then digested with restriction enzyme StuI by adding 3 $\mu$l of 10X StuI buffer (1.0M NaCl; 100 mM Tris-HCl, pH = 8.0; 100 mM $MgCl_2$; 60 mM DTT; and 1 mg/ml BSA), 5 $\mu$l of $H_2O$, and about 2 $\mu$l (about 10 units) of restriction enzyme StuI. The resulting reaction was incubated at 37°C for 2 hours. The reaction was terminated by extracting the reaction mixture once with phenol, then twice with chloroform. About 0.5 $\mu$g of the desired fragment was obtained and dissolved in 20 $\mu$l of TE buffer.

About 4 $\mu$l of the AccI-StuI-digested plasmid pSV2cat DNA were mixed with about 7 $\mu$l of the ~0.32 kb AccI-PvuII (with BclI linkers attached) restriction fragment of Ad2, and after the addition of 3 $\mu$l of 10X ligase buffer, 15 $\mu$l of $H_2O$, and 2 $\mu$l (about 1000 units) of T4 DNA ligase, the ligation reaction was incubated at 16°C overnight. The ligated DNA constituted the desired plasmid pLPcat, a plasmid that comprises the Ad2 late promoter positioned so as to drive transcription, and thus expression, of the chloramphenicol acetyltransferase gene. A restriction site and function map of plasmid pLPcat is presented in Figure 1, Part B, of the accompanying drawings.

The ligated DNA was used to transform E. coli K12 HB101 cells in substantial accordance with the procedure of Example 2B. The transformed cells were plated on L-agar plates containing 50 $\mu$g/ml ampicillin; restriction enzyme analysis of plasmid DNA was used to identify the E. coli K12 HB101/pLPcat transformants. Plasmid pLPcat DNA was isolated from the transformants for use in subsequent constructions in substantial accordance with the plasmid isolation procedure described in Example 1A, except that ampicillin was used as the selective agent in place of tetracycline.

D. Construction of Plasmid pBLcat

About 88 $\mu$g of plasmid pBKneo1 DNA in 50 $\mu$l of TE buffer were added to 7.5 $\mu$l of 10X AccI buffer, 30 $\mu$l of $H_2O$, and 15 $\mu$l (about 75 units) of restriction enzyme AccI, and the resulting reaction was incubated at 37°C for 2 hours. The AccI-digested plasmid pBKneo1 DNA was loaded on an agarose gel, and the ~1.4 kb fragment that contains the BK enhancer was separated from the other digestion products. The ~1.4 kb AccI restriction fragment was then isolated from the gel and purified. About 5 $\mu$g of the fragment were resuspended in 5 $\mu$l of 10X PvuII buffer, 45 $\mu$l of $H_2O$, and 5 $\mu$l (about 25 units) of restriction enzyme PvuII, and the resulting reaction was incubated at 37°C for 2 hours. The PvuII-digested DNA was then isolated, purified, and prepared for ligation. About 2 $\mu$g of the desired ~1.28 kb AccI-PvuII fragment were obtained and dissolved in 5 $\mu$l of TE buffer.

About 1 $\mu$g of plasmid pLPcat DNA was dissolved in 5 $\mu$l of 10X AccI buffer and 40 $\mu$l of $H_2O$. About 5 $\mu$l (~25 units) of restriction enzyme AccI were added to the solution of plasmid pLPcat DNA, and the resulting reaction was incubated at 37°C. The AccI-digested plasmid pLPcat DNA was precipitated with ethanol and resuspended in 5 $\mu$l of 10X StuI buffer, 40 $\mu$l of $H_2O$, and 5 $\mu$l (about 25 units) of restriction enzyme StuI, and the resulting reaction was incubated at 37°C for 2 hours. The AccI-StuI-digested plasmid pLPcat DNA was precipitated with ethanol several times to purify the ~4.81 kb AccI-StuI restriction fragment that comprises the E. coli origin of replication and Ad2 late promoter away from the other digestion product, a restriction fragment about 16 bp in size. About 1 $\mu$g of the desired ~4.81 kb restriction fragment was obtained and dissolved in 20 $\mu$l of TE buffer.

The 5 $\mu$l of ~4.81 kb AccI-StuI restriction fragment of plasmid pLPcat were added to 5 $\mu$l of ~1.28 kb AccI-PvuII restriction fragment of plasmid pBKneo1. After the addition of 3 $\mu$l of 10X ligase buffer, 15 $\mu$l of $H_2O$, and 2 $\mu$l (about 10 units) of T4 DNA ligase to the mixture of DNA, the resulting ligation reaction was incubated at 16°C overnight. The ligated DNA constituted the desired plasmid pBLcat. A restriction site and function map of plasmid pBLcat is presented in Figure 1, Part C, of the accompanying drawings.

The ligated DNA was used to transform E. coli K12 HB101 cells in substantial accordance with the procedure described in Example 2B. E. coli K12 HB101/pBLcat transformants were identified by restriction enzyme analysis of their plasmid DNA. Plasmid pBLcat DNA was prepared for use in subsequent constructions in substantial accordance with the procedure of Example 1A, except that ampicillin was used as the selective agent in place of tetracycline.

E. Construction of Plasmid pL133

Plasmid pL133 is a human protein C expression vector. As described below, plasmid pL133 can be constructed using starting vector plasmids pSV2gpt and pHC7 (the preparation of plasmid pHC7 is described above in Example 1A) to construct intermediate vector plasmid pSV2-HPC8, which is then combined with plasmid pSV2-$\beta$-globin to yield plasmid pL133. The construction protocol for plasmid pL133 is described in detail, below, and schematically illustrated in Figure 2 of the accompanying drawings.

Fifty $\mu$l (~50 $\mu$g) of plasmid pHC7 DNA were mixed with 5 $\mu$l (~50 units) of restriction enzyme BanI, 10 $\mu$l of 10X BanI reaction buffer (1.5 M NaCl; 60 mM Tris-HCl, pH = 7.9; 60 mM $MgCl_2$; and 1 mg/ml BSA), and 35 $\mu$l of $H_2O$ and incubated until the digestion was complete. The BanI-digested plasmid pHC7 DNA was then electrophoresed on a 3.5% polyacrylamide gel (29:1, acrylamide:bis-acrylamide), until the ~1.25 kb BanI restriction fragment was separated from the other digestion products.

The region of the gel containing the ~1.25 kb BanI restriction fragment was cut from the gel, placed in a test tube, and broken into small fragments. One ml of extraction buffer (500 mM $NH_4OAc$, 10 mM MgOAc, 1 mM EDTA, 1% SDS, and 10 mg/ml tRNA) was added to the tube containing the fragments, and the tube was placed at 37°C overnight. Centrifugation was used to pellet the debris, and the supernatant was transferred to a new tube. The debris was washed once with 200 $\mu$l of extraction buffer; the wash supernatant was combined with the first supernatant from the overnight extraction. After passing the supernatant through a plug of glass wool, two volumes of ethanol were added to and mixed with the supernatant. The resulting solution was placed in a dry ice-ethanol bath for ~10 minutes, and then, the DNA was pelleted by centrifugation.

Approximately 8 $\mu$g of the ~1.25 kb BanI restriction fragment were obtained by this procedure. The purified fragment was suspended in 10 $\mu$l of TE buffer and stored at -20°C. The BanI restriction fragment had to be modified by the addition of a linker to construct plasmid pSV2-HPC8. The DNA fragments used in the construction of the linker were synthesized either by using a Systec 1450A DNA Synthesizer (Systec Inc., 3816 Chandler Drive, Minneapolis, MN) or an ABS 380A DNA Synthesizer (Applied Biosystems, Inc., 850 Lincoln Centre Drive, Foster City, CA 94404). Many DNA synthesizing instruments are known in the art and can be used to make the fragments. In addition, the fragments can also be conventionally prepared in substantial accordance with the procedures of Itakura et al., 1977, Science, 198:1056 and Crea et al., 1978, Proc. Nat. Acad. Sci. USA, 75:5765.

Five hundred picomoles of each single strand of the linker were kinased in 20 $\mu$l of reaction buffer, which contained 15 units (~0.5 $\mu$l) T4 polynucleotide kinase, 2 $\mu$l 10X ligase buffer, 10 $\mu$l of 500 $\mu$M ATP, and 7.5 $\mu$l of $H_2O$. The kinase reaction was incubated at 37°C for 30 minutes, and the reaction was terminated by incubation at 100°C for 10 minutes. To ensure complete kination, the reaction was chilled on ice, 2 $\mu$l of 0.2 M dithiothreitol, 2.5 $\mu$l of 5 mM ATP, and 15 units of T4 polynucleotide kinase were added to the reaction mixture and mixed, and the reaction mixture was incubated another 30 minutes at 37°C. The reaction was stopped by another 10 minute incubation at 100°C and then chilled on ice.

Although kinased separately, the two single strands of the DNA linker were mixed together after the kinase reaction. To anneal the strands, the kinase reaction mixture was incubated at 100°C for 10 minutes in a water bath containing ~150 ml of water. After this incubation, the water bath was shut off and allowed to cool to room temperature, a process taking about 3 hours. The water bath, still containing the tube of kinased DNA, was then incubated at 4°C overnight. This process annealed the single strands. The linker constructed had the following structure:

```
5'-AGCTTTGATCAG-3'
   | | | | | | | |
  3'-AACTAGTCCACG-5'
```

The linker was stored at -20°C until use.

The 8 ~µg of ~1.25 kb BanI fragment were added to and mixed with the ~50 µl of linker (~500 picomoles), 1 µl of T4 DNA ligase (~5 units), 10 µl of 10X ligase buffer, and 29 µl of $H_2O$, and the resulting ligation reaction was incubated at 4°C overnight. The ligation reaction was stopped by a 10 minute incubation at 65°C. The DNA was pelleted by adding NaOAc to a final concentration of 0.3 M, adding 2 volumes of ethanol, chilling in a dry ice-ethanol bath, and then centrifuging the solution.

The DNA pellet was dissolved in 10 µl of 10X ApaI reaction buffer (60 mM NaCl; 60 mM Tris-HCl, pH = 7.4; 60 mM $MgCl_2$; and 60 mM 2-mercaptoethanol), 5 µl (~50 units) of restriction enzyme ApaI, and 85 µl of $H_2O$, and the reaction was placed at 37°C for two hours. The reaction was then stopped and the DNA pelleted as above. The DNA pellet was dissolved in 10 µl of 10X HindIII reaction buffer, 5 µl (~50 units) of restriction enzyme HindIII, and 85 µl of $H_2O$, and the reaction was placed at 37°C for two hours. After the HindIII digestion, the reaction mixture was loaded onto a 3.5% polyacrylamide gel, and the desired ~1.23 kb HindIII-ApaI restriction fragment was isolated from the gel and purified. Approximately 5 µg of the desired fragment were obtained, suspended in 10 µl of TE buffer, and stored at -20°C.

Fifty µl (~50 µg) of plasmid pHC7 DNA were mixed with 5 µl (~50 units) of restriction enzyme PstI, 10 µl of 10X PstI reaction buffer (1.0 M NaCl; 100 mM Tris-HCl, pH = 7.5; 100 mM $MgCl_2$; and 1 mg/ml BSA), and 35 µl of $H_2O$ and incubated at 37°C for two hours. The PstI-digested plasmid pHC7 DNA was then electrophoresed on a 3.5% polyacrylamide gel, and the desired ~0.88 kb fragment was purified in substantial accordance with the procedure described above. Approximately 5 µg of the desired fragment were obtained, suspended in 10 µl of TE buffer, and stored at -20°C.

The ~5 µg of 0.88 kb PstI fragment were added to and mixed with ~50 µl of the following linker, which was constructed on an automated DNA synthesizer:

```
5'-GTGATCAA-3'
   | | | | | | | |
 3'-ACGTCACTAGTTCTAG-5'
```

About 1 µl of T4 DNA ligase (~10 units), 10 µl 10X ligase buffer, and 29 µl $H_2O$ were added to the mixture of DNA, and the resulting ligation reaction was incubated at 4°C overnight.

The ligation reaction was stopped by a 10 minute incubation at 65°C. After precipitation of the ligated DNA, the DNA pellet was dissolved in 10 µl of 10X ApaI reaction buffer, 5 µl (~50 units) of restriction enzyme ApaI, and 85 µl of $H_2O$, and the reaction was placed at 37° for two hours. The reaction was then stopped and the DNA pelleted once again. The DNA pellet was dissolved in 10 µl 10X BglII reaction buffer (1 M NaCl; 100 mM Tris-HCl, pH = 7.4; 100 mM $MgCl_2$; 100 mM 2-mercaptoethanol; and 1 mg/ml BSA), 5 µl (~50 units) of restriction enzyme BglII, and 85 µl $H_2O$, and the reaction was placed at 37°C for two hours. After the BglII digestion, the reaction mixture was loaded onto a 3.5% polyacrylamide gel, and the desired ~0.19 kb ApaI-BglII restriction fragment was isolated in substantial accordance with the procedure described above. Approximately 1 µg of the desired fragment was obtained, suspended in 10 µl of TE buffer, and stored at -20°C.

Approximately 10 µg of plasmid pSV2gpt DNA (ATCC 37145) were dissolved in 10 µl of 10X HindIII reaction buffer, 5 µl (~50 units) of restriction enzyme HindIII, and 85 µl of $H_2O$, and the reaction was placed at 37°C for 2 hours. The reaction mixture was then made 0.25 M in NaOAc, and after the addition of two volumes of ethanol and incubation in a dry ice-ethanol bath, the DNA was pelleted by centrifugation. The DNA pellet was dissolved in 10 µl of 10X BglII buffer, 5 µl (~50 units) of restriction enzyme BglII, and 85 µl

of $H_2O$, and the reaction was placed at 37°C for two hours. After the BglII digestion, the reaction mixture was loaded onto a 1% agarose gel, and the fragments were separated by electrophoresis. The gel was stained with ethidium bromide and viewed under ultraviolet light, and the band containing the desired ~5.1 kb HindIII-BglII fragment was cut from the gel and placed in dialysis tubing, and electrophoresis was continued until the DNA was out of the agarose. The buffer containing the DNA from the dialysis tubing was extracted with phenol and $CHCl_3$, and then, the DNA was precipitated. The pellet was resuspended in 10 $\mu l$ of TE buffer and constituted 5 ~$\mu g$ of the desired ~5.1 kb HindIII-BglII restriction fragment of plasmid pSV2gpt.

Two $\mu l$ of the ~1.23 kb HindIII-ApaI restriction fragment, 3 $\mu l$ of the ~0.19 kb ApaI-BglII fragment, and 2 $\mu l$ of the ~5.1 kb HindIII-BglII fragment were mixed together and then incubated with 10 $\mu l$ of 10X ligase buffer, 1 $\mu l$ of T4 DNA ligase (~500 units), and 82 $\mu l$ of $H_2O$ at 16°C overnight. The ligated DNA constituted the desired plasmid pSV2-HPC8; a restriction site and function map of the plasmid is presented in Figure 2 of the accompanying drawings.

E. coli K12 RR1 (NRRL B-15210) cells were made competent for transformation in substantial accordance with the procedure described for E. coli K12 HB101 in Example 2B. The ligated DNA prepared above was used to transform the cells, and aliquots of the transformation mix were plated on L-agar plates containing 100 $\mu g/ml$ ampicillin. The plates were then incubated at 37°C. E. coli K12 RR1/pSV2-HPC8 transformants were verified by restriction enzyme analysis of their plasmid DNA. Plasmid pSV2-HPC8 DNA was prepared from the transformants in substantial accordance with the procedure of Example 1A, except that ampicillin, and not tetracycline, was used as the selective agent during culture of the cells.

Fifty $\mu g$ of plasmid pSV2-HPC8 were dissolved in 10 $\mu l$ of 10X HindIII reaction buffer, 5 $\mu l$ (~50 units) of restriction enzyme HindIII, and 85 $\mu l$ of $H_2O$, and the reaction was incubated at 37°C for two hours. After the HindIII digestion, the DNA was precipitated, and the DNA pellet was dissolved in 10 $\mu l$ of 10X SalI reaction buffer (1.5 M NaCl; 60 mM Tris-HCl, pH = 7.9; 60 mM $MgCl_2$; 60 mM 2-mercaptoethanol; and 1 mg/ml BSA), 5 $\mu l$ (~50 units) of restriction enzyme SalI, and 85 $\mu l$ of $H_2O$. The resulting SalI reaction mixture was incubated for 2 hours at 37°C. The HindIII-SalI-digested plasmid pSV2-HPC8 was loaded onto a 3.5% polyacrylamide gel and electrophoresed until the desired ~0.29 kb HindIII-SalI restriction fragment was separated from the other reaction products. The desired fragment was isolated from the gel; about 2 $\mu g$ of the fragment were obtained and suspended in 10 $\mu l$ of TE buffer.

Fifty $\mu g$ of plasmid pSV2-HPC8 were dissolved in 10 $\mu l$ of 10X BglII reaction buffer, 5 $\mu l$ (50 units) of restriction enzyme BglII, and 85 $\mu l$ of $H_2O$, and the reaction was incubated at 37°C for two hours. After the BglII digestion, the DNA was precipitated, and the DNA pellet was dissolved in 10 $\mu l$ of 10X SalI reaction buffer, 5 $\mu l$ (~50 units) of restriction enzyme SalI, and 85 $\mu l$ of $H_2O$. The resulting SalI reaction mixture was incubated for 2 hours at 37°C. The SalI-BglII-digested plasmid pSV2-HPC8 was loaded onto a 3.5% polyacrylamide gel and electrophoresed until the desired ~1.15 kb SalI-BglII restriction fragment was separated from the other reaction products. The ~1.15 kb SalI-BglII restriction fragment was isolated from the gel; about 8 $\mu g$ of fragment were obtained and suspended in 10 $\mu l$ of TE buffer.

Approximately 10 $\mu g$ of plasmid pSV2-$\beta$-globin DNA (NRRL B-15928) were dissolved in 10 $\mu l$ of 10X HindIII reaction buffer, 5 $\mu l$ (~50 units) of restriction enzyme HindIII, and 85 $\mu l$ of $H_2O$, and the reaction was placed at 37°C for 2 hours. The reaction mixture was then made 0.25 M in NaOAc, and after the addition of two volumes of ethanol and incubation in a dry ice-ethanol bath, the DNA was pelleted by centrifugation. The HindIII-digested plasmid pSV2-$\beta$-globin was dissolved in 10 $\mu l$ of 10X BglII buffer, 5 $\mu l$ (~50 units) of restriction enzyme BglII, and 85 $\mu l$ of $H_2O$, and the reaction was placed at 37°C for two hours. After the BglII digestion, the reaction mixture was loaded onto a 1% agarose gel, and the fragments were separated by electrophoresis. The desired ~4.2 kb HindIII-BglII restriction fragment was isolated from the gel; about 5 $\mu g$ of the desired fragment were obtained and suspended in 10 $\mu l$ of TE buffer.

Two $\mu l$ of the ~0.29 kb HindIII-SalI fragment of plasmid pSV2-HPC8, 2 $\mu l$ of the ~1.15 kb SalI-BglII fragment of plasmid pSV2-HPC8, and 2 $\mu l$ of the ~4.2 kb HindIII-BglII fragment of plasmid pSV2-$\beta$-globin were mixed together and ligated with T4 DNA ligase. The ligated DNA constituted the desired plasmid pL133; a restriction site and function map of plasmid pL133 is presented in Figure 2 of the accompanying drawings. The ligated DNA was used to transform E. coli K12 RR1, and the desired E. coli K12 RR1/pL133 transformants were identified by their ampicillin-resistant phenotype and by restriction enzyme analysis of their plasmid DNA.

F. Construction of Plasmid pLPC From Plasmids pL133 and pBLcat

About 20 $\mu g$ of plasmid pBLcat DNA were dissolved in 10 $\mu l$ of 10X HindIII buffer and 80 $\mu l$ of $H_2O$. About 10 $\mu l$ (~100 units) of restriction enzyme HindIII were added to the solution of plasmid pBLcat DNA, and the resulting reaction was incubated at 37°C for 2 hours. The HindIII-digested plasmid pBLcat DNA was loaded onto an agarose gel and electrophoresed until the ~0.87 kb HindIII restriction fragment that comprises the BK enhancer and Ad2 late promoter was separated from the other digestion products; then, the ~0.87 kb fragment was isolated, purified, and prepared for ligation. About 2 μg of the desired fragment were obtained and dissolved in 5 μl of TE buffer.

About 1.5 μg of plasmid pL133 DNA were dissolved in 2 μl of 10X HindIII buffer and 16 μl of $H_2O$. About 1 μl (~10 units) of restriction enzyme HindIII was added to the solution of DNA, and the resulting reaction was incubated at 37°C for 2 hours. The DNA was then diluted to 100 μl with TE buffer and treated with ~0.06 units of calf-intestinal alkaline phosphatase, and the resulting reaction was incubated at 37°C for 30 minutes. The solution was adjusted to contain 1X SET (5 mM Tris-HCl, pH = 7.8; 5 mM EDTA; and 150 mM NaCl), 0.3M NaOAc, and 0.5% SDS and then incubated at 65°C for 45 minutes. The HindIII-digested plasmid pL133 DNA was then extracted twice with phenol and once with chloroform, precipitated with ethanol, and resuspended in 10 μl of TE buffer.

About 5 μl of the ~0.87 kb HindIII restriction fragment of plasmid pBLcat were added to the 1.5 μg (10 μl) of HindIII-digested plasmid pL133, and then, 2 μl of 10X ligase buffer, 1 μl (~10 units) of T4 DNA ligase, and 2 μl of $H_2O$ were added to the solution of DNA, and the resulting reaction was incubated at 16°C overnight. The ligated DNA constituted the desired plasmid pLPC.

The ligated DNA was used to transform E. coli K12 HB101 in substantial accordance with the procedure of Example 2B. The transformed cells were plated on L-agar plates containing ampicillin, and the plasmid DNA of the ampicillin-resistant transformants was examined by restriction enzyme analysis to identify the E. coli K12 HB101/pLPC transformants. The ~0.87 kb HindIII restriction fragment that encodes the BK enhancer and Ad2 late promoter could insert into HindIII-digested plasmid pL133 in one of two orientations, only one of which yields plasmid pLPC. A restriction site and function map of plasmid pLPC is presented in Figure 1, Part D, of the accompanying drawings.

Example 3

The Construction of Plasmid pLPC-167G

Plasmid pLPC-167G was constructed in substantial accordance with the site-specific mutagenesis and other construction protocols used in the construction of plasmid pLAPC, as described in Example 1. Buffers and annealing conditions used in the construction of plasmid pLPC-167G, however, were as described by Zoller and Smith, 1984, DNA 3:479-489.

In the construction of plasmid pLPC-167G, phage M13mp18-HE1 (see Example 1B) were subjected to site-specific mutagenesis using the mutagenizing oligonucleotide depicted below:
5′-GACCAAGAAGACCAAGTAGGCCCGCGGCTCATTGATG-3′.
The mutagenized phage resulting from the site-specific mutagenesis were designated M13mp18-HE4.

Final construction of plasmid pLPC-167G proceeded in a manner analogous to the construction of plasmid pLAPC, set forth in Example 1C. However, plasmid pLAPC was constructed using two restriction fragments originating from plasmid pLPC. In the construction of plasmid pLPC-167G, these same two fragments were instead obtained from plasmid pLAPC. The reason for using plasmid pLAPC as the source of the fragments, instead of plasmid pLPC, was to facilitate restriction analysis in identifying the plasmid pLPC-167G transformants. Because plasmids pLPC and pLPC-167G are very close to the same size, it would have been difficult to distinguish "parentals" (plasmid pLPC) from plasmid pLPC-167G. These parentals could be present, despite the purification of the fragments used in the ligation, due to a variety of factors. However, because plasmid pLAPC is smaller than plasmid pLPC-167G, by obtaining the two fragments from plasmid pLAPC, one could readily distinguish parentals (plasmid pLAPC) from the desired plasmid pLPC-167G. Thus, to construct plasmid pLPC-167G, the ~0.7 kb SstI-SalI restriction fragment of phage M13mp18-HE4 was ligated to the ~3.76 kb EcoRI-SalI restriction fragment of plasmid pLAPC and the ~2.0 kb EcoRI-SstI restriction fragment of plasmid pLAPC. The ligated DNA constituted the desired plasmid pLPC-167G, which was transformed into E. coli K12 RV308. The resulting E. coli K12 RV308/pLPC-167G transformants were used to obtain a large-scale preparation of plasmid pLPC-167G DNA for use in transformations of eukaryotic cells.

34

Example 4

The Construction of Plasmid pLPC-167F

Plasmid pLPC-167F was constructed in substantial accordance with the site-specific mutagenesis and other construction protocols used in the construction of plasmid pLAPC, as described in Example 1. Buffers and annealing conditions used in the construction of plasmid pLPC-167F, however, were as described by Zoller and Smith, 1984, DNA 3:479-489.

In the construction of plasmid pLPC-167F, phage M13mp18-HE1 (see Example 1B) were subjected to site-specific mutagenesis using the mutagenizing oligonucleotide depicted below:

5′-GACCAAGAAGACCAAGTATTCCCGCGGCTCATTGATG-3′.

The mutagenized phage resulting from the site-specific mutagenesis were designated M13mp18-HE5.

Final construction of plasmid pLPC-167F proceeded in a manner analogous to the construction of plasmid pLAPC, set forth in Example 1C. However, plasmid pLAPC was constructed using two restriction fragments originating from plasmid pLPC. In the construction of plasmid pLAPC-167F, these same two fragments were instead obtained from plasmid pLAPC. The reason for using plasmid pLAPC as the source of the fragments, instead of plasmid pLPC, was to facilitate restriction analysis in identifying the plasmid pLPC-167F transformants. Because plasmids pLPC and pLPC-167F are very close to the same size, it would have been difficult to distinguish "parentals" (plasmid pLPC) from plasmid pLPC-167F. However, because plasmid pLAPC is smaller than plasmid pLPC-167F, by obtaining the two fragments from plasmid pLAPC, one could readily distinguish parentals (plasmid pLAPC) from the desired plasmid pLPC-167F. Thus, to construct plasmid pLPC-167F, the ~0.7 kb SstI-SalI restriction fragment of phage M13mp18-HE5 was ligated to the ~3.76 kb EcoRI-SalI restriction fragment of plasmid pLAPC and the ~2.0 kb EcoRI-SstI restriction fragment of plasmid pLAPC. The ligated DNA constituted the desired plasmid pLPC-167F, which was transformed into E. coli K12 RV308. The resulting E. coli K12 RV308/pLPC-167F transformants were used to obtain a large-scale preparation of plasmid pLPC-167F DNA for use in transformations of eukaryotic cells.

Example 5

Construction of Adenovirus-transformed Human Embryonic Kidney Cell Line 293 and Adenovirus-transformed Syrian Hamster Cell Line AV12 Transformants Using Plasmids pLPC-167G and pLPC-167F

Human Embryonic Kidney Cell Line 293 is available from the American Type Culture Collection under the accession number ATCC CRL 1573. The adenovirus-transformed Syrian hamster cell line AV12 is also available from the American Type Culture Collection under the accession number ATCC CRL 9595. The transformation procedure described below refers to 293 cells as the host cell line; however, the procedure is generally applicable to most eukaryotic cell lines, including the AV12 cell line, and to the expression vectors of the invention.

293 cells are obtained from the ATCC under the accession number CRL 1573 in a 25 mm$^2$ flask containing a confluent monolayer of about $5.5 \times 10^6$ cells in Eagle's Minimum Essential Medium (Gibco) with 10% heat-inactivated horse serum. The flask is incubated at 37°C; medium is changed twice weekly. Media is composed of DMEM (Gibco) supplemented with 10% fetal calf serum, 50 $\mu$g/ml gentamicin, and 10 $\mu$g/ml AquaMEPHYTON® phytonadione vitamin $K_1$ (Merck Sharp and Dohme, Merck and Co., Inc., West Point, PA 19486). The cells are subcultured by removing the medium, rinsing with Hank's Balanced Salts solution (Gibco), adding 0.25% trypsin (containing 0.2 g/L EDTA) for 1-2 minutes, rinsing with fresh medium, aspirating, and dispensing into new flasks at a subcultivation ratio of 1:5 or 1:10.

One day prior to transformation, cells are seeded at $0.7 \times 10^6$ cells per 100 mm dish. Sterile, ethanol-precipitated plasmid DNA dissolved in TE buffer is used to prepare a 2X DNA-CaCl$_2$ solution containing 25 $\mu$g/ml of the transforming plasmid DNA (for plasmid pLPC-167F or pLPC-167G transformations, usually two plasmids are used, plasmid pLPC-167F or pLPC-167G and a plasmid that contains a selectable marker, as discussed below) and 250 mM CaCl$_2$. 2X HBSS is prepared containing 280 mM NaCl, 50 mM Hepes, and 1.5 mM sodium phosphate, with the pH adjusted to 7.05-7.15. The 2X DNA-CaCl$_2$ solution is added dropwise to an equal volume of sterile 2X HBSS. A one ml sterile plastic pipette with a cotton plug is inserted into the mixing tube that contains the 2X HBSS, and bubbles are introduced by blowing while the DNA is being added. The calcium-phosphate-DNA precipitate is allowed to form without agitation for 30-45 minutes at room temperature.

The precipitate is then mixed by gentle pipetting with a plastic pipette, and one ml (per plate) of precipitate is added directly to the 10 ml of growth medium that covers the recipient cells. After 4 hours of incubation at 37°C, the media is replaced with fresh media and the cells allowed to incubate for an additional 72 hours before providing selective pressure. For plasmids that do not comprise a selectable marker that functions in eukaryotic cells, such as either plasmid pLPC-167F or pLPC-167G, the transformation procedure utilizes a mixture of plasmids: the expression vector of the present invention that lacks a selectable marker; and an expression vector that comprises a selectable marker that functions in eukaryotic cells. A variety of vectors are available for use in such cotransformation systems and include plasmids pSV2-dhfr (ATCC 37146), pSV2-neo (ATCC 37149), pSV2-gpt (ATCC 37145), and pSV2-hyg (NRRL B-18039). Plasmid pSV2-hyg confers resistance to hygromycin B to eukaryotic host cells. This co-transformation technique allows for the selection of cells that contain the plasmid with the selectable marker. These cells are further examined to identify cells that comprise both of the transforming plasmids. Of course, the present invention also comprises expression vectors that contain a selectable marker for eukaryotic cells and thus do not require use of the cotransformation technique.

For cells transfected with plasmids containing the hygromycin resistance-conferring gene, hygromycin B is added to the growth medium to a final concentration of about 200 $\mu$g/ml. The cells are then incubated at 37°C for 2-4 weeks with medium changes at 3 to 4 day intervals. The resulting hygromycin-resistant colonies are transferred to individual culture flasks for characterization. Plasmid pSV2-neo confers resistance to neomycin (G418 is also used in place of neomycin), and selection of G418-resistant colonies is performed in substantial accordance with the selection procedure for hygromycin-resistant cells, except that G418 is added to a final concentration of 400 $\mu$g/ml.

The use of the dihydrofolate reductase (dhfr) gene or the methotrexate resistance-conferring derivative of the dhfr gene (dhfr-mtx) as a selectable marker for introducing a gene or plasmid into a dhfr-deficient cell line and the subsequent use of methotrexate to amplify the copy number of the plasmid has been well established in the literature. 293 cells are dhfr positive, so 293 transformants that contain plasmids comprising the dhfr gene are not selected solely on the basis of the dhfr-positive phenotype, which is the ability to grow in media that lacks hypoxanthine and thymine. Cell lines that do lack a functional dhfr gene and are transformed with dhfr-containing plasmids can be selected for on the basis of the dhfr + phenotype. Although the use of dhfr as a selectable and amplifiable marker in dhfr-producing cells has not been well studied, evidence in the literature would suggest that dhfr can be used as a selectable marker and for gene amplification in dhfr-producing cells. The present invention is not limited by the selectable marker used on expression vectors. Moreover, amplifiable markers such as metallothionein genes, adenosine deaminase genes, or members of the multigene resistance family, exemplified by the P-glyco-protein gene, can be utilized.

Transformation of the 293 and AV12 cell lines with a mixture of plasmid pLPC-167F or pLPC-167G and a hygromycin resistance-conferring vector and subsequent selection for hygromycin-resistant cells yielded a number of transformants. (Other transformants were obtained by using plasmid pSV2-neo as the cotransforming vector and selecting for G418-resistant cells.) These transformants are analyzed, as described in Example 6, to determine which hygromycin-resistant cells contained plasmid pLPC-167F or pLPC-167G.

Example 6

Selection of High-Secreting Transformants

The hygromycin-resistant transformants obtained in Example 5 are grown on 100 mm$^2$ tissue culture dishes at a density of several hundred cell clones per tissue culture dish. The media is decanted, and the cells are rinsed twice with 5 ml aliquots of Hank's Balanced salt solution (Gibco). A solution of sterile 0.45% agar (Sigma Type 4 agarose, catalogue #A3643, Sigma Chemical Co., P.O. Box 14508, St. Louis, MO 63178) is prepared by mixing 1 ml of 1.8% agar (47°C) with 3 ml of Dulbecco's Modified Eagle's (DME) Salts (Gibco) (37°C), and 2 ml of this 0.45% agar solution are layered over the cells.

Nitrocellulose filters (Schleicher and Schuell, Inc., Keene, NH 03431) are boiled and then autoclaved 2 hours to remove the wetting agent, which is toxic to the cells. The filters are then placed on top of the agar layer, and after air bubbles are removed, the plates are incubated at 37°C for 1 to 3 hours. The filters, previously marked to indicate the original orientation of the filter on the dish so as to facilitate later identification of colonies, are then removed and placed in PBS (50 mM Tris-HCl, pH = 7.2, and 150 mM NaCl).

To keep the cells on the dish viable during analysis of the filters, the cells are overlayed with 8 ml of a mixture containing 2 ml of 1.8% agar (47°C), 2 ml of DME salts (37°C), and 4 ml of DME salts with 20% fetal bovine serum (37°C). The cells are then placed in a 37°C incubator.

All washes and reactions carried out on the filters are accomplished while the filters are on a rocking platform. The filters are first blocked by incubation at room temperature in 5% milk in PBS. The filters are then rinsed (5 minutes/rinse) four times in PBS. A 10 $\mu$g/ml biotinylated goat anti-human protein C polyclonal antibody in 2.5% bovine serum albumin is added to the filter (in sufficient quantities to cover the filter), which is then incubated at 37°C for 1 hour.

Purification of protein C, for subsequent use to prepare antibody against protein C, can be accomplished as described by Kisiel, 1979, J. Clin. Invest. 64:761. Polyclonal antibody can be prepared by the procedure disclosed in Structural Concepts in Immunology and Immunochemistry by E.A. Kabat, published in 1968 by Holt, Rhinehart, and Winston. Monoclonal antibody, which is also suitable for use in the assay, can be prepared as disclosed in Kohler and Milstein, 1975, Nature, 256:495, or as disclosed in U.S. Patent No. 4,696,895; EPO Pub. No. 205046; Laurell et al., 1985, FEBS 191(1):75; Suzuki et al., 1985, J. Biochem. 97:127-138; and EPO Pub. No. 138222. The avidin D and biotinylated horse radish peroxidase (HRP) used in the assay are obtained in a Vectastain™ kit (Vector Laboratories, Inc., 30 Ingold Road, Burlingame, CA 94010). Biotin is also obtained from Vector Laboratories, Inc.

The filters are rinsed four times with PBS at 4°C. Then, avidin D and biotinylated horse radish peroxidase are prepared and added as per the manufacturer's instructions in the Vectastain™ (Vector Laboratories) kit. The filters are incubated with the HRP-conjugated avidin D for 1 hour at 4°C (longer incubation times, i.e., overnight, can be used when small amounts of protein are being secreted); then, the filters are rinsed four times with PBS at 4°C.

To develop the indicator color on the filters, about 30 mg of HRP color-development reagent (4-chloro-1-napthol, Sigma) dissolved in ice-cold 100% methanol are added to 50 ml of PBS and 30 $\mu$l of 30% $H_2O_2$. This mixture is added to the nitrocellulose filters, which are incubated at room temperature until the color develops. Colonies secreting the most human protein C zymogen of the invention will be indicated on the filters not only by earliest appearance of the color but also by darker spots on the filter.

After the filters have been developed, the filters are again realigned with the original plates to determine which colonies are associated with which spots on the filter. The colonies secreting the most human protein C zymogen of the invention are then selected and used for production of the zymogen.

Those skilled in the art will recognize that the above assay is merely illustrative of the method of identifying high secreting cell lines. A variety of assay procedures can be successfully employed in the method. For instance, a double-antibody reaction can be employed in which the biotinylated goat anti protein C antibody is replaced with a goat anti-protein C antibody (IgG) and a biotinylated anti-goat IgG antibody.

**Claims**
**Claims for the following Contracting States : AT, BE, CH, DE, FR, GB, IT, LI, LU, NL, SE**

1. A DNA compound comprising a coding sequence for a protein that comprises from the amino terminus to the carboxy terminus:
   a) a signal peptide and propeptide of a $\gamma$-carboxylated, secreted protein;
   b) the light chain of human protein C;
   c) the dipeptide lysine-arginine, arginine-lysine, lysine-lysine, or arginine-arginine; and

d) the amino acid residue sequence:

```
                                  ASP THR GLU ASP GLN GLU ASP GLN VAL

        R₁  R₂  ARG LEU ILE R₃  GLY LYS MET THR ARG ARG GLY ASP SER PRO

        TRP GLN VAL VAL LEU LEU ASP SER LYS LYS LYS LEU ALA CYS GLY ALA

        VAL LEU ILE HIS PRO SER TRP VAL LEU THR ALA ALA HIS CYS MET ASP

        GLU SER LYS LYS LEU LEU VAL ARG LEU GLY GLU TYR ASP LEU ARG ARG

        TRP GLU LYS TRP GLU LEU ASP LEU ASP ILE LYS GLU VAL PHE VAL HIS

        PRO ASN TYR SER LYS SER THR THR ASP ASN ASP ILE ALA LEU LEU HIS

        LEU ALA GLN PRO ALA THR LEU SER GLN THR ILE VAL PRO ILE CYS LEU

        PRO ASP SER GLY LEU ALA GLU ARG GLU LEU ASN GLN ALA GLY GLN GLU

        THR LEU VAL THR GLY TRP GLY TYR HIS SER SER ARG GLU LYS GLU ALA

        LYS ARG ASN ARG THR PHE VAL LEU ASN PHE ILE LYS ILE PRO VAL VAL

        PRO HIS ASN GLU CYS SER GLU VAL MET SER ASN MET VAL SER GLU ASN

        MET LEU CYS ALA GLY ILE LEU GLY ASP ARG GLN ASP ALA CYS GLU GLY


        ASP SER GLY GLY PRO MET VAL ALA SER PHE HIS GLY THR TRP PHE LEU

        VAL GLY LEU VAL SER TRP GLY GLU GLY CYS GLY LEU LEU HIS ASN TYR

        GLY VAL TYR THR LYS VAL SER ARG TYR LEU ASP TRP ILE HIS GLY HIS

        ILE ARG ASP LYS GLU ALA PRO GLN LYS SER TRP ALA PRO-COOH
```

wherein $R_1$ is PHE, GLY, TYR, or TRP, $R_2$ is VAL or PRO, $R_3$ is ASP or ASN, ARG is Arginine, ASN is Asparagine, ASP is Aspartic acid, -COOH is the carboxy terminus, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine.

2. The DNA compound of Claim 1, wherein the signal peptide and propeptide are the signal peptide and propeptide of nascent human protein C.

3. The DNA compound of Claim 1 or 2, wherein the dipeptide is lysine-arginine.

**4.** The DNA compound of Claim 3, wherein the polypeptide encoded by the DNA is:

```
H₂N-MET TRP GLN LEU THR SER LEU LEU LEU PHE VAL ALA THR TRP GLY ILE

     SER GLY THR PRO ALA PRO LEU ASP SER VAL PHE SER SER SER GLU ARG

     ALA HIS GLN VAL LEU ARG ILE ARG LYS ARG ALA ASN SER PHE LEU GLU

     GLU LEU ARG HIS SER SER LEU GLU ARG GLU CYS ILE GLU GLU ILE CYS

     ASP PHE GLU GLU ALA LYS GLU ILE PHE GLN ASN VAL ASP ASP THR LEU
```

```
ALA PHE TRP SER LYS HIS VAL ASP GLY ASP GLN CYS LEU VAL LEU PRO

LEU GLU HIS PRO CYS ALA SER LEU CYS CYS GLY HIS GLY THR CYS ILE

ASP GLY ILE GLY SER PHE SER CYS ASP CYS ARG SER GLY TRP GLU GLY

ARG PHE CYS GLN ARG GLU VAL SER PHE LEU ASN CYS SER LEU ASP ASN

GLY GLY CYS THR HIS TYR CYS LEU GLU GLU VAL GLY TRP ARG ARG CYS

SER CYS ALA PRO GLY TYR LYS LEU GLY ASP ASP LEU LEU GLN CYS HIS

PRO ALA VAL LYS PHE PRO CYS GLY ARG PRO TRP LYS ARG MET GLU LYS

LYS ARG SER HIS LEU LYS ARG ASP THR GLU ASP GLN GLU ASP GLN VAL
```

$R_1$  $R_2$  ARG LEU ILE $R_3$  GLY LYS MET THR ARG ARG GLY ASP SER PRO

```
TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

wherein $-H_2N$ is the amino terminus; $R_1$ is PHE, GLY, TYR, or TRP; $R_2$ is PRO or VAL; and $R_3$ is ASP or ASN.

5. A recombinant DNA expression vector comprising the DNA compound of Claim 1, 2, 3, or 4.

6. The vector of Claim 5, wherein $R_1$ is PHE, $R_2$ is PRO, and $R_3$ is ASP.

7. The vector of Claim 6 that is plasmid pLPC-167F produced as described in Example 4.

**8.** The vector of Claim 5, wherein $R_1$ is GLY, $R_2$ is PRO, and $R_3$ is ASP.

**9.** The vector of Claim 8 that is plasmid pLPC-167G produced as described in Example 3.

**10.** A eukaryotic host cell transformed with a vector of Claim 5.

**11.** The eukaryotic host cell of Claim 10 that is 293/pLPC-167F, produced by transforming Human Embryonic Kidney Cell Line 293 with plasmid pLPC-167F as defined in Claim 7.

**12.** The eukaryotic host cell of Claim 10 that is 293/pLPC-167G, produced by transforming Human Embryonic Kidney Cell Line 293 with plasmid pLPC-167G as defined in Claim 9.

**13.** The eukaryotic host cell of Claim 10 that is AV12/pLPC-167F produced by transforming Adenovirus-transformed Syrian Hamster Cell Line AV12 with plasmid pLPC-167F as defined in Claim 7.

**14.** The eukaryotic host cell of Claim 10 that is AV12/pLPC-167G, produced by transforming Adenovirus-transformed Syrian Hamster Cell Line AV12 with plasmid pLPC-167G as defined in Claim 9.

**15.** A method for the recombinant production of a zymogen form of human protein C upon secretion from a eukaryotic host cell, which comprises
   (A) transforming a eukaryotic host cell with a recombinant DNA vector which comprises:
      (i) a DNA sequence that encodes an amino acid residue sequence that comprises, from the amino terminus to the carboxy terminus:
         a) a signal peptide and pro-peptide of a $\gamma$-carboxylated, secreted protein;
         b) the light chain of human protein C;
         c) the dipeptide LYS-ARG, ARG-LYS, LYS-LYS, or ARG-ARG; and

d) the amino acid residue sequence:

```
                                    ASP THR GLU ASP GLN GLU ASP GLN VAL

R₁  R₂  ARG LEU ILE R₃  GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

wherein R₁ is PHE, GLY, TYR, or TRP, R₂ is VAL or PRO, R₃ is ASP or ASN, ARG is Arginine, ASN is Asparagine, ASP is Aspartic acid, -COOH is the carboxy terminus, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine; and
(ii) a promoter positioned to drive expression of the DNA sequence; and
(B) culturing the host cell transformed in step (A) under conditions that allow for expression of the DNA sequence.

16. The method of Claim 15, wherein the recombinant DNA expression vector is plasmid pLPC-167F, as defined in Claim 7.

17. The method of Claim 15, wherein the recombinant DNA expression vector is plasmid pLPC-167G, as defined in Claim 9.

18. The method of Claim 15, wherein the host cell is the 293 or AV12 host cell.

19. The method of Claim 18, wherein the host cell cultured in step (B) is the 293/pLPC-167F, 293/pLPC-167G, AV12/pLPC-167F, or AV12/pLPC-167G host cell, as defined in Claims 11 to 14.

**20.** A protein C zymogen with the amino acid residue sequence:

```
H₂N-MET TRP GLN LEU THR SER LEU LEU LEU PHE VAL ALA THR TRP GLY ILE

      SER GLY THR PRO ALA PRO LEU ASP SER VAL PHE SER SER SER GLU ARG

      ALA HIS GLN VAL LEU ARG ILE ARG LYS ARG ALA ASN SER PHE LEU GLU

      GLU LEU ARG HIS SER SER LEU GLU ARG GLU CYS ILE GLU GLU ILE CYS

      ASP PHE GLU GLU ALA LYS GLU ILE PHE GLN ASN VAL ASP ASP THR LEU

      ALA PHE TRP SER LYS HIS VAL ASP GLY ASP GLN CYS LEU VAL LEU PRO
```

```
LEU GLU HIS PRO CYS ALA SER LEU CYS CYS GLY HIS GLY THR CYS ILE

ASP GLY ILE GLY SER PHE SER CYS ASP CYS ARG SER GLY TRP GLU GLY

ARG PHE CYS GLN ARG GLU VAL SER PHE LEU ASN CYS SER LEU ASP ASN

GLY GLY CYS THR HIS TYR CYS LEU GLU GLU VAL GLY TRP ARG ARG CYS

SER CYS ALA PRO GLY TYR LYS LEU GLY ASP ASP LEU LEU GLN CYS HIS

PRO ALA VAL LYS PHE PRO CYS GLY ARG PRO TRP LYS ARG MET GLU LYS

LYS ARG SER HIS LEU LYS ARG ASP THR GLU ASP GLN GLU ASP GLN VAL

R₁  R₂  ARG LEU ILE R₃  GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

wherein $H_2N$ is the amino terminus, $R_1$ is PHE, GLY, TYR, or TRP, $R_2$ is VAL or PRO, $R_3$ is ASP or ASN, ARG is Arginine, ASN is Asparagine, ASP is Aspartic acid, -COOH is the carboxy terminus, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine.

**21.** The zymogen of Claim 20, wherein $R_1$ is PHE, $R_2$ is PRO, and $R_3$ is ASP.

**22.** The zymogen of Claim 20, wherein $R_1$ is GLY, $R_2$ is PRO, and $R_3$ is ASP.

23. An activated protein C molecule comprising the light chain of activated human protein C and the heavy chain:

$H_2N$-LEU ILE ASN GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH

wherein ALA is Alanine, ARC is Arginine, ASN is Asparagine, ASP is Aspartic acid, -COOH is the carboxy terminus, CYS is Cysteine, GLN is Glutamine, GLU is Glutamic Acid, GLY is Glycine, -$H_2N$ is the amino terminus, HIS is Histidine, $H_2N$-is the amino terminus, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine.

24. A pharmaceutical formulation which comprises the zymogen of Claim 20 associated with a pharmaceutically-acceptable carrier therefor.

25. A pharmaceutical formulation which comprises the activated protein C of Claim 23 associated with a pharmaceutically-acceptable carrier therefor.

**Claims for the following Contracting State : ES**

1. A process for preparing a recombinant DNA vector that comprises ligating together (I) a DNA compound comprising a coding sequence for a protein that comprises from the amino terminus to the carboxy terminus:
   a) a signal peptide and propeptide of a γ-carboxylated, secreted protein;
   b) the light chain of human protein C;
   c) the dipeptide lysine-arginine, arginine-lysine, lysine-lysine, or arginine-arginine; and

d) the amino acid residue sequence:

```
                                        ASP THR GLU ASP GLN GLU ASP GLN VAL

 R₁  R₂  ARG LEU ILE R₃  GLY LYS MET THR ARG ARG GLY ASP SER PRO

 TRP GLN VAL VAL LEU LEU ASP SER LYS LYS LYS LEU ALA CYS GLY ALA

 VAL LEU ILE HIS PRO SER TRP VAL LEU THR ALA ALA HIS CYS MET ASP

 GLU SER LYS LYS LEU LEU VAL ARG LEU GLY GLU TYR ASP LEU ARG ARG

 TRP GLU LYS TRP GLU LEU ASP LEU ASP ILE LYS GLU VAL PHE VAL HIS

 PRO ASN TYR SER LYS SER THR THR ASP ASN ASP ILE ALA LEU LEU HIS

 LEU ALA GLN PRO ALA THR LEU SER GLN THR ILE VAL PRO ILE CYS LEU

 PRO ASP SER GLY LEU ALA GLU ARG GLU LEU ASN GLN ALA GLY GLN GLU

 THR LEU VAL THR GLY TRP GLY TYR HIS SER SER ARG GLU LYS GLU ALA

 LYS ARG ASN ARG THR PHE VAL LEU ASN PHE ILE LYS ILE PRO VAL VAL

 PRO HIS ASN GLU CYS SER GLU VAL MET SER ASN MET VAL SER GLU ASN

 MET LEU CYS ALA GLY ILE LEU GLY ASP ARG GLN ASP ALA CYS GLU GLY


 ASP SER GLY GLY PRO MET VAL ALA SER PHE HIS GLY THR TRP PHE LEU

 VAL GLY LEU VAL SER TRP GLY GLU GLY CYS GLY LEU LEU HIS ASN TYR

 GLY VAL TYR THR LYS VAL SER ARG TYR LEU ASP TRP ILE HIS GLY HIS

 ILE ARG ASP LYS GLU ALA PRO GLN LYS SER TRP ALA PRO-COOH
```

wherein R₁ is PHE, GLY, TYR, or TRP, R₂ is VAL or PRO, R₃ is ASP or ASN, ARG is Arginine, ASN is Asparagine, ASP is Aspartic acid, -COOH is the carboxy terminus, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine, and (II) a DNA compound comprising a promoter, such that the promoter is positioned to drive expression of the coding sequence upon ligation.

2. The process of Claim 1, wherein the polypeptide encoded by the DNA is:

```
H₂N-MET TRP GLN LEU THR SER LEU LEU LEU PE   VAL ALA THR TRP GLY ILE
      SER GLY THR PRO ALA PRO LEU ASP SER VAL PHE SER SER SER GLU ARG
      ALA HIS GLN VAL LEU ARG ILE ARG LYS ARG ALA ASN SER PHE LEU GLU
      GLU LEU ARG HIS SER SER LEU GLU ARG GLU CYS ILE GLU GLU ILE CYS
      ASP PHE GLU GLU ALA LYS GLU ILE PHE GLN ASN VAL ASP ASP THR LEU
      ALA PHE TRP SER LYS HIS VAL ASP GLY ASP GLN CYS LEU VAL LEU PRO
      LEU GLU HIS PRO CYS ALA SER LEU CYS CYS GLY HIS GLY THR CYS ILE
      ASP GLY ILE GLY SER PHE SER CYS ASP CYS ARG SER GLY TRP GLU GLY
      ARG PHE CYS GLN ARG GLU VAL SER PHE LEU ASN CYS SER LEU ASP ASN
      GLY GLY CYS THR HIS TYR CYS LEU GLU GLU VAL GLY TRP ARG ARG CYS
```

```
SER CYS ALA PRO GLY TYR LYS LEU GLY ASP ASP LEU LEU GLN CYS HIS

PRO ALA VAL LYS PHE PRO CYS GLY ARG PRO TRP LYS ARG MET GLU LYS

LYS ARG SER HIS LEU LYS ARG ASP THR GLU ASP GLN GLU ASP GLN VAL

R₁  R₂  ARG LEU ILE R₃  GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

wherein -H₂N is the amino terminus; R₁ is PHE, GLY, TYR, or TRP; R₂ is PRO or VAL; and R₃ is ASP or ASN.

3. The process of Claim 1 that yields plasmid pLPC-167F, said plasmid being defined as described in Example 4.

4. The process of Claim 1 that yields plasmid pLPC-167G, said plasmid being defined as described in Example 3.

5. A method for the recombinant production of a zymogen form of human protein C upon secretion from a eukaryotic host cell, which comprises
   (A) transforming a eukaryotic host cell with a recombinant DNA vector that comprises:
     (i) a DNA sequence that encodes an amino acid residue sequence that comprises, from the amino terminus to the carboxy terminus:
       a) a signal peptide and pro-peptide of a γ-carboxylated, secreted protein;
       b) the light chain of human protein C;
       c) the dipeptide LYS-ARG, ARG-LYS, LYS-LYS, or ARG-ARG; and

d) the amino acid residue sequence:

```
                                    ASP THR GLU ASP GLN GLU ASP GLN VAL

R₁  R₂  ARG LEU ILE R₃  GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY


ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

wherein R₁ is PHE, GLY, TYR, or TRP, R₂ is VAL or PRO, R₃ is ASP or ASN, ARG is Arginine, ASN is Asparagine, ASP is Aspartic acid, -COOH is the carboxy terminus, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine; and

(ii) a promoter positioned to drive expression of the DNA sequence; and

(B) culturing the host cell transformed in step (A) under conditions that allow for expression of the DNA sequence.

6. The method of Claim 5, wherein the recombinant DNA expression vector is plasmid pLPC-167F, as defined in Claim 3.

7. The method of Claim 5, wherein the recombinant DNA expression vector is plasmid pLPC-167G, as defined in Claim 4.

8. The method of Claim 5, 6, or 7 wherein the host cell is the 293 or AV12 host cell.

9. The method of Claim 8, wherein the host cell cultured in step (B) is
293/pLPC-167F, produced by transforming Human Embryonic Kidney Cell Line 293 with plasmid pLPC-167F defined in Claim 3,

293/pLPC-167G, produced by transforming Human Embryonic Kidney Cell Line 293 with plasmid pLPC-167G defined in Claim 4,

AV12/pLPC-167F, produced by transforming Adenovirus-transformed Syrian Hamster Cell Line AV12 with plasmid pLPC-167F defined in Claim 3, or

AV12/pLPC-167G, produced by transforming Adenovirus-transformed Syrian Hamster Cell Line AV12 with plasmid pLPC-167G defined in Claim 4.

**Claims for the following Contracting State : GR**

1.  A DNA compound comprising a coding sequence for a protein that comprises from the amino terminus to the carboxy terminus:

a) a signal peptide and propeptide of a $\gamma$-carboxylated, secreted protein;

b) the light chain of human protein C;

c) the dipeptide lysine-arginine, arginine-lysine, lysine-lysine, or arginine-arginine; and

d) the amino acid residue sequence:

$$\begin{array}{llllllll}
 & & & \text{ASP} & \text{THR} & \text{GLU} & \text{ASP} & \text{GLN} & \text{GLU} & \text{ASP} & \text{GLN} & \text{VAL} \\
\end{array}$$

R$_1$ R$_2$ ARG LEU ILE R$_3$ GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH

wherein R$_1$ is PHE, GLY, TYR, or TRP, R$_2$ is VAL or PRO, R$_3$ is ASP or ASN, ARG is Arginine, ASN is Asparagine, ASP is Aspartic acid, -COOH is the carboxy terminus, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine.

2. The DNA compound of Claim 1, wherein the signal peptide and propeptide are the signal peptide and propeptide of nascent human protein C.

3. The DNA compound of Claim 1 or 2, wherein the dipeptide is lysine-arginine.

4. The DNA compound of Claim 3, wherein the polypeptide encoded by the DNA is:

```
H₂N-MET TRP GLN LEU THR SER LEU LEU LEU PHE VAL ALA THR TRP GLY ILE

     SER GLY THR PRO ALA PRO LEU ASP SER VAL PHE SER SER SER GLU ARG

     ALA HIS GLN VAL LEU ARG ILE ARG LYS ARG ALA ASN SER PHE LEU GLU

     GLU LEU ARG HIS SER SER LEU GLU ARG GLU CYS ILE GLU GLU ILE CYS

     ASP PHE GLU GLU ALA LYS GLU ILE PHE GLN ASN VAL ASP ASP THR LEU
```

```
ALA PHE TRP SER LYS HIS VAL ASP GLY ASP GLN CYS LEU VAL LEU PRO

LEU GLU HIS PRO CYS ALA SER LEU CYS CYS GLY HIS GLY THR CYS ILE

ASP GLY ILE GLY SER PHE SER CYS ASP CYS ARG SER GLY TRP GLU GLY

ARG PHE CYS GLN ARG GLU VAL SER PHE LEU ASN CYS SER LEU ASP ASN

GLY GLY CYS THR HIS TYR CYS LEU GLU GLU VAL GLY TRP ARG ARG CYS

SER CYS ALA PRO GLY TYR LYS LEU GLY ASP ASP LEU LEU GLN CYS HIS

PRO ALA VAL LYS PHE PRO CYS GLY ARG PRO TRP LYS ARG MET GLU LYS

LYS ARG SER HIS LEU LYS ARG ASP THR GLU ASP GLN GLU ASP GLN VAL
```

$R_1$  $R_2$  ARG LEU ILE $R_3$  GLY LYS MET THR ARG ARG GLY ASP SER PRO

```
TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

wherein -$H_2$N is the amino terminus; $R_1$ is PHE, GLY, TYR, or TRP; $R_2$ is PRO or VAL; and $R_3$ is ASP or ASN.

**5.** A recombinant DNA expression vector comprising the DNA compound of Claim 1, 2, 3, or 4.

**6.** The vector of Claim 5, wherein $R_1$ is PHE, $R_2$ is PRO, and $R_3$ is ASP.

**7.** The vector of Claim 6 that is plasmid pLPC-167F produced as described in Example 4.

8. The vector of Claim 5, wherein $R_1$ is GLY, $R_2$ is PRO, and $R_3$ is ASP.

9. The vector of Claim 8 that is plasmid pLPC-167G produced as described in Example 3.

10. A eukaryotic host cell transformed with a vector of Claim 5.

11. The eukaryotic host cell of Claim 10 that is 293/pLPC-167F, produced by transforming Human Embryonic Kidney Cell Line 293 with plasmid pLPC-167F as defined in Claim 7.

12. The eukaryotic host cell of Claim 10 that is 293/pLPC-167G, produced by transforming Human Embryonic Kidney Cell Line 293 with plasmid pLPC-167G as defined in Claim 9.

13. The eukaryotic host cell of Claim 10 that is AV12/pLPC-167F produced by transforming Adenovirus-transformed Syrian Hamster Cell Line AV12 with plasmid pLPC-167F as defined in Claim 7.

14. The eukaryotic host cell of Claim 10 that is AV12/pLPC-167G, produced by transforming Adenovirus-transformed Syrian Hamster Cell Line AV12 with plasmid pLPC-167G as defined in Claim 9.

15. A method for the recombinant production of a zymogen form of human protein C upon secretion from a eukaryotic host cell, which comprises
    (A) transforming a eukaryotic host cell with a recombinant DNA vector which comprises:
        (i) a DNA sequence that encodes an amino acid residue sequence that comprises, from the amino terminus to the carboxy terminus:
            a) a signal peptide and pro-peptide of a $\gamma$-carboxylated, secreted protein;
            b) the light chain of human protein C;
            c) the dipeptide LYS-ARG, ARG-LYS, LYS-LYS, or ARG-ARG; and

d) the amino acid residue sequence:

```
                                      ASP THR GLU ASP GLN GLU ASP GLN VAL

    R₁  R₂  ARG LEU ILE R₃  GLY LYS MET THR ARG ARG GLY ASP SER PRO

    TRP GLN VAL VAL LEU LEU ASP SER LYS LYS LYS LEU ALA CYS GLY ALA

    VAL LEU ILE HIS PRO SER TRP VAL LEU THR ALA ALA HIS CYS MET ASP

    GLU SER LYS LYS LEU LEU VAL ARG LEU GLY GLU TYR ASP LEU ARG ARG

    TRP GLU LYS TRP GLU LEU ASP LEU ASP ILE LYS GLU VAL PHE VAL HIS

    PRO ASN TYR SER LYS SER THR THR ASP ASN ASP ILE ALA LEU LEU HIS

    LEU ALA GLN PRO ALA THR LEU SER GLN THR ILE VAL PRO ILE CYS LEU

    PRO ASP SER GLY LEU ALA GLU ARG GLU LEU ASN GLN ALA GLY GLN GLU

    THR LEU VAL THR GLY TRP GLY TYR HIS SER SER ARG GLU LYS GLU ALA

    LYS ARG ASN ARG THR PHE VAL LEU ASN PHE ILE LYS ILE PRO VAL VAL

    PRO HIS ASN GLU CYS SER GLU VAL MET SER ASN MET VAL SER GLU ASN

    MET LEU CYS ALA GLY ILE LEU GLY ASP ARG GLN ASP ALA CYS GLU GLY

    ASP SER GLY GLY PRO MET VAL ALA SER PHE HIS GLY THR TRP PHE LEU

    VAL GLY LEU VAL SER TRP GLY GLU GLY CYS GLY LEU LEU HIS ASN TYR

    GLY VAL TYR THR LYS VAL SER ARG TYR LEU ASP TRP ILE HIS GLY HIS

    ILE ARG ASP LYS GLU ALA PRO GLN LYS SER TRP ALA PRO-COOH
```

wherein R₁ is PHE, GLY, TYR, or TRP, R₂ is VAL or PRO, R₃ is ASP or ASN, ARG is Arginine, ASN is Asparagine, ASP is Aspartic acid, -COOH is the carboxy terminus, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine; and

(ii) a promoter positioned to drive expression of the DNA sequence; and

(B) culturing the host cell transformed in step (A) under conditions that allow for expression of the DNA sequence.

16. The method of Claim 15, wherein the recombinant DNA expression vector is plasmid pLPC-167F, as defined in Claim 7.

17. The method of Claim 15, wherein the recombinant DNA expression vector is plasmid pLPC-167G, as defined in Claim 9.

18. The method of Claim 15, wherein the host cell is the 293 or AV12 host cell.

19. The method of Claim 18, wherein the host cell cusltured in step (B) is the 293/pLPC-167F, 293/pLPC-167G, AV12/pLPC-167F, or AV12/pLPC-167G host cell, as defined in Claims 11 to 14.

**20.** A pharmaceutical formulation which comprises a protein C zymogen with the amino acid residue sequence:

```
H₂N-MET TRP GLN LEU THR SER LEU LEU LEU PHE VAL ALA THR TRP GLY ILE

     SER GLY THR PRO ALA PRO LEU ASP SER VAL PHE SER SER SER GLU ARG

     ALA HIS GLN VAL LEU ARG ILE ARG LYS ARG ALA ASN SER PHE LEU GLU

     GLU LEU ARG HIS SER SER LEU GLU ARG GLU CYS ILE GLU GLU ILE CYS

     ASP PHE GLU GLU ALA LYS GLU ILE PHE GLN ASN VAL ASP ASP THR LEU

     ALA PHE TRP SER LYS HIS VAL ASP GLY ASP GLN CYS LEU VAL LEU PRO

     LEU GLU HIS PRO CYS ALA SER LEU CYS CYS GLY HIS GLY THR CYS ILE

     ASP GLY ILE GLY SER PHE SER CYS ASP CYS ARG SER GLY TRP GLU GLY

     ARG PHE CYS GLN ARG GLU VAL SER PHE LEU ASN CYS SER LEU ASP ASN

     GLY GLY CYS THR HIS TYR CYS LEU GLU GLU VAL GLY TRP ARG ARG CYS

     SER CYS ALA PRO GLY TYR LYS LEU GLY ASP ASP LEU LEU GLN CYS HIS

     PRO ALA VAL LYS PHE PRO CYS GLY ARG PRO TRP LYS ARG MET GLU LYS

     LYS ARG SER HIS LEU LYS ARG ASP THR GLU ASP GLN GLU ASP GLN VAL

     R₁  R₂  ARG LEU ILE R₃  GLY LYS MET THR ARG ARG GLY ASP SER PRO

     TRP GLN VAL VAL LEU LEU ASP SER LYS LYS LYS LEU ALA CYS GLY ALA
```

```
VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

wherein H$_2$N is the amino terminus, R$_1$ is PHE, GLY, TYR, or TRP, R$_2$ is VAL or PRO, R$_3$ is ASP or ASN, ARG is Arginine, ASN is Asparagine, ASP is Aspartic acid, -COOH is the carboxy terminus, CYS is Cysteine, GLN is Glutamine, GLU is Glutamic Acid, GLY is Glycine, HIS is Histidine, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine, associated with a pharmaceutically-acceptable carrier therefor.

21. A pharmaceutical formulation which comprises an activated protein C molecule comprising the light chain of activated human protein C and the heavy chain:

56

```
            H₂N-LEU ILE ASN GLY LYS MET THR ARG ARG GLY ASP SER PRO

      TRP GLN VAL VAL LEU LEU ASP SER LYS LYS LYS LEU ALA CYS GLY ALA

      VAL LEU ILE HIS PRO SER TRP VAL LEU THR ALA ALA HIS CYS MET ASP

      GLU SER LYS LYS LEU LEU VAL ARG LEU GLY GLU TYR ASP LEU ARG ARG

      TRP GLU LYS TRP GLU LEU ASP LEU ASP ILE LYS GLU VAL PHE VAL HIS

      PRO ASN TYR SER LYS SER THR THR ASP ASN ASP ILE ALA LEU LEU HIS

      LEU ALA GLN PRO ALA THR LEU SER GLN THR ILE VAL PRO ILE CYS LEU

      PRO ASP SER GLY LEU ALA GLU ARG GLU LEU ASN GLN ALA GLY GLN GLU

      THR LEU VAL THR GLY TRP GLY TYR HIS SER SER ARG GLU LYS GLU ALA

      LYS ARG ASN ARG THR PHE VAL LEU ASN PHE ILE LYS ILE PRO VAL VAL

      PRO HIS ASN GLU CYS SER GLU VAL MET SER ASN MET VAL SER GLU ASN

      MET LEU CYS ALA GLY ILE LEU GLY ASP ARG GLN ASP ALA CYS GLU GLY

      ASP SER GLY GLY PRO MET VAL ALA SER PHE HIS GLY THR TRP PHE LEU

      VAL GLY LEU VAL SER TRP GLY GLU GLY CYS GLY LEU LEU HIS ASN TYR

      GLY VAL TYR THR LYS VAL SER ARG TYR LEU ASP TRP ILE HIS GLY HIS

      ILE ARG ASP LYS GLU ALA PRO GLN LYS SER TRP ALA PRO-COOH
```

wherein ALA is Alanine, ARG is Arginine, ASN is Asparagine, ASP is Aspartic acid, -COOH is the carboxy terminus, CYS is Cysteine, GLN is Glutamine, GLU is Glutamic Acid, GLY is Glycine, -H₂N is the amino terminus, HIS is Histidine, H₂N-is the amino terminus, ILE is Isoleucine, LEU is Leucine, LYS is Lysine, MET is Methionine, PHE is Phenylalanine, PRO is Proline, SER is Serine, THR is Threonine, TRP is Tryptophan, TYR is Tyrosine, and VAL is Valine, associated with a pharmaceutically-acceptable carrier therefor.

**Patentansprüche**

**Patentansprüche für folgende Vertragsstaaten : AT, BE, CH, DE, FR, GB, IT, LI, LU, NL, SE**

1. DNA Verbindung, die eine kodierende Sequenz für ein Protein umfaßt, das vom Aminoterminus bis zum Carboxyterminus umfaßt:
   a) ein Signalpeptid und Propeptid eines gamma-carboxylierten, sekretierten Proteins,
   b) die leichte Kette von humanem Protein C,
   c) das Dipeptid Lysin-Arginin, Arginin-Lysin, Lysin-Lysin oder Arginin-Arginin, und

d) folgende Aminosäuresequenz

```
                                    ASP THR GLU ASP GLN GLU ASP GLN VAL

R₁   R₂   ARG LEU ILE R₃   GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

worin R₁ für PHE, GLY, TYR oder TRP steht, R₂ für VAL oder PRO steht, R₃ für ASP oder ASN steht, ARG für Arginin steht, ASN für Asparagin steht, ASP für Asparaginsäure steht, -COOH für den Carboxyterminus steht, CYS für Cystein steht, GLN für Glutamin steht, GLU für Glutaminsäure steht, GLY für Glycin steht, HIS für Histidin steht, ILE für Isoleucin steht, LEU für Leucin steht, LYS für Lysin steht, MET für Methionin steht, PHE für Phenylalanin steht, PRO für Prolin steht, SER für Serin steht, THR für Threonin steht, TRP für Tryptophan steht, TYR für Tyrosin steht und VAL für Valin steht.

2. DNA Verbindung nach Anspruch 1, worin das Signalpeptid und das Propeptid das Signalpeptid und das Propeptid von unreifem humanem Protein C sind.

3. DNA Verbindung nach Anspruch 1 oder 2, worin das Dipeptid Lysin-Arginin ist.

4. DNA Verbindung nach Anspruch 3, worin das durch die DNA kodierte Polypeptid folgendes ist

```
H₂N-MET TRP GLN LEU THR SER LEU LEU LEU PHE VAL ALA THR TRP GLY ILE

     SER GLY THR PRO ALA PRO LEU ASP SER VAL PHE SER SER SER GLU ARG

     ALA HIS GLN VAL LEU ARG ILE ARG LYS ARG ALA ASN SER PHE LEU GLU

     GLU LEU ARG HIS SER SER LEU GLU ARG GLU CYS ILE GLU GLU ILE CYS

     ASP PHE GLU GLU ALA LYS GLU ILE PHE GLN ASN VAL ASP ASP THR LEU

     ALA PHE TRP SER LYS HIS VAL ASP GLY ASP GLN CYS LEU VAL LEU PRO

     LEU GLU HIS PRO CYS ALA SER LEU CYS CYS GLY HIS GLY THR CYS ILE

     ASP GLY ILE GLY SER PHE SER CYS ASP CYS ARG SER GLY TRP GLU GLY

     ARG PHE CYS GLN ARG GLU VAL SER PHE LEU ASN CYS SER LEU ASP ASN

     GLY GLY CYS THR HIS TYR CYS LEU GLU GLU VAL GLY TRP ARG ARG CYS

     SER CYS ALA PRO GLY TYR LYS LEU GLY ASP ASP LEU LEU GLN CYS HIS

     PRO ALA VAL LYS PHE PRO CYS GLY ARG PRO TRP LYS ARG MET GLU LYS

     LYS ARG SER HIS LEU LYS ARG ASP THR GLU ASP GLN GLU ASP GLN VAL

     R₁  R₂  ARG LEU ILE R₃  GLY LYS MET THR ARG ARG GLY ASP SER PRO

     TRP GLN VAL VAL LEU LEU ASP SER LYS LYS LYS LEU ALA CYS GLY ALA
```

```
VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

worin -$H_2N$ für den Aminoterminus steht, $R_1$ für PHE, GLY, TYR oder TRP steht, $R_2$ für PRO oder VAL steht und $R_3$ für ASP oder ASN steht.

5. Rekombinanter DNA Expressionsvektor, der die DNA Verbindung nach Anspruch 1, 2, 3 oder 4 umfaßt.

6. Vektor nach Anspruch 5, worin $R_1$ für PHE steht, $R_2$ für PRO steht und $R_3$ für ASP steht.

7. Vektor nach Anspruch 6, der das wie in Beispiel 4 beschrieben hergestellte Plasmid pLPC-167F ist.

8. Vektor nach Anspruch 5, worin $R_1$ für GLY steht, $R_2$ für PRO steht und $R_3$ für ASP steht.

9. Vektor nach Anspruch 8, der das wie in Beispiel 3 beschrieben hergestellte Plasmid pLPC-167G ist.

10. Eukaryontische Wirtszelle, die mit einem Vektor nach Anspruch 5 transformiert ist.

11. Eukaryontische Wirtszelle nach Anspruch 10, die 293/pLPC-167F ist, welche durch Transformation der humanen embryonalen Nierenzellinie 293 mit dem Plasmid pLPC-167F nach Anspruch 7 hergestellt ist.

12. Eukaryontische Wirtszelle nach Anspruch 10, die 293/pLPC-167G ist, welche durch Transformation der humanen embryonalen Nierenzellinie 293 mit dem Plasmid pLPC-167G nach Anspruch 9 hergestellt ist.

13. Eukaryontische Wirtszelle nach Anspruch 10, die AV12/pLPC-167F ist, welche durch Transformation der Adenovirus-transformierten syrischen Hamsterzellinie AV12 mit dem Plasmid pLPC-167F nach Anspruch 7 hergestellt ist.

14. Eukaryontische Wirtszelle nach Anspruch 10, die AV12/pLPC-167G ist, welche durch Transformation der Adenovirus-transformierten syrischen Hamsterzellinie AV12 mit dem Plasmid pLPC-167G nach Anspruch 9 hergestellt ist.

**15.** Verfahren zur rekombinanten Herstellung einer Zymogenform von humanem Protein C durch Sekretion aus einer eukaryontischen Wirtszelle, gekennzeichnet durch

(A) Transformation einer eukaryontischen Wirtszelle mit einem rekombinanten DNA Vektor, der umfaßt

(i) eine DNA Sequenz, die eine Aminosäuresequenz kodiert, welche vom Aminoterminus zum Carboxyterminus umfaßt

a) ein Signalpeptid und ein Propeptid eines gamma-carboxylierten, sekretierten Proteins,
b) die leichte Kette von humanem Protein C,
c) das Dipeptid LYS-ARG, ARG-LYS, LYS-LYS oder ARG-ARG, und
d) folgende Aminosäuresequenz

$$\begin{array}{llllllll} & & & & & & \text{ASP} & \text{THR} & \text{GLU} & \text{ASP} & \text{GLN} & \text{GLU} & \text{ASP} & \text{GLN} & \text{VAL} \end{array}$$

$$R_1 \quad R_2 \quad \text{ARG} \quad \text{LEU} \quad \text{ILE} \quad R_3 \quad \text{GLY} \quad \text{LYS} \quad \text{MET} \quad \text{THR} \quad \text{ARG} \quad \text{ARG} \quad \text{GLY} \quad \text{ASP} \quad \text{SER} \quad \text{PRO}$$

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH

worin $R_1$ für PHE, GLY, TYR oder TRP steht, $R_2$ für VAL oder PRO steht, $R_3$ für ASP oder ASN steht, ARG für Arginin steht, ASN für Asparagin steht, ASP für Asparaginsäure steht, -COOH für den Carboxyterminus steht, CYS für Cystein steht, GLN für Glutamin steht, GLU für Glutaminsäure steht, GLY für Glycin steht, HIS für Histidin steht, ILE für Isoleucin steht, LEU für Leucin steht, LYS für Lysin steht, MET für Methionin steht, PHE für Phenylalanin steht, PRO für Prolin steht, SER für Serin steht, THR für Threonin steht, TRP für Tryptophan steht, TYR für Tyrosin steht und VAL für Valin steht, und

(ii) einen Promotor, der zur Expressionssteuerung der DNA Sequenz positioniert ist, und

(B) Kultivierung der in Schritt (A) transformierten Wirtszelle unter Bedingungen, die die Expression der DNA Sequenz erlauben.

**16.** Verfahren nach Anspruch 15, worin der rekombinante DNA Expressionsvektor das Plasmid pLPC-167F nach Anspruch 7 ist.

**17.** Verfahren nach Anspruch 15, worin der rekombinante DNA Expressionsvektor das Plasmid pLPC-167G nach Anspruch 9 ist.

**18.** Verfahren nach Anspruch 15, worin die Wirtszelle die 293- oder AV12-Wirtszelle ist.

**19.** Verfahren nach Anspruch 18, worin die in Schritt (B) kultivierte Wirtszelle die 293/pLPC-167F, 293/pLPC-167G, AV12/pLPC-167F oder AV12/pLPC-167G Wirtszelle nach den Ansprüchen 11 bis 14 ist.

**20.** Protein C Zymogen mit der folgenden Aminosäuresequenz

$H_2$N-MET TRP GLN LEU THR SER LEU LEU LEU PHE VAL ALA THR TRP GLY ILE

SER GLY THR PRO ALA PRO LEU ASP SER VAL PHE SER SER SER GLU ARG

ALA HIS GLN VAL LEU ARG ILE ARG LYS ARG ALA ASN SER PHE LEU GLU

GLU LEU ARG HIS SER SER LEU GLU ARG GLU CYS ILE GLU GLU ILE CYS

ASP PHE GLU GLU ALA LYS GLU ILE PHE GLN ASN VAL ASP ASP THR LEU

ALA PHE TRP SER LYS HIS VAL ASP GLY ASP GLN CYS LEU VAL LEU PRO

LEU GLU HIS PRO CYS ALA SER LEU CYS CYS GLY HIS GLY THR CYS ILE

ASP GLY ILE GLY SER PHE SER CYS ASP CYS ARG SER GLY TRP GLU GLY

ARG PHE CYS GLN ARG GLU VAL SER PHE LEU ASN CYS SER LEU ASP ASN

GLY GLY CYS THR HIS TYR CYS LEU GLU GLU VAL GLY TRP ARG ARG CYS

SER CYS ALA PRO GLY TYR LYS LEU GLY ASP ASP LEU LEU GLN CYS HIS

PRO ALA VAL LYS PHE PRO CYS GLY ARG PRO TRP LYS ARG MET GLU LYS

LYS ARG SER HIS LEU LYS ARG ASP THR GLU ASP GLN GLU ASP GLN VAL

$R_1$   $R_2$   ARG LEU ILE $R_3$   GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH

worin $H_2N$ für den Aminoterminus steht, $R_1$ für PHE, GLY, TYR oder TRP steht, $R_2$ für VAL oder PRO steht, $R_3$ für ASP oder ASN steht, ARG für Arginin steht, ASN für Asparagin steht, ASP für Asparaginsäure steht, -COOH für den Carboxyterminus steht, CYS für Cystein steht, GLN für Glutamin steht, GLU für Glutaminsäure steht, GLY für Glycin steht, HIS für Histidin steht, ILE für Isoleucin steht, LEU für Leucin steht, LYS für Lysin steht, MET für Methionin steht, PHE für Phenylalanin steht, PRO für Prolin steht, SER für Serin steht, THR für Threonin steht, TRP für Tryptophan steht, TYR für Tyrosin steht und VAL für Valin steht.

21. Zymogen nach Anspruch 20, worin $R_1$ für PHE steht, $R_2$ für PRO steht und $R_3$ für ASP steht.

22. Zymogen nach Anspruch 20, worin $R_1$ für GLY steht, $R_2$ für PRO steht und $R_3$ für ASP steht.

23. Aktiviertes Protein C Molekül, das die leichte Kette von aktiviertem humanem Protein C und die folgende schwere Kette umfaßt

```
          H₂N-LEU ILE ASN GLY LYS MET THR ARG ARG GLY ASP SER PRO

     TRP GLN VAL VAL LEU LEU ASP SER LYS LYS LYS LEU ALA CYS GLY ALA

     VAL LEU ILE HIS PRO SER TRP VAL LEU THR ALA ALA HIS CYS MET ASP

     GLU SER LYS LYS LEU LEU VAL ARG LEU GLY GLU TYR ASP LEU ARG ARG

     TRP GLU LYS TRP GLU LEU ASP LEU ASP ILE LYS GLU VAL PHE VAL HIS

     PRO ASN TYR SER LYS SER THR THR ASP ASN ASP ILE ALA LEU LEU HIS

     LEU ALA GLN PRO ALA THR LEU SER GLN THR ILE VAL PRO ILE CYS LEU

     PRO ASP SER GLY LEU ALA GLU ARG GLU LEU ASN GLN ALA GLY GLN GLU

     THR LEU VAL THR GLY TRP GLY TYR HIS SER SER ARG GLU LYS GLU ALA

     LYS ARG ASN ARG THR PHE VAL LEU ASN PHE ILE LYS ILE PRO VAL VAL

     PRO HIS ASN GLU CYS SER GLU VAL MET SER ASN MET VAL SER GLU ASN

     MET LEU CYS ALA GLY ILE LEU GLY ASP ARG GLN ASP ALA CYS GLU GLY

     ASP SER GLY GLY PRO MET VAL ALA SER PHE HIS GLY THR TRP PHE LEU

     VAL GLY LEU VAL SER TRP GLY GLU GLY CYS GLY LEU LEU HIS ASN TYR

     GLY VAL TYR THR LYS VAL SER ARG TYR LEU ASP TRP ILE HIS GLY HIS

     ILE ARG ASP LYS GLU ALA PRO GLN LYS SER TRP ALA PRO-COOH
```

worin ALA für Alanin steht, ARG für Arginin steht, ASN für Asparagin steht, ASP für Asparaginsäure steht, -COOH für den Carboxyterminus steht, CYS für Cystein steht, GLN für Glutamin steht, GLU für Glutaminsäure steht, GLY für Glycin steht, H₂N für den Aminoterminus steht, HIS für Histidin steht, ILE für Isoleucin steht, LEU für Leucin steht, LYS für Lysin steht, MET für Methionin steht, PHE für Phenylalanin steht, PRO für Prolin steht, SER für Serin steht, THR für Threonin steht, TRP für Tryptophan steht, TYR für Tyrosin steht und VAL für Valin steht.

**24.** Pharmazeutische Formulierung, die das Zymogen nach Anspruch 20 zusammen mit einem pharmazeutisch annehmbaren Träger hierfür umfaßt.

**25.** Pharmazeutische Formulierung, die das aktivierte Protein C nach Anspruch 23 zusammen mit einem pharmazeutisch annehmbaren Träger hierfür umfaßt.

**Patentansprüche für folgenden Vertragsstaat : ES**

**1.** Verfahren zur Herstellung eines rekombinanten DNA Vektors, gekennzeichnet durch Ligation (I) einer DNA Verbindung, die eine kodierende Sequenz für ein Protein umfaßt, das vom Aminoterminus bis zum Carboxyterminus umfaßt

a) ein Signalpeptid und Propeptid eines gamma-carboxylierten, sekretierten Proteins,

b) die leichte Kette von humanem Protein C,

c) das Dipeptid Lysin-Arginin, Arginin-Lysin, Lysin-Lysin oder Arginin-Arginin, und

d) folgende Aminosäuresequenz

```
                                    ASP THR GLU ASP GLN GLU ASP GLN VAL

R₁  R₂  ARG LEU ILE R₃  GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

worin R₁ für PHE, GLY, TYR oder TRP steht, R₂ für VAL oder PRO steht, R₃ für ASP oder ASN steht, ARG für Arginin steht, ASN für Asparagin steht, ASP für Asparaginsäure steht, -COOH für den Carboxyterminus steht, CYS für Cystein steht, GLN für Glutamin steht, GLU für Glutaminsäure steht, GLY für Glycin steht, HIS für Histidin steht, ILE für Isoleucin steht, LEU für Leucin steht, LYS für Lysin steht, MET für Methionin steht, PHE für Phenylalanin steht, PRO für Prolin steht, SER für Serin steht, THR für Threonin steht, TRP für Tryptophan steht, TYR für Tyrosin steht und VAL für Valin steht, und

(II) einer DNA Verbindung, die einen Promotor umfaßt, wobei der Promotor zur Expressionssteuerung der kodierenden Sequenz nach der Ligation positioniert ist.

2. Verfahren nach Anspruch 1, worin das durch die DNA kodierte Polypeptid folgendes ist

```
H₂N-MET TRP GLN LEU THR SER LEU LEU LEU PH. JAL ALA THR TRP GLY ILE

     SER GLY THR PRO ALA PRO LEU ASP SER VAL PHE SER SER SER GLU ARG

     ALA HIS GLN VAL LEU ARG ILE ARG LYS ARG ALA ASN SER PHE LEU GLU

     GLU LEU ARG HIS SER SER LEU GLU ARG GLU CYS ILE GLU GLU ILE CYS

     ASP PHE GLU GLU ALA LYS GLU ILE PHE GLN ASN VAL ASP ASP THR LEU

     ALA PHE TRP SER LYS HIS VAL ASP GLY ASP GLN CYS LEU VAL LEU PRO

     LEU GLU HIS PRO CYS ALA SER LEU CYS CYS GLY HIS GLY THR CYS ILE

     ASP GLY ILE GLY SER PHE SER CYS ASP CYS ARG SER GLY TRP GLU GLY

     ARG PHE CYS GLN ARG GLU VAL SER PHE LEU ASN CYS SER LEU ASP ASN

     GLY GLY CYS THR HIS TYR CYS LEU GLU GLU VAL GLY TRP ARG ARG CYS

     SER CYS ALA PRO GLY TYR LYS LEU GLY ASP ASP LEU LEU GLN CYS HIS

     PRO ALA VAL LYS PHE PRO CYS GLY ARG PRO TRP LYS ARG MET GLU LYS

     LYS ARG SER HIS LEU LYS ARG ASP THR GLU ASP GLN GLU ASP GLN VAL

     R₁  R₂  ARG LEU ILE R₃  GLY LYS MET THR ARG ARG GLY ASP SER PRO

     TRP GLN VAL VAL LEU LEU ASP SER LYS LYS LYS LEU ALA CYS GLY ALA

     VAL LEU ILE HIS PRO SER TRP VAL LEU THR ALA ALA HIS CYS MET ASP
```

```
GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

worin -H$_2$N für den Aminoterminus steht, R$_1$ für PHE, GLY, TYR oder TRP steht, R$_2$ für PRO oder VAL steht und R$_3$ für ASP oder ASN steht.

3. Verfahren nach Anspruch 1, das das Plasmid pLPC-167F ergibt, wobei dieses Plasmid wie in Beispiel 4 beschrieben definiert ist.

4. Verfahren nach Anspruch 1, das das Plasmid pLPC-167G ergibt, wobei dieses Plasmid wie in Beispiel 3 beschrieben definiert ist.

5. Verfahren zur rekombinanten Herstellung einer Zymogenform von humanem Protein C durch Sekretion aus einer eukaryontischen Wirtszelle, gekennzeichnet durch
    (A) Transformation einer eukaryontischen Wirtszelle mit einem rekombinanten DNA Vektor, der umfaßt
        (i) eine DNA Sequenz, die eine Aminosäuresequenz kodiert, welche vom Aminoterminus zum Carboxyterminus umfaßt
            a) ein Signalpeptid und ein Propeptid eines gamma-carboxylierten, sekretierten Proteins,
            b) die leichte Kette von humanem Protein C,
            c) das Dipeptid LYS-ARG, ARG-LYS, LYS-LYS oder ARG-ARG, und

d) folgende Aminosäuresequenz

```
                              ASP THR GLU ASP GLN GLU ASP GLN VAL

R₁  R₂  ARG LEU ILE R₃  GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

worin R₁ für PHE, GLY, TYR oder TRP steht, R₂ für VAL oder PRO steht, R₃ für ASP oder ASN steht, ARG für Arginin steht, ASN für Asparagin steht, ASP für Asparaginsäure steht, -COOH für den Carboxyterminus steht, CYS für Cystein steht, GLN für Glutamin steht, GLU für Glutaminsäure steht, GLY für Glycin steht, HIS für Histidin steht, ILE für Isoleucin steht, LEU für Leucin steht, LYS für Lysin steht, MET für Methionin steht, PHE für Phenylalanin steht, PRO für Prolin steht, SER für Serin steht, THR für Threonin steht, TRP für Tryptophan steht, TYR für Tyrosin steht und VAL für Valin steht, und

(ii) einen Promotor, der zur Expressionssteuerung der DNA Sequenz positioniert ist, und
(B) Kultivierung der in Schritt (A) transformierten Wirtszelle unter Bedingungen, die die Expression der DNA Sequenz erlauben.

6. Verfahren nach Anspruch 5, worin der rekombinante DNA Expressionsvektor das Plasmid pLPC-167F nach Anspruch 3 ist.

7. Verfahren nach Anspruch 5, worin der rekombinante DNA Expressionsvektor das Plasmid pLPC-167G nach Anspruch 4 ist.

8. Verfahren nach Anspruch 5, 6 oder 7, worin die Wirtszelle die 293- oder AV12-Wirtszelle ist.

9. Verfahren nach Anspruch 8, worin die in Schritt (B) kultivierte Wirtszelle ist
293/pLPC-167F, hergestellt durch Transformation der humanen embryonalen Nierenzellinie 293 mit dem Plasmid pLPC-167F nach Anspruch 3,

293/pLPC-167G, hergestellt durch Transformation der humanen embryonalen Nierenzellinie 293 mit dem Plasmid pLPC-167G nach Anspruch 4,

AV12/pLPC-167F, hergestellt durch Transformation der Adenovirustransformierten syrischen Hamsterzellinie AV12 mit dem Plasmid pLPC-167F nach Anspruch 3, oder

AV12/pLPC-167G hergestellt durch Transformation der Adenovirustransformierten syrischen Hamsterzellinie AV12 mit dem Plasmid pLPC-167G nach Anspruch 4.

**Patentansprüche für folgenden Vertragsstaat : GR**

1. DNA Verbindung, die eine kodierende Sequenz für ein Protein umfaßt, das vom Aminoterminus bis zum Carboxyterminus umfaßt

    a) ein Signalpeptid und Propeptid eines gamma-carboxylierten, sekretierten Proteins,

    b) die leichte Kette von humanem Protein C,

    c) das Dipeptid Lysin-Arginin, Arginin-Lysin, Lysin-Lysin oder Arginin-Arginin, und

    d) folgende Aminosäuresequenz

```
                          ASP THR GLU ASP GLN GLU ASP GLN VAL

        R₁  R₂  ARG LEU ILE R₃  GLY LYS MET THR ARG ARG GLY ASP SER PRO

        TRP GLN VAL VAL LEU LEU ASP SER LYS LYS LYS LEU ALA CYS GLY ALA

        VAL LEU ILE HIS PRO SER TRP VAL LEU THR ALA ALA HIS CYS MET ASP

        GLU SER LYS LYS LEU LEU VAL ARG LEU GLY GLU TYR ASP LEU ARG ARG

        TRP GLU LYS TRP GLU LEU ASP LEU ASP ILE LYS GLU VAL PHE VAL HIS

        PRO ASN TYR SER LYS SER THR THR ASP ASN ASP ILE ALA LEU LEU HIS

        LEU ALA GLN PRO ALA THR LEU SER GLN THR ILE VAL PRO ILE CYS LEU

        PRO ASP SER GLY LEU ALA GLU ARG GLU LEU ASN GLN ALA GLY GLN GLU

        THR LEU VAL THR GLY TRP GLY TYR HIS SER SER ARG GLU LYS GLU ALA

        LYS ARG ASN ARG THR PHE VAL LEU ASN PHE ILE LYS ILE PRO VAL VAL

        PRO HIS ASN GLU CYS SER GLU VAL MET SER ASN MET VAL SER GLU ASN

        MET LEU CYS ALA GLY ILE LEU GLY ASP ARG GLN ASP ALA CYS GLU GLY

        ASP SER GLY GLY PRO MET VAL ALA SER PHE HIS GLY THR TRP PHE LEU

        VAL GLY LEU VAL SER TRP GLY GLU GLY CYS GLY LEU LEU HIS ASN TYR

        GLY VAL TYR THR LYS VAL SER ARG TYR LEU ASP TRP ILE HIS GLY HIS

        ILE ARG ASP LYS GLU ALA PRO GLN LYS SER TRP ALA PRO-COOH
```

worin R₁ für PHE, GLY, TYR oder TRP steht, R₂ für VAL oder PRO steht, R₃ für ASP oder ASN steht, ARG für Arginin steht, ASN für Asparagin steht, ASP für Asparaginsäure steht, -COOH für den Carboxyterminus steht, CYS für Cystein steht, GLN für Glutamin steht, GLU für Glutaminsäure steht, GLY für Glycin steht, HIS für Histidin steht, ILE für Isoleucin steht, LEU für Leucin steht, LYS für Lysin steht, MET für Methionin steht, PHE für Phenylalanin steht, PRO für Prolin steht, SER für Serin steht, THR für Threonin steht, TRP für Tryptophan steht, TYR für Tyrosin steht und VAL für Valin steht.

2. DNA Verbindung nach Anspruch 1, worin das Signalpeptid und das Propeptid das Signalpeptid und das Propeptid von unreifem humanem Protein C sind.

3. DNA Verbindung nach Anspruch 1 oder 2, worin das Dipeptid Lysin-Arginin ist.

4. DNA Verbindung nach Anspruch 3, worin das durch die DNA kodierte Polypeptid folgendes ist

```
H₂N-MET TRP GLN LEU THR SER LEU LEU LEU PHE VAL ALA THR TRP GLY ILE

    SER GLY THR PRO ALA PRO LEU ASP SER VAL PHE SER SER SER GLU ARG

    ALA HIS GLN VAL LEU ARG ILE ARG LYS ARG ALA ASN SER PHE LEU GLU

    GLU LEU ARG HIS SER SER LEU GLU ARG GLU CYS ILE GLU GLU ILE CYS

    ASP PHE GLU GLU ALA LYS GLU ILE PHE GLN ASN VAL ASP ASP THR LEU

    ALA PHE TRP SER LYS HIS VAL ASP GLY ASP GLN CYS LEU VAL LEU PRO

    LEU GLU HIS PRO CYS ALA SER LEU CYS CYS GLY HIS GLY THR CYS ILE

    ASP GLY ILE GLY SER PHE SER CYS ASP CYS ARG SER GLY TRP GLU GLY

    ARG PHE CYS GLN ARG GLU VAL SER PHE LEU ASN CYS SER LEU ASP ASN

    GLY GLY CYS THR HIS TYR CYS LEU GLU GLU VAL GLY TRP ARG ARG CYS

    SER CYS ALA PRO GLY TYR LYS LEU GLY ASP ASP LEU LEU GLN CYS HIS

    PRO ALA VAL LYS PHE PRO CYS GLY ARG PRO TRP LYS ARG MET GLU LYS

    LYS ARG SER HIS LEU LYS ARG ASP THR GLU ASP GLN GLU ASP GLN VAL

    R₁  R₂  ARG LEU ILE R₃  GLY LYS MET THR ARG ARG GLY ASP SER PRO

    TRP GLN VAL VAL LEU LEU ASP SER LYS LYS LYS LEU ALA CYS GLY ALA

    VAL LEU ILE HIS PRO SER TRP VAL LEU THR ALA ALA HIS CYS MET ASP
```

```
GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

worin -H$_2$N für den Aminoterminus steht, R$_1$ für PHE, GLY, TYR oder TRP steht, R$_2$ für PRO oder VAL steht und R$_3$ für ASP oder ASN steht.

5. Rekombinanter DNA Expressionsvektor, der die DNA Verbindung nach Anspruch 1, 2, 3 oder 4 umfaßt.

6. Vektor nach Anspruch 5, worin R$_1$ für PHE steht, R$_2$ für PRO steht und R$_3$ für ASP steht.

7. Vektor nach Anspruch 6, der das wie in Beispiel 4 beschrieben hergestellte Plasmid pLPC-167F ist.

8. Vektor nach Anspruch 5, worin R$_1$ für GLY steht, R$_2$ für PRO steht und R$_3$ für ASP steht.

9. Vektor nach Anspruch 8, der das wie in Beispiel 3 beschrieben hergestellte Plasmid pLPC-167G ist.

10. Eukaryontische Wirtszelle, die mit einem Vektor nach Anspruch 5 transformiert ist.

11. Eukaryontische Wirtszelle nach Anspruch 10, die 293/pLPC-167F ist, welche durch Transformation der humanen embryonalen Nierenzellinie 293 mit dem Plasmid pLPC-167F nach Anspruch 7 hergestellt ist.

12. Eukaryontische Wirtszelle nach Anspruch 10, die 293/pLPC-167G ist, welche durch Transformation der humanen embryonalen Nierenzellinie 293 mit dem Plasmid pLPC-167G nach Anspruch 9 hergestellt ist.

13. Eukaryontische Wirtszelle nach Anspruch 10, die AV12/pLPC-167F ist, welche durch Transformation der Adenovirus-transformierten syrischen Hamsterzellinie AV12 mit dem Plasmid pLPC-167F nach Anspruch 7 hergestellt ist.

14. Eukaryontische Wirtszelle nach Anspruch 10, die AV12/pLPC-167G ist, welche durch Transformation der Adenovirus-transformierten syrischen Hamsterzellinie AV12 mit dem Plasmid pLPC-167G nach Anspruch 9 hergestellt ist.

15. Verfahren zur rekombinanten Herstellung einer Zymogenform von humanem Protein C durch Sekretion aus einer eukaryontischen Wirtszelle, gekennzeichnet durch

(A) Transformation einer eukaryontischen Wirtszelle mit einem rekombinanten DNA Vektor, der umfaßt

(i) eine DNA Sequenz, die eine Aminosäuresequenz kodiert, welche vom Aminoterminus zum Carboxyterminus umfaßt

a) ein Signalpeptid und ein Propeptid eines gamma-carboxylierten, sekretierten Proteins,
b) die leichte Kette von humanem Protein C,
c) das Dipeptid LYS-ARG, ARG-LYS, LYS-LYS oder ARG-ARG, und
d) folgende Aminosäuresequenz

```
                          ASP THR GLU ASP GLN GLU ASP GLN VAL

R₁  R₂  ARG LEU ILE R₃  GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

worin R₁ für PHE, GLY, TYR oder TRP steht, R₂ für VAL oder PRO steht, R₃ für ASP oder ASN steht, ARG für Arginin steht, ASN für Asparagin steht, ASP für Asparaginsäure steht, -COOH für den Carboxyterminus steht, CYS für Cystein steht, GLN für Glutamin steht, GLU für Glutaminsäure steht, GLY für Glycin steht, HIS für Histidin steht, ILE für Isoleucin steht, LEU für Leucin steht, LYS für Lysin steht, MET für Methionin steht, PHE für Phenylalanin steht, PRO für Prolin steht, SER für Serin steht, THR für Threonin steht, TRP für Tryptophan steht, TYR für Tyrosin steht und VAL für Valin steht, und

(ii) einen Promotor, der zur Expressionssteuerung der DNA Sequenz positioniert ist, und

(B) Kultivierung der in Schritt (A) transformierten Wirtszelle unter Bedingungen, die die Expression der DNA Sequenz erlauben.

16. Verfahren nach Anspruch 15, worin der rekombinante DNA Expressionsvektor das Plasmid pLPC-167F nach Anspruch 7 ist.

72

**17.** Verfahren nach Anspruch 15, worin der rekombinante DNA Expressionsvektor das Plasmid pLPC-167G nach Anspruch 9 ist.

**18.** Verfahren nach Anspruch 15, worin die Wirtszelle die 293- oder AV12-Wirtszelle ist.

**19.** Verfahren nach Anspruch 18, worin die in Schritt (B) kultivierte Wirtszelle die 293/pLPC-167F, 293/pLPC-167G, AV12/pLPC-167F oder AV12/pLPC-167G Wirtszelle nach den Ansprüchen 11 bis 14 ist.

**20.** Pharmazeutische Formulierung, die ein Protein C Zymogen mit der folgenden Aminosäuresequenz enthält

```
H₂N-MET TRP GLN LEU THR SER LEU LEU LEU PHE VAL ALA THR TRP GLY ILE

      SER GLY THR PRO ALA PRO LEU ASP SER VAL PHE SER SER SER GLU ARG

      ALA HIS GLN VAL LEU ARG ILE ARG LYS ARG ALA ASN SER PHE LEU GLU

      GLU LEU ARG HIS SER SER LEU GLU ARG GLU CYS ILE GLU GLU ILE CYS

      ASP PHE GLU GLU ALA LYS GLU ILE PHE GLN ASN VAL ASP ASP THR LEU

      ALA PHE TRP SER LYS HIS VAL ASP GLY ASP GLN CYS LEU VAL LEU PRO

      LEU GLU HIS PRO CYS ALA SER LEU CYS CYS GLY HIS GLY THR CYS ILE

      ASP GLY ILE GLY SER PHE SER CYS ASP CYS ARG SER GLY TRP GLU GLY

      ARG PHE CYS GLN ARG GLU VAL SER PHE LEU ASN CYS SER LEU ASP ASN

      GLY GLY CYS THR HIS TYR CYS LEU GLU GLU VAL GLY TRP ARG ARG CYS

      SER CYS ALA PRO GLY TYR LYS LEU GLY ASP ASP LEU LEU GLN CYS HIS

      PRO ALA VAL LYS PHE PRO CYS GLY ARG PRO TRP LYS ARG MET GLU LYS

      LYS ARG SER HIS LEU LYS ARG ASP THR GLU ASP GLN GLU ASP GLN VAL

      R₁  R₂  ARG LEU ILE R₃  GLY LYS MET THR ARG ARG GLY ASP SER PRO

      TRP GLN VAL VAL LEU LEU ASP SER LYS LYS LYS LEU ALA CYS GLY ALA

      VAL LEU ILE HIS PRO SER TRP VAL LEU THR ALA ALA HIS CYS MET ASP

      GLU SER LYS LYS LEU LEU VAL ARG LEU GLY GLU TYR ASP LEU ARG ARG
```

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH

worin H₂N für den Aminoterminus steht, $R_1$ für PHE, GLY, TYR oder TRP steht, $R_2$ für VAL oder PRO steht, $R_3$ für ASP oder ASN steht, ARG für Arginin steht, ASN für Asparagin steht, ASP für Asparaginsäure steht, -COOH für den Carboxyterminus steht, CYS für Cystein steht, GLN für Glutamin steht, GLU für Glutaminsäure steht, GLY für Glycin steht, HIS für Histidin steht, ILE für Isoleucin steht, LEU für Leucin steht, LYS für Lysin steht, MET für Methionin steht, PHE für Phenylalanin steht, PRO für Prolin steht, SER für Serin steht, THR für Threonin steht, TRP für Tryptophan steht, TYR für Tyrosin steht und VAL für Valin steht,
zusammen mit einem pharmazeutisch annehmbaren Träger hierfür.

21. Pharmazeutische Formulierung, die ein aktiviertes Protein C Molekül, das die leichte Kette von aktiviertem humanem Protein C und die folgende schwere Kette enthält

```
        H₂N-LEU ILE ASN GLY LYS MET THR ARG ARG GLY ASP SER PRO
TRP GLN VAL VAL LEU LEU ASP SER LYS LYS LYS LEU ALA CYS GLY ALA
VAL LEU ILE HIS PRO SER TRP VAL LEU THR ALA ALA HIS CYS MET ASP
GLU SER LYS LYS LEU LEU VAL ARG LEU GLY GLU TYR ASP LEU ARG ARG
TRP GLU LYS TRP GLU LEU ASP LEU ASP ILE LYS GLU VAL PHE VAL HIS
PRO ASN TYR SER LYS SER THR THR ASP ASN ASP ILE ALA LEU LEU HIS
LEU ALA GLN PRO ALA THR LEU SER GLN THR ILE VAL PRO ILE CYS LEU
PRO ASP SER GLY LEU ALA GLU ARG GLU LEU ASN GLN ALA GLY GLN GLU
THR LEU VAL THR GLY TRP GLY TYR HIS SER SER ARG GLU LYS GLU ALA
LYS ARG ASN ARG THR PHE VAL LEU ASN PHE ILE LYS ILE PRO VAL VAL
PRO HIS ASN GLU CYS SER GLU VAL MET SER ASN MET VAL SER GLU ASN
MET LEU CYS ALA GLY ILE LEU GLY ASP ARG GLN ASP ALA CYS GLU GLY
ASP SER GLY GLY PRO MET VAL ALA SER PHE HIS GLY THR TRP PHE LEU
VAL GLY LEU VAL SER TRP GLY GLU GLY CYS GLY LEU LEU HIS ASN TYR
GLY VAL TYR THR LYS VAL SER ARG TYR LEU ASP TRP ILE HIS GLY HIS
ILE ARG ASP LYS GLU ALA PRO GLN LYS SER TRP ALA PRO-COOH
```

worin ALA für Alanin steht, ARG für Arginin steht, ASN für Asparagin steht, ASP für Asparaginsäure steht, -COOH für den Carboxyterminus steht, CYS für Cystein steht, GLN für Glutamin steht, GLU für Glutaminsäure steht, GLY für Glycin steht, $H_2N$ für den Aminoterminus steht, HIS für Histidin steht, ILE für Isoleucin steht, LEU für Leucin steht, LYS für Lysin steht, MET für Methionin steht, PHE für Phenylalanin steht, PRO für Prolin steht, SER für Serin steht, THR für Threonin steht, TRP für Tryptophan steht, TYR für Tyrosin steht und VAL für Valin steht,
zusammen mit einem pharmazeutisch annehmbaren Träger hierfür.

**Revendications**
**Revendications pour les Etats contractants suivants : AT, BE, CH, DE, FR, GB, IT, LI, LU, NL, SE**

1. Composé d'ADN comprenant une séquence de codage pour une protéine, qui comprend, de la terminaison amino à la terminaison carboxyle :

   a) un peptide signal et un propeptide d'une protéine sécrétée γ-carboxylée;

   b) la chaîne légère de la protéine C humaine;

   c) le dipeptide lysine-arginine, arginine-lysine, lysine-lysine ou arginine-arginine; et

d) la séquence de résidus d'aminoacides :

```
                                        ASP THR GLU ASP GLN GLU ASP GLN VAL

R₁  R₂  ARG LEU ILE R₃  GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

dans laquelle R₁ représente PHE, GLY, TYR ou TRP, R₂ représente VAL ou PRO, R₃ représente ASP ou ASN, ARG représente l'arginine, ASN représente l'asparagine, ASP représente l'acide aspartique, -COOH représente la terminaison carboxyle, CYS représente la cystéine, GLN représente la glutamine, GLU représente l'acide glutamique, GLY représente la glycine, HIS représente l'histidine, ILE représente l'isoleucine, LEU représente la leucine, LYS représente la lysine, MET représente la méthionine, PHE représente la phénylalanine, PRO représente la proline, SER représente la sérine, THR représente la thréonine, TRP représente le tryptophane, TYR représente la tyrosine et VAL représente la valine.

2. Composé d'ADN selon la revendication 1, dans lequel le peptide signal et le propeptide sont le peptide signal et le propeptide de la protéine C humaine naissante.

3. Composé d'ADN selon la revendication 1 ou 2, dans lequel le dipeptide est la lysine-arginine.

**4.** Composé d'ADN selon la revendication 3, dans lequel le polypeptide encodé par l'ADN est :

$H_2N$-MET TRP GLN LEU THR SER LEU LEU LEU PHE VAL ALA THR TRP GLY ILE

SER GLY THR PRO ALA PRO LEU ASP SER VAL PHE SER SER SER GLU ARG

ALA HIS GLN VAL LEU ARG ILE ARG LYS ARG ALA ASN SER PHE LEU GLU

GLU LEU ARG HIS SER SER LEU GLU ARG GLU CYS ILE GLU GLU ILE CYS

ASP PHE GLU GLU ALA LYS GLU ILE PHE GLN ASN VAL ASP ASP THR LEU

ALA PHE TRP SER LYS HIS VAL ASP GLY ASP GLN CYS LEU VAL LEU PRO

LEU GLU HIS PRO CYS ALA SER LEU CYS CYS GLY HIS GLY THR CYS ILE

ASP GLY ILE GLY SER PHE SER CYS ASP CYS ARG SER GLY TRP GLU GLY

ARG PHE CYS GLN ARG GLU VAL SER PHE LEU ASN CYS SER LEU ASP ASN

GLY GLY CYS THR HIS TYR CYS LEU GLU GLU VAL GLY TRP ARG ARG CYS

SER CYS ALA PRO GLY TYR LYS LEU GLY ASP ASP LEU LEU GLN CYS HIS

PRO ALA VAL LYS PHE PRO CYS GLY ARG PRO TRP LYS ARG MET GLU LYS

LYS ARG SER HIS LEU LYS ARG ASP THR GLU ASP GLN GLU ASP GLN VAL

$R_1$ $R_2$ ARG LEU ILE $R_3$ GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

78

```
GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

dans lequel -$H_2N$ représente la terminaison amino; $R_1$ représente PHE, GLY, TYR ou TRP, $R_2$ représente PRO ou VAL et $R_3$ représente ASP ou ASN.

5. Vecteur d'expression d'ADN recombinant comprenant le composé d'ADN selon la revendication 1, 2, 3 ou 4.

6. Vecteur selon la revendication 5, dans lequel $R_1$ représente PHE, $R_2$ représente PRO et $R_3$ représente ASP.

7. Vecteur selon la revendication 6, à savoir le plasmide pLPC-167F obtenu comme décrit à l'exemple 4.

8. Vecteur selon la revendication 5, dans lequel $R_1$ représente GLY, $R_2$ représente PRO et $R_3$ représente ASP.

9. Vecteur selon la revendication 8, à savoir le plasmide pLPC-167G obtenu comme décrit à l'exemple 3.

10. Cellule hôte eucaryote transformée avec un vecteur selon la revendication 5.

**11.** Cellule hôte eucaryote selon la revendication 10, à savoir 293/pLPC-167F obtenu en transformant la lignée cellulaire 293 de rein embryonnaire humain avec le plasmide pLPC-167F tel que défini à la revendication 7.

**12.** Cellule hôte eucaryote selon la revendication 10, à savoir 293/pLPC-167G obtenu en transformant la lignée cellulaire 293 de rein embryonnaire humain avec le plasmide pLPC-167G tel que défini à la revendication 9.

**13.** Cellule hôte eucaryote selon la revendication 10, à savoir AV12/pLPC-167F obtenu en transformant la lignée cellulaire AV12 de hamster syrien transformé par adénovirus, avec le plasmide pLPC-167F tel que défini à la revendication 7.

**14.** Cellule hôte eucaryote selon la revendication 10, à savoir AV12/pLPC-167G obtenu en transformant la lignée cellulaire AV12 de hamster syrien transformé par adénovirus, avec le plasmide pLPC-167G tel que défini à la revendication 9.

**15.** Procédé pour la production recombinante d'une forme zymogénique de la protéine C humaine lors d'une sécrétion à partir d'une cellule hôte eucaryote, qui comprend

(A) le fait de transformer une cellule hôte eucaryote avec un vecteur d'ADN recombinant, qui comprend

(i) une séquence d'ADN qui encode une séquence de résidus d'aminoacides, qui comprend, de la terminaison amino à la terminaison carboxyle,

a) un peptide signal et un propeptide d'une protéine sécrétée $\gamma$-carboxylée;

b) la chaîne légère de la protéine C humaine;

c) le dipeptide LYS-ARG, ARG-LYS, LYS-LYS ou ARG-ARG; et

d) la séquence de résidus d'aminoacides :

ASP THR GLU ASP GLN GLU ASP GLN VAL

$R_1$ $R_2$ ARG LEU ILE $R_3$ GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

```
LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

dans laquelle $R_1$ représente PHE, GLY, TYR ou TRP, $R_2$ représente VAL ou PRO, $R_3$ représente ASP ou ASN, ARG représente l'arginine, ASN représente l'asparagine, ASP représente l'acide aspartique, -COOH représente la terminaison carboxyle, CYS représente la cystéine, GLN représente la glutamine, GLU représente l'acide glutamique, GLY représente la glycine, HIS représente l'histidine, ILE représente l'isoleucine, LEU représente la leucine, LYS représente la lysine, MET représente la méthionine, PHE représente la phénylalanine, PRO représente la proline, SER représente la sérine, THR représente la thréonine, TRP représente le tryptophane, TYR représente la tyrosine et VAL représente la valine; et

(ii) un promoteur positionné pour commander l'expression de la séquence d'ADN; et

(B) le fait de cultiver la cellule hôte transformée à l'étape (A) dans des conditions qui permettent l'expression de la séquence d'ADN.

**16.** Procédé selon la revendication 15, dans lequel le vecteur d'expression d'ADN recombinant est le plasmide pLPC-167F tel que défini à la revendication 7.

**17.** Procédé selon la revendication 15, dans lequel le vecteur d'expression d'ADN recombinant est le plasmide pLPC-167G tel que défini à la revendication 9.

**18.** Procédé selon la revendication 15, dans lequel la cellule hôte est la cellule hôte 293 ou AV12.

**19.** Procédé selon la revendication 18, dans lequel la cellule hôte cultivée à l'étape (B) est la cellule hôte 293/pLPC-167F, 293/pLPC-167G, AV12/pLPC-167F ou AV12/pLPC-167G, telles que définies dans les revendications 11 à 14.

**20.** Zymogène de la protéine C contenant la séquence de résidus d'aminoacides :

```
H₂N-MET TRP GLN LEU THR SER LEU LEU LEU PHE VAL ALA THR TRP GLY ILE

SER GLY THR PRO ALA PRO LEU ASP SER VAL PHE SER SER SER GLU ARG

ALA HIS GLN VAL LEU ARG ILE ARG LYS ARG ALA ASN SER PHE LEU GLU

GLU LEU ARG HIS SER SER LEU GLU ARG GLU CYS ILE GLU GLU ILE CYS

ASP PHE GLU GLU ALA LYS GLU ILE PHE GLN ASN VAL ASP ASP THR LEU

ALA PHE TRP SER LYS HIS VAL ASP GLY ASP GLN CYS LEU VAL LEU PRO

LEU GLU HIS PRO CYS ALA SER LEU CYS CYS GLY HIS GLY THR CYS ILE

ASP GLY ILE GLY SER PHE SER CYS ASP CYS ARG SER GLY TRP GLU GLY
```

ARG PHE CYS GLN ARG GLU VAL SER PHE LEU ASN CYS SER LEU ASP ASN

GLY GLY CYS THR HIS TYR CYS LEU GLU GLU VAL GLY TRP ARG ARG CYS

SER CYS ALA PRO GLY TYR LYS LEU GLY ASP ASP LEU LEU GLN CYS HIS

PRO ALA VAL LYS PHE PRO CYS GLY ARG PRO TRP LYS ARG MET GLU LYS

LYS ARG SER HIS LEU LYS ARG ASP THR GLU ASP GLN GLU ASP GLN VAL

R₁ R₂ ARG LEU ILE R₃ GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

```
VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

dans laquelle $H_2N$ représente la terminaison amino, $R_1$ représente PHE, GLY, TYR ou TRP, $R_2$ représente VAL ou PRO, $R_3$ représente ASP ou ASN, ARG représente l'arginine, ASN représente l'asparagine, ASP représente l'acide aspartique, -COOH représente la terminaison carboxyle, CYS représente la cystéine, GLN représente la glutamine, GLU représente l'acide glutamique, GLY représente la glycine, HIS représente l'histidine, ILE représente l'isoleucine, LEU représente la leucine, LYS représente la lysine, MET représente la méthionine, PHE représente la phénylalanine, PRO représente la proline, SER représente la sérine, THR représente la thréonine, TRP représente le tryptophane, TYR représente la tyrosine et VAL représente la valine.

21. Zymogène selon la revendication 20, dans lequel $R_1$ représente PHE, $R_2$ représente PRO et $R_3$ représente ASP.

22. Zymogène selon la revendication 20, dans lequel $R_1$ représente GLY, $R_2$ représente PRO et $R_3$ représente ASP.

23. Molécule de protéine C activée, comprenant la chaîne légère de la protéine C humaine activée et la chaîne lourde :

H$_2$N-LEU ILE ASN GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH

dans laquelle ALA représente l'alanine, ARG représente l'arginine, ASN représente l'asparagine, ASP représente l'acide aspartique, -COOH représente la terminaison carboxyle, CYS représente la cystéine, GLN représente la glutamine, GLU représente l'acide glutamique, GLY représente la glycine, -H$_2$N représente la terminaison amino, HIS représente l'histidine, H$_2$N- représente la terminaison amino, ILE représente l'isoleucine, LEU représente la leucine, LYS représente la lysine, MET représente la méthionine, PHE représente la phénylalanine, PRO représente la proline, SER représente la sérine, THR représente la thréonine, TRP représente le tryptophane, TYR représente la tyrosine et VAL représente la valine.

**24.** Formulation pharmaceutique qui comprend le zymogène de la revendication 20, en association avec un support pharmaceutiquement acceptable pour ce dernier.

**25.** Formulation pharmaceutique qui comprend la protéine C activée selon la revendication 23, en association avec un support pharmaceutiquement acceptable pour cette dernière.

**Revendications pour l'Etat contractant suivant : ES**

**1.** Procédé pour préparer un vecteur d'ADN recombinant, qui comprend le fait de soumettre à une ligation mutuelle (I) un composé d'ADN comprenant une séquence de codage pour une protéine, qui comprend, de la terminaison amino à la terminaison carboxyle:
a) un peptide signal et un propeptide d'une protéine sécrétée $\gamma$-carboxylée;
b) la chaîne légère de la protéine C humaine;
c) le dipeptide lysine-arginine, arginine-lysine, lysine-lysine ou arginine-arginine; et
d) la séquence de résidus d'aminoacides :

```
                           ASP THR GLU ASP GLN GLU ASP GLN VAL

R₁   R₂   ARG LEU ILE R₃   GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL
```

```
PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

dans laquelle $R_1$ représente PHE, GLY, TYR ou TRP, $R_2$ représente VAL ou PRO, $R_3$ représente ASP ou ASN, ARG représente l'arginine, ASN représente l'asparagine, ASP représente l'acide aspartique, -COOH représente la terminaison carboxyle, CYS représente la cystéine, GLN représente la glutamine, GLU représente l'acide glutamique, GLY représente la glycine, HIS représente l'histidine, ILE représente l'isoleucine, LEU représente la leucine, LYS représente la lysine, MET représente la méthionine, PHE représente la phénylalanine, PRO représente la proline, SER représente la sérine, THR représente la thréonine, TRP représente le tryptophane, TYR représente la tyrosine et VAL représente la valine, et (II) un composé d'ADN comprenant un promoteur de telle sorte que le promoteur est positionné pour commander l'expression de la séquence de codage lors de la ligation.

87

**2.** Procédé selon la revendication 1, dans lequel le polypeptide encodé par l'ADN est :

```
H₂N-MET TRP GLN LEU THR SER LEU LEU LEU PHE VAL ALA THR TRP GLY ILE

     SER GLY THR PRO ALA PRO LEU ASP SER VAL PHE SER SER SER GLU ARG

     ALA HIS GLN VAL LEU ARG ILE ARG LYS ARG ALA ASN SER PHE LEU GLU

     GLU LEU ARG HIS SER SER LEU GLU ARG GLU CYS ILE GLU GLU ILE CYS

     ASP PHE GLU GLU ALA LYS GLU ILE PHE GLN ASN VAL ASP ASP THR LEU

     ALA PHE TRP SER LYS HIS VAL ASP GLY ASP GLN CYS LEU VAL LEU PRO

     LEU GLU HIS PRO CYS ALA SER LEU CYS CYS GLY HIS GLY THR CYS ILE

     ASP GLY ILE GLY SER PHE SER CYS ASP CYS ARG SER GLY TRP GLU GLY

     ARG PHE CYS GLN ARG GLU VAL SER PHE LEU ASN CYS SER LEU ASP ASN

     GLY GLY CYS THR HIS TYR CYS LEU GLU GLU VAL GLY TRP ARG ARG CYS

     SER CYS ALA PRO GLY TYR LYS LEU GLY ASP ASP LEU LEU GLN CYS HIS

     PRO ALA VAL LYS PHE PRO CYS GLY ARG PRO TRP LYS ARG MET GLU LYS

     LYS ARG SER HIS LEU LYS ARG ASP THR GLU ASP GLN GLU ASP GLN VAL

     R₁  R₂  ARG LEU ILE R₃  GLY LYS MET THR ARG ARG GLY ASP SER PRO

     TRP GLN VAL VAL LEU LEU ASP SER LYS LYS LYS LEU ALA CYS GLY ALA

     VAL LEU ILE HIS PRO SER TRP VAL LEU THR ALA ALA HIS CYS MET ASP
```

```
GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

dans lequel $H_2N$ représente la terminaison amino; $R_1$ représente PHE, GLY, TYR ou TRP, $R_2$ représente PRO ou VAL et $R_3$ représente ASP ou ASN.

**3.** Procédé selon la revendication 1, qui procure le plasmide pLPC-167F, ledit plasmide étant défini comme décrit à l'exemple 4.

**4.** Procédé selon la revendication 1, qui procure le plasmide pLPC-167G, ledit plasmide étant défini comme décrit à l'exemple 3.

**5.** Procédé pour la production recombinante d'une forme zymogénique de la protéine C humaine lors de la sécrétion à partir d'une cellule hôte eucaryote, qui comprend
(A) le fait de transformer une cellule hôte eucaryote avec un vecteur d'ADN recombinant, qui comprend
(i) une séquence d'ADN qui encode une séquence de résidus d'aminoacides, qui comprend, de la terminaison amino à la terminaison carboxyle,
a) un peptide signal et un propeptide d'une protéine sécrétée γ-carboxylée;
b) la chaîne légère de la protéine C humaine;
c) le dipeptide LYS-ARG, ARG-LYS, LYS-LYS ou ARG-ARG; et

89

d) la séquence de résidus d'aminoacides :

```
                        ASP THR GLU ASP GLN GLU ASP GLN VAL

R₁  R₂  ARG LEU ILE R₃  GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU


    PRO ASP SER GLY LEU ALA GLU ARG GLU LEU ASN GLN ALA GLY GLN GLU

    THR LEU VAL THR GLY TRP GLY TYR HIS SER SER ARG GLU LYS GLU ALA

    LYS ARG ASN ARG THR PHE VAL LEU ASN PHE ILE LYS ILE PRO VAL VAL

    PRO HIS ASN GLU CYS SER GLU VAL MET SER ASN MET VAL SER GLU ASN

    MET LEU CYS ALA GLY ILE LEU GLY ASP ARG GLN ASP ALA CYS GLU GLY

    ASP SER GLY GLY PRO MET VAL ALA SER PHE HIS GLY THR TRP PHE LEU

    VAL GLY LEU VAL SER TRP GLY GLU GLY CYS GLY LEU LEU HIS ASN TYR

    GLY VAL TYR THR LYS VAL SER ARG TYR LEU ASP TRP ILE HIS GLY HIS

    ILE ARG ASP LYS GLU ALA PRO GLN LYS SER TRP ALA PRO-COOH
```

dans laquelle R₁ représente PHE, GLY, TYR ou TRP, R₂ représente VAL ou PRO, R₃ représente ASP ou ASN, ARG représente l'arginine, ASN représente l'asparagine, ASP représente l'acide aspartique, -COOH représente la terminaison carboxyle, CYS représente la cystéine, GLN représente la glutamine, GLU représente l'acide glutamique, GLY représente la glycine, HIS représente l'histidine, ILE représente l'isoleucine, LEU représente la leucine, LYS représente la lysine, MET représente la méthionine, PHE représente la phénylalanine, PRO représente la proline, SER représente la sérine, THR représente la thréonine, TRP représente le tryptophane, TYR représente la tyrosine et VAL représente la valine; et

(ii) un promoteur positionné pour commander l'expression de la séquence d'ADN; et

(B) le fait de cultiver la cellule hôte transformée à l'étape (A) dans des conditions qui permettent l'expression de la séquence d'ADN.

6. Procédé selon la revendication 5, dans lequel le vecteur d'expression d'ADN recombinant est le plasmide pLPC-167F tel que défini à la revendication 3.

7. Procédé selon la revendication 5, dans lequel le vecteur d'expression d'ADN recombinant est le plasmide pLPC-167G tel que défini à la revendication 4.

8. Procédé selon la revendication 5, 6 ou 7, dans lequel la cellule hôte est la cellule hôte 293 ou AV12.

9. Procédé selon la revendication 8, dans lequel la cellule hôte cultivée à l'étape (B) est
293/pLPC-167F, obtenue en transformant la lignée cellulaire 293 de rein embryonnaire humain avec le plasmide pLPC-167F tel que défini à la revendication 3,
293/pLPC-167G, obtenue en transformant la lignée cellulaire 293 de rein embryonnaire humain avec le plasmide pLPC-167G tel que défini à la revendication 4,
AV12/pLPC-167F, obtenue en transformant la lignée cellulaire AV12 de hamster syrien transformé par adénovirus, avec le plasmide pLPC-167F tel que défini à la revendication 3, ou
AV12/pLPC-167G, obtenue en transformant la lignée cellulaire AV12 de hamster syrien transformé par adénovirus, avec le plasmide pLPC-167G tel que défini à la revendication 4.

**Revendications pour l'Etat contractant suivant : GR**

1. Composé d'ADN comprenant une séquence de codage pour une protéine, qui comprend, de la terminaison amino à la terminaison carboxyle :
a) un peptide signal et un propeptide d'une protéine sécrétée $\gamma$-carboxylée;
b) la chaîne légère de la protéine C humaine;
c) le dipeptide lysine-arginine, arginine-lysine, lysine-lysine ou arginine-arginine; et

d) la séquence de résidus d'aminoacides :

```
                                   ASP THR GLU ASP GLN GLU ASP GLN VAL

R₁  R₂  ARG LEU ILE R₃  GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

dans laquelle R₁ représente PHE, GLY, TYR ou TRP, R₂ représente VAL ou PRO, R₃ représente ASP ou ASN, ARG représente l'arginine, ASN représente l'asparagine, ASP représente l'acide aspartique, -COOH représente la terminaison carboxyle, CYS représente la cystéine, GLN représente la glutamine,

92

GLU représente l'acide glutamique, GLY représente la glycine, HIS représente l'histidine, ILE représente l'isoleucine, LEU représente la leucine, LYS représente la lysine, MET représente la méthionine, PHE représente la phénylalanine, PRO représente la proline, SER représente la sérine, THR représente la thréonine, TRP représente le tryptophane, TYR représente la tyrosine et VAL représente la valine.

2. Composé d'ADN selon la revendication 1, dans lequel le peptide signal et le propeptide sont le peptide signal et le propeptide de la protéine C humaine naissante.

3. Composé d'ADN selon la revendication 1 ou 2, dans lequel le dipeptide est la lysine-arginine.

**4.** Composé d'ADN selon la revendication 3, dans lequel le polypeptide encodé par l'ADN est :

$H_2N$-MET TRP GLN LEU THR SER LEU LEU LEU PHE VAL ALA THR TRP GLY ILE

SER GLY THR PRO ALA PRO LEU ASP SER VAL PHE SER SER SER GLU ARG

ALA HIS GLN VAL LEU ARG ILE ARG LYS ARG ALA ASN SER PHE LEU GLU

GLU LEU ARG HIS SER SER LEU GLU ARG GLU CYS ILE GLU GLU ILE CYS

ASP PHE GLU GLU ALA LYS GLU ILE PHE GLN ASN VAL ASP ASP THR LEU

ALA PHE TRP SER LYS HIS VAL ASP GLY ASP GLN CYS LEU VAL LEU PRO

LEU GLU HIS PRO CYS ALA SER LEU CYS CYS GLY HIS GLY THR CYS ILE

ASP GLY ILE GLY SER PHE SER CYS ASP CYS ARG SER GLY TRP GLU GLY

ARG PHE CYS GLN ARG GLU VAL SER PHE LEU ASN CYS SER LEU ASP ASN

GLY GLY CYS THR HIS TYR CYS LEU GLU GLU VAL GLY TRP ARG ARG CYS

SER CYS ALA PRO GLY TYR LYS LEU GLY ASP ASP LEU LEU GLN CYS HIS

PRO ALA VAL LYS PHE PRO CYS GLY ARG PRO TRP LYS ARG MET GLU LYS

LYS ARG SER HIS LEU LYS ARG ASP THR GLU ASP GLN GLU ASP GLN VAL

$R_1$  $R_2$  ARG LEU ILE $R_3$  GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

```
GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH
```

dans lequel -H$_2$N représente la terminaison amino; R$_1$ représente PHE, GLY, TYR ou TRP, R$_2$ représente PRO ou VAL et R$_3$ représente ASP ou ASN.

5. Vecteur d'expression d'ADN recombinant comprenant le composé d'ADN selon la revendication 1, 2, 3 ou 4.

6. Vecteur selon la revendication 5, dans lequel R$_1$ représente PHE, R$_2$ représente PRO et R$_3$ représente ASP.

7. Vecteur selon la revendication 6, à savoir le plasmide pLPC-167F obtenu comme décrit à l'exemple 4.

8. Vecteur selon la revendication 5, dans lequel R$_1$ représente GLY, R$_2$ représente PRO et R$_3$ représente ASP.

9. Vecteur selon la revendication 8, à savoir le plasmide pLPC-167G obtenu comme décrit à l'exemple 3.

10. Cellule hôte eucaryote transformée avec un vecteur selon la revendication 5.

**11.** Cellule hôte eucaryote selon la revendication 10, à savoir 293/pLPC-167F obtenu en transformant la lignée cellulaire 293 de rein embryonnaire humain avec le plasmide pLPC-167F tel que défini à la revendication 7.

**12.** Cellule hôte eucaryote selon la revendication 10, à savoir 293/pLPC-167G obtenu en transformant la lignée cellulaire 293 de rein embryonnaire humain avec le plasmide pLPC-167G tel que défini à la revendication 9.

**13.** Cellule hôte eucaryote selon la revendication 10, à savoir AV12/pLPC-167F obtenu en transformant la lignée cellulaire AV12 de hamster syrien transformé par adénovirus, avec le plasmide pLPC-167F tel que défini à la revendication 7.

**14.** Cellule hôte eucaryote selon la revendication 10, à savoir AV12/pLPC-167G obtenu en transformant la lignée cellulaire AV12 de hamster syrien transformé par adénovirus, avec le plasmide pLPC-167G tel que défini à la revendication 9.

**15.** Procédé pour la production recombinante d'une forme zymogénique de la protéine C humaine lors de la sécrétion à partir d'une cellule hôte eucaryote, qui comprend

(A) le fait de transformer une cellule hôte eucaryote avec un vecteur d'ADN recombinant, qui comprend

(i) une séquence d'ADN qui encode une séquence de résidus d'aminoacides, qui comprend, de la terminaison amino à la terminaison carboxyle,

a) un peptide signal et un propeptide d'une protéine sécrétée $\gamma$-carboxylée;

b) la chaîne légère de la protéine C humaine;

c) le dipeptide LYS-LYS, ARG-ARG, LYS-LYS ou ARG-ARG; et

d) la séquence de résidus d'aminoacides :

```
                    ASP THR GLU ASP GLN GLU ASP GLN VAL


R₁  R₂  ARG LEU ILE R₃  GLY LYS MET THR ARG ARG GLY ASP SER PRO


TRP GLN VAL VAL LEU LEU ASP SER LYS LYS LYS LEU ALA CYS GLY ALA


VAL LEU ILE HIS PRO SER TRP VAL LEU THR ALA ALA HIS CYS MET ASP


GLU SER LYS LYS LEU LEU VAL ARG LEU GLY GLU TYR ASP LEU ARG ARG


TRP GLU LYS TRP GLU LEU ASP LEU ASP ILE LYS GLU VAL PHE VAL HIS


PRO ASN TYR SER LYS SER THR THR ASP ASN ASP ILE ALA LEU LEU HIS


LEU ALA GLN PRO ALA THR LEU SER GLN THR ILE VAL PRO ILE CYS LEU


PRO ASP SER GLY LEU ALA GLU ARG GLU LEU ASN GLN ALA GLY GLN GLU


THR LEU VAL THR GLY TRP GLY TYR HIS SER SER ARG GLU LYS GLU ALA
```

```
LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY
        .

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO−COOH
```

dans laquelle $R_1$ représente PHE, GLY, TYR ou TRP, $R_2$ représente VAL ou PRO, $R_3$ représente ASP ou ASN, ARG représente l'arginine, ASN représente l'asparagine, ASP représente l'acide aspartique, -COOH représente la terminaison carboxyle, CYS représente la cystéine, GLN représente la glutamine, GLU représente l'acide glutamique, GLY représente la glycine, HIS représente l'histidine, ILE représente l'isoleucine, LEU représente la leucine, LYS représente la lysine, MET représente la méthionine, PHE représente la phénylalanine, PRO représente la proline, SER représente la sérine, THR représente la thréonine, TRP représente le tryptophane, TYR représente la tyrosine et VAL représente la valine; et

(ii) un promoteur positionné pour commander l'expression de la séquence d'ADN; et

(B) le fait de cultiver la cellule hôte transformée à l'étape (A) dans des conditions qui permettent l'expression de la séquence d'ADN.

16. Procédé selon la revendication 15, dans lequel le vecteur d'expression d'ADN recombinant est le plasmide pLPC-167F tel que défini à la revendication 7.

17. Procédé selon la revendication 15, dans lequel le vecteur d'expression d'ADN recombinant est le plasmide pLPC-167G tel que défini à la revendication 9.

18. Procédé selon la revendication 15, dans lequel la cellule hôte est la cellule hôte 293 ou AV12.

19. Procédé selon la revendication 18, dans lequel la cellule hôte cultivée à l'étape (B) est la cellule hôte 293/pLPC-167F, 293/pLPC-167G, AV12/pLPC-167F, ou AV12/pLPC-167G telles que définies dans les revendications 11 à 14.

20. Formulation pharmaceutique qui comprend un zymogène de la protéine C contenant la séquence de résidus d'aminoacides :

H₂N-MET TRP GLN LEU THR SER LEU LEU LEU PHE VAL ALA THR TRP GLY ILE

SER GLY THR PRO ALA PRO LEU ASP SER VAL PHE SER SER SER GLU ARG

ALA HIS GLN VAL LEU ARG ILE ARG LYS ARG ALA ASN SER PHE LEU GLU

GLU LEU ARG HIS SER SER LEU GLU ARG GLU CYS ILE GLU GLU ILE CYS

ASP PHE GLU GLU ALA LYS GLU ILE PHE GLN ASN VAL ASP ASP THR LEU

ALA PHE TRP SER LYS HIS VAL ASP GLY ASP GLN CYS LEU VAL LEU PRO

LEU GLU HIS PRO CYS ALA SER LEU CYS CYS GLY HIS GLY THR CYS ILE

ASP GLY ILE GLY SER PHE SER CYS ASP CYS ARG SER GLY TRP GLU GLY

ARG PHE CYS GLN ARG GLU VAL SER PHE LEU ASN CYS SER LEU ASP ASN

GLY GLY CYS THR HIS TYR CYS LEU GLU GLU VAL GLY TRP ARG ARG CYS

SER CYS ALA PRO GLY TYR LYS LEU GLY ASP ASP LEU LEU GLN CYS HIS

PRO ALA VAL LYS PHE PRO CYS GLY ARG PRO TRP LYS ARG MET GLU LYS

LYS ARG SER HIS LEU LYS ARG ASP THR GLU ASP GLN GLU ASP GLN VAL

$R_1$  $R_2$  ARG LEU ILE $R_3$  GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO-COOH

dans laquelle $H_2N$ représente la terminaison amino, $R_1$ représente PHE, GLY, TYR ou TRP, $R_2$ représente VAL ou PRO, $R_3$ représente ASP ou ASN, ARG représente l'arginine, ASN représente l'asparagine, ASP représente l'acide aspartique, -COOH représente la terminaison carboxyle, CYS représente la cystéine, GLN représente la glutamine, GLU représente l'acide glutamique, GLY représente la glycine, HIS représente l'histidine, ILE représente l'isoleucine, LEU représente la leucine, LYS représente la lysine, MET représente la méthionine, PHE représente la phénylalanine, PRO représente la proline, SER représente la sérine, THR représente la thréonine, TRP représente le tryptophane, TYR représente la tyrosine et VAL représente la valine, en association avec un support pharmaceutiquement acceptable pour le zymogène.

21. Formulation pharmaceutique qui comprend une molécule de protéine C activée comprenant la chaîne légère de la protéine C humaine activée et la chaîne lourde :

$H_2N$-LEU ILE ASN GLY LYS MET THR ARG ARG GLY ASP SER PRO

TRP GLN VAL VAL LEU LEU ASP SER LYS LYS LYS LEU ALA CYS GLY ALA

VAL LEU ILE HIS PRO SER TRP VAL LEU THR ALA ALA HIS CYS MET ASP

GLU SER LYS LYS LEU LEU VAL ARG LEU GLY GLU TYR ASP LEU ARG ARG

TRP GLU LYS TRP GLU LEU ASP LEU ASP ILE LYS GLU VAL PHE VAL HIS

100

```
PRO ASN TYR SER LYS SER THR THR ASP ASN ASP ILE ALA LEU LEU HIS

LEU ALA GLN PRO ALA THR LEU SER GLN THR ILE VAL PRO ILE CYS LEU

PRO ASP SER GLY LEU ALA GLU ARG GLU LEU ASN GLN ALA GLY GLN GLU

THR LEU VAL THR GLY TRP GLY TYR HIS SER SER ARG GLU LYS GLU ALA

LYS ARG ASN ARG THR PHE VAL LEU ASN PHE ILE LYS ILE PRO VAL VAL

PRO HIS ASN GLU CYS SER GLU VAL MET SER ASN MET VAL SER GLU ASN

MET LEU CYS ALA GLY ILE LEU GLY ASP ARG GLN ASP ALA CYS GLU GLY

ASP SER GLY GLY PRO MET VAL ALA SER PHE HIS GLY THR TRP PHE LEU

VAL GLY LEU VAL SER TRP GLY GLU GLY CYS GLY LEU LEU HIS ASN TYR

GLY VAL TYR THR LYS VAL SER ARG TYR LEU ASP TRP ILE HIS GLY HIS

ILE ARG ASP LYS GLU ALA PRO GLN LYS SER TRP ALA PRO—COOH
```

dans laquelle ALA représente l'alanine, ARG représente l'arginine, ASN représente l'asparagine, ASP représente l'acide aspartique, -COOH représente la terminaison carboxyle, CYS représente la cystéine, GLN représente la glutamine, GLU représente l'acide glutamique, GLY représente la glycine, -H$_2$N représente la terminaison amino, HIS représente l'histidine, H$_2$N- représente la terminaison amino, ILE représente l'isoleucine, LEU représente la leucine, LYS représente la lysine, MET représente la méthionine, PHE représente la phénylalanine, PRO représente la proline, SER représente la sérine, THR représente la thréonine, TRP représente le tryptophane, TYR représente la tyrosine et VAL représente la valine, en association avec un support pharmaceutiquement acceptable pour la molécule.

# FIG.1
## The Construction of Plasmid pLPC
### Part A. Construction of Plasmid pBKneo1

pdBPV-MMTneo
ATCC 37224

BamHI

BamHI

Ligate

**Plasmid pBKneo1 (11.5 kb)**

EcoRI, HindIII, EcoRI, KpnI, BglII, PstI, PvuII, SmaI, PvuII, PstI, HpaI, BamHI, EcoRI, PvuII, BglII, HindIII, HindIII, BglII, HindIII, PvuII, HindIII, BamHI, SalI, PstI, EcoRI

MMTpro, NeoR, pA, IVS, APR, enhancer, BK

**BK Virus (5.2 kb) ATCC VR-837**

PvuII, HindIII, SstI, AccI, StuI, StuI, StuI, BamHI, EcoRI, StuI, PvuII, StuI, BglII, HindIII, StuI, AccI, StuI, SstI, AccI, AccI, HindIII, BglII, StuI, SstI, HindIII, StuI

enh, late, early

# FIG.I
## The Construction of Plasmid pLPC
### Part B. Construction of Plasmid pLPcat

Adenovirus 2 DNA

↓ BalI
↓ Isolate ~ 2.4 kb BalI
   Restriction Fragment
↓ AccI
↓ PvuII
↓ Isolate ~ 0.32 kb AccI-PvuII
   Restriction Fragment
↓ Ligate BclI Linkers

Ligate             StuI     AccI

**pLPcat (4.83 kb)**

EcoRI, PstI, BamHI, PstI, APR, PA SVI, CAT, Ad2LP, NdeI, AccI, StuI, BclI, HindIII

**pSV2cat (5.015 kb) ATCC 37155**

EcoRI, PstI, BamHI, PstI, APR, PA SVI, CAT, SV40ep, NdeI, AccI, PvuII, StuI, HindIII

EP 0 323 149 B1

# FIG. I
## The Construction of Plasmid pLPC
### Part C.   Construction of Plasmid pBLcat

# FIG.I
## The Construction of Plasmid pLPC
### Part D.   Final Construction of Plasmid pLPC

EcoRI
PstI
PstI
BamHI
ApR
PÀ IVS
pBLcat
(6.09 kb)
HindIII
Ad2
CAT
LP
Enh
NdeI
AccI
HindIII
BglII
HindIII
StuI  StuI
HindIII
BclI
StuI/PvuII

pL133
(Figure 2)
HindIII

Isolate
~0.87 kb
HindIII
Restriction
Fragment

Ligate

EcoRI
ApR
PÀ IVS
pLPC
(6.5 kb)
BclI
SstI
SV40ep Enh
Ad2
LP
protein C
NdeI
PvuII
HindIII
StuI
SstI  StuI
BclI
BclI
PvuII
SalI
SstI
StuI/PvuII  HindIII

EP 0 323 149 B1

# FIG.2
## Construction of Plasmid pL133

EP 0 323 149 B1